# EUROPEAN PATENT SPECIFICATION

(11) **EP 1 086 078 B1**
(45) Date of publication and mention of the grant of the patent: **05.02.2003**
(21) Application number: 99955470.2
(22) Date of filing: 07.06.1999
(51) Int. Cl.: C07C 321/02, C07C 311/00, C07D 211/96, A61K 31/10, A61K 31/18, A61K 31/445

(54) **NEUROPEPTIDE Y5 RECEPTOR ANTAGONISTS**
NEUROPEPTID Y5-REZEPTORANTAGONISTEN
ANTAGONISTES DES RECEPTEURS Y5 NEUROPEPTIDIQUES

(30) Priority: 08.06.1998 US 93132
(43) Date of publication of application: 28.03.2001
(73) Proprietor: SCHERING CORPORATION, Kenilworth, New Jersey 07033-0530 (US)
(72) Inventor: DUGAR, Sundeep, Bridgewater, NJ 08807 (US); NEUSTADT, Bernard, R., West Orange, NJ 07052 (US); STAMFORD, Andrew, W., Chatham Township, NJ 07928 (US); WU, Yusheng, New York, NY 10016 (US)
(74) Representative: Ritter, Stephen David
(86) International application number: US9911795
(87) International publication number: WO99064394

(56) References cited:
- WO-A-97/20821
- WO-A-98/35957

## Description

### BACKGROUND OF THE INVENTION

The present invention relates to selective neuropeptide Y Y5 receptor antagonists useful in the treatment of eating disorders and diabetes, pharmaceutical compositions containing the compounds, and methods of treatment using the compounds.

Neuropeptide Y is a 36 amino acid peptide that is widely distributed in the central and peripheral nervous systems. This peptide mediates a number of physiological effects through its various receptor subtypes. Studies in animals have shown that neuropeptide Y is a powerful stimulus of food intake, and it has been demonstrated that activation of neuropeptide Y Y5 receptors results in hyperphagia and decreased thermogenesis. Therefore compounds that antagonize neuropeptide Y at the Y5 receptor subtype represent an approach to the treatment of eating disorders such as obesity and hyperphagia.

Phenyl amides and ureas are known as antiatherosclerotic agents, see for example U.S. 4,623,662, and benzoic acid amides are disclosed as antidiabetic agents in U.S. 5,378,728. N,N-alkylenebis-(benzamides) are known as endocrinological agents, see U.S. 4,009,208. WO 98/35957, published August 20, 1998, discloses amide derivatives as selective neuropeptide Y receptor antagonists.

### SUMMARY OF THE INVENTION

The present invention relates to compounds represented by the structural formula I 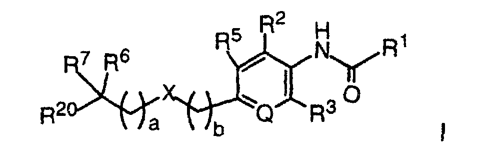 or a pharmaceutically acceptable salt thereof, wherein
a and b are independently 0, 1 or 2, provided that the sum of a and b is 0 to 3;
Q is 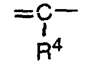 or ―N= ;
X is -O-, -S-, -SO-, -SO₂-, -CH(OR⁸)-, -C(O)-, -C(R²³)₂-, -C(R²⁵)=C(R²⁵)-, -C≡C- or 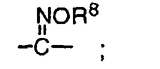 R¹ is R¹⁵-aryl, R²⁴-heteroaryl, -NHR¹², -N(R¹²)₂, -(C₁-C₉)alkyl-OC(O)R⁸, aryloxy(C₁-C₉)alkyl, 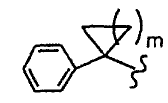 wherein m is 1-4, or 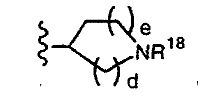 wherein d and e are independently 0, 1 or 2;
R², R³, R⁴ and R⁵ are independently selected from the group consisting of H, C₁-C₅ straight or branched alkyl, (C₃-C₁₂)cycloalkyl, R¹⁴-(C₃-C₁₂)cycloalkyl, halogen, -OR⁸, -N(R⁸)₂, -CO₂R⁸ and CF₃;
R⁶ and R⁷ are independently selected from the group consisting of H, (C₁-C₉)alkyl, (C₁-C₉)alkenyl, hydroxy-(C₁-C₉)alkyl, amino-(C₁-C₉)-alkyl, (C₁-C₉)alkoxy-(C₁-C₉)alkyl, (C₃-C₁₂)cycloalkyl and (C₃-C₁₂)-cycloalkyl-(C₁-C₆)alkyl, or R⁶ and R⁷, together with the carbon to which they are attached, form a 3, 4, 5, 6 or 7-membered carbocyclic ring, or a 4, 5, 6 or 7-membered heterocyclic ring, wherein 1, 2 or 3 ring members are independently selected from the group consisting of O, S and N;
R⁸ is independently selected from the group consisting of H, (C₁-C₆)alkyl, (C₃-C₁₂)cycloalkyl, R¹⁵-aryl and R²⁴-heteroaryl;
R⁹ is (C₁-C₆)alkyl, (C₃-C₁₂)cycloalkyl, R¹⁵-aryl or R²⁴-heteroaryl;
R¹¹ is independently selected from the group consisting of H, (C₁-C₆)alkyl and (C₃-C₁₂)cycloalkyl;
R¹² is independently selected from the group consisting of straight or branched (C₁-C₉)alkyl, hydroxy(C₂-C₉)alkyl, (C₁-C₉)alkoxy(C₂-C₉)-alkyl, N(R¹¹)(R¹⁹)-(C₂-C₉)-alkyl, HS(C₂-C₉)-alkyl, (C₁-C₉)-alkylthio-(C₂-C₉)-alkyl, (C₃-C₁₂)-cycloalkyl, R¹⁴-(C₃-C₁₂) cycloalkyl, R¹⁵-aryl,
R²⁴-heteroaryl, R¹⁵-aryl(C₁-C₆)-alkyl, R²⁴-heteroaryl(C₁-C₆)-alkyl, 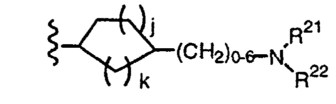 wherein j and k are independently 0, 1 or 2, and 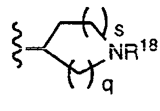 wherein q is 1 or 2, and s is 0, 1 or 2; or two R¹² groups, together with the nitrogen to which they are attached, form a ring of the formula 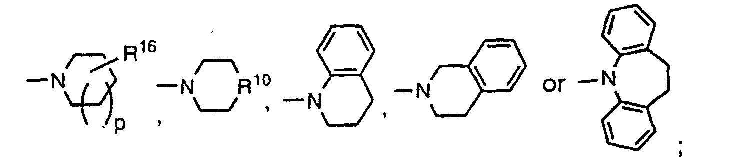 wherein p is 0, 1 or 2;
R¹⁰ is -NR¹⁸-, -O- or -S-;
R¹³ is 1 to 3 substituents independently selected from the group consisting of hydrogen, (C₁-C₆)alkyl, halogen, (C₁-C₆)alkoxy and CF₃;
R¹⁴ is 1 to 3 substituents independently selected from the group consisting of (C₁-C₆)alkyl, benzyl, R¹³-aryl and R¹³-heteroaryl;
R¹⁵ is 1 to 3 substituents independently selected from the group consisting of hydrogen, (C₁-C₆)alkyl, halo, polyhalo(C₁-C₆)alkyl, R¹⁷O-, -N(R¹⁷)₂, -S(R¹⁷), R¹⁷O-(C₁-C₆)alkyl, (R¹⁷)₂N-(C₁-C₆)alkyl, formyl, -C(O)R¹⁷, -COOR¹⁷, -CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷- and tri(C₁-C₆)-alkylsilyl;
R¹⁶ is 1-3 substituents independently selected from the group consisting of H, (C₁-C₆)alkyl, (C₃-C₁₂)cycloalkyl, (C₃-C₁₂)spirocycloalkyl, (C₃-C₄)spiro-alkylenedioxy, R¹⁵-aryl, R²⁴-heteroaryl, benzyl, N-piperidinyl, -COR⁸, -C(O)NR⁸R⁹, -NR⁸R⁹ and -NR⁸C(O)R⁹, or when two R¹⁶ groups are attached to adjacent ring carbon atoms, together with said carbon atoms, they can form a (C₅-C₇)cycloalkyl ring;
R¹⁷ is independently selected from the group consisting of H, (C₁-C₆)alkyl, (C₃-C₁₂)cycloalkyl, (C₃-C₁₂)cycloalkyl(C₁-C₆)alkyl, R¹³-aryl and R¹³-heteroaryl;
R¹⁸ is independently selected from the group consisting of H, (C₁-C₆)alkyl, (C₃-C₁₂)cycloalkyl, (C₃-C₁₂)cycloalkyl-(C₁-C₆)alkyl, R¹⁵-aryl, R²⁴-heteroaryl, -CO₂R⁹, -C(O)N(R⁸)₂, -COR⁸ and -SO₂R⁹;
R¹⁹ is H, (C₃-C₁₂)cycloalkyl-(C₁-C₆)alkyl, R¹⁵-aryl, R²⁴-heteroaryl, -CO₂R⁹, -C(O)N(R⁸)₂, -COR⁸ or -SO₂R⁹;
R²⁰ is (C₁-C₆)alkyl, (C₃-C₁₂)cycloalkyl, (C₃-C₁₂)cycloalkyl-(C₁-C₆)alkyl, hydroxy(C₁-C₆)alkyl, oxo(C₁-C₆)alkyl or polyhalo(C₁-C₆)alkyl;
R²¹ and R²² are independently selected from the group consisting of H, (C₁-C₆)alkyl, (C₃-C₁₂)cycloalkyl-(C₁-C₆)alkyl, hydroxy(C₁-C₆)alkyl, R¹⁵-aryl, R²⁴-heteroaryl, R¹⁵-aryl(C₁-C₆)alkyl or R²⁴-heteroaryl(C₁-C₆)-alkyl;
R²³ is independently selected from the group consisting of H, halogen, (C₁-C₆)alkyl, (C₃-C₁₂)cycloalkyl, R¹⁵-aryl, and R²⁴-heteroaryl;
R²⁴ is 1 to 2 substituents independently selected from the group consisting of hydrogen, (C₁-C₆)alkyl, halo, polyhalo(C₁-C₆)alkyl, R¹⁷O-, -N(R¹⁷)₂, -S(R¹⁷), R¹⁷O-(C₁-C₆)alkyl, (R¹⁷)₂N-(C₁-C₆)alkyl, formyl, -C(O)R¹⁷, -COOR¹⁷, -CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷- and tri(C₁-C₆)-alkylsilyl; and
R²⁵ is independently selected from the group consisting of hydrogen, halogen, (C₁-C₆)-alkyl and polyhalo(C₁-C₆)alkyl.

In a preferred group of compounds of formula I, Q is 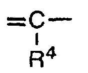 wherein R⁴ is H. Also preferred are compounds wherein R³ is H, and wherein R² and R⁵ are independently H or halogen. R⁶ and R⁷ are preferably (C₁-C₉)alkyl, especially methyl, or R⁶ and R⁷, together with the carbon to which they are attached, form a C₃-C₆ carbocyclic ring.

In compounds of formula I, X is preferably -S-; -C(O)-; or -C(R⁸)₂, especially wherein R⁸ is H. More preferably, X is -C(R⁸)₂-, and compounds wherein X is -CH₂- are especially preferred.

In compounds of formula I, a is preferably 1 or 2 and b is preferably 0.

In compounds of formula I, R¹ is preferably -NHR¹² or -N(R¹²)₂, especially 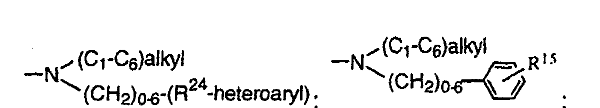 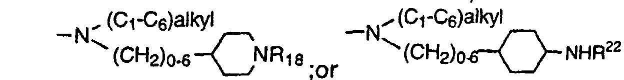 wherein R¹⁸ is (C₁-C₆)alkyl or -SO₂R⁹; R⁹ is (C₁-C₆)alkyl or aryl; and R²² is (C₁-C₆)-alkyl or (C₃-C₁₂)cycloalkyl(C₁-C₆)alkyl.

Another aspect of the invention is a pharmaceutical composition for treating eating disorders or diabetes which comprises an effective amount of a compound of formula I in combination with a pharmaceutically acceptable carrier.

Yet another aspect of this invention is a method of treating an eating disorder or diabetes comprising administering an effective amount of a compound of formula I to a patient in need of such treatment.

Also claimed are novel compounds similar to those of formula I wherein b is 0, X is -O- or -S- and the substituent corresponding to R¹ is optionally substituted alkyl.

### DETAILED DESCRIPTION

Except where stated otherwise the following definitions apply throughout the present specification and claims. These definitions apply regardless of whether a term is used by itself or in combination with other terms. Hence the definition of "alkyl" applies to "alkyl" as well as the "alkyl" portions of "alkoxy", etc.

Alkyl represents a straight or branched saturated hydrocarbon chains having the designated number of carbon atoms. If the number of carbon atoms is not specified, e.g., if the term lower alkyl is used, chain lengths of 1 to 6 carbons are intended.

When X is -C(R²⁵)=C(R²⁵)-, both cis and trans configurations are comtemplated.

Cycloalkyl represents a saturated carbocyclic ring having 3 to 12 carbon atoms. Preferred are C₃-C₆cycloalkyl rings.

In the definition of R¹⁶, the term (C₃-C₁₂)spirocycloalkyl refers to a (C₂-C₁₁)alkylene chain joined at both ends to the same ring carbon, i.e., 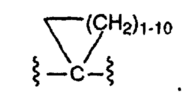 Similarly, the term (C₃-C₄)spiroalkylenedioxy refers to a (C₂-C₃)alkylenedioxy group joined at both ends to the same ring carbon atom, i.e.,

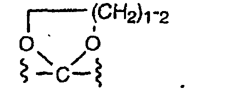

In the definition of R⁶ and R⁷, the term "heterocyclic ring" refers to 4- to 7-membered saturated rings comprising 1 to 3 heteroatoms independently selected from the group consisting of -O-, -S- and -NH-, with the remaining ring members being carbon. Where a heterocyclic ring comprises more than one heteroatom, no rings are formed where there are adjacent oxygen atoms, adjacent sulfur atoms, or three consecutive heteroatoms. Examples of heterocyclic rings are tetrahydrofuranyl, pyrrolidinyl, piperidinyl, morpholinyl, thiomorpholinyl and piperazinyl.

When two R¹² groups form a ring of the formula 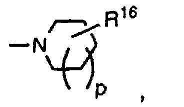 those skilled in the art will recognize that -NR⁸R⁹ and -NR⁸C(O)R⁹ substituents cannot be attached to a carbon adjacent to the piperizinyl nitrogen.

Halogen represents fluoro, chloro, bromo or iodo.

Polyhalo(C₁-C₆)alkyl refers to a straight or branched alkyl chain substituted by 1 to 5 halogen atoms, which can be attached to the same or different carbon atoms, e.g., -CH₂F, -CHF₂, -CF₃, F₃CCH₂- and -CF₂CF₃.

Hydroxy(C₁-C₆)alkyl refers to an alkyl chain substituted on any substitutable carbon by a hydroxy group. Similarly, oxo(C₁-C₆)alkyl refers to an alkyl chain substituted by an =O moiety.

Aryl represents phenyl or naphthyl.

Heteroaryl refers to 5- to 10-membered single or benzofused aromatic rings comprising 1 to 3 heteroatoms independently selected from the group consisting of -O-, -S- and -N=, provided that the rings do not include adjacent oxygen and/or sulfur atoms.. Examples of single-ring heteroaryl groups are pyridyl, isoxazolyl, oxadiazolyl, furanyl, pyrrolyl, thienyl, imidazolyl, pyrazolyl, tetrazolyl, thiazolyl, thiadiazolyl, pyrazinyl, pyrimidinyl, pyridazinyl and triazolyl. Examples of benzofused heteroaryl groups are quinolinyl, isoquinolinyl, quinazolinyl, thianaphthenyl (i.e., benzothienyl), indolyl, benzimidazolyl, benzofuranyl and benzofurazanyl. N-oxides of nitrogen-containing heteroaryl groups are also included. All positional isomers are contemplated, e.g., 2-pyridyl, 3-pyridyl and 4-pyridyl. Preferred heteroaryl groups are pyridyl, isoxazolyl, thienyl, thiazolyl, pyrazinyl, pyrimidinyl, pyridazinyl, quinolinyl, isoquinolinyl and quinazolinyl.

When a variable appears more than once in the structural formula, for example R⁸, the identity of each variable appearing more than once may be independently selected from the definition for that variable.

For compounds of the invention having at least one asymmetrical carbon atom, all isomers, including diastereomers, enantiomers and rotational isomers are contemplated as being part of this invention. The invention includes d and i isomers in both pure form and in admixture, including racemic mixtures. Isomers can be prepared using conventional techniques, either by reacting optically pure or optically enriched starting materials or by separating isomers of a compound of formula I.

Compounds of formula I can exist in unsolvated as well as solvated forms, including hydrated forms. In general, the solvated forms, with pharmaceutically acceptable solvents such as water, ethanol and the like, are equivalent to the unsolvated forms for purposes of this invention.

A compound of formula I may form pharmaceutically acceptable salts with organic and inorganic acids. Examples of suitable acids for salt formation are hydrochloric, sulfuric, phosphoric, acetic, citric, malonic, salicylic, malic, fumaric, succinic, ascorbic, maleic, methanesulfonic and other mineral and carboxylic acids well known to those skilled in the art. The salts are prepared by contacting the free base forms with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia or sodium bicarbonate. The free base forms differ from their respective salt forms somewhat in certain physical properties, such as solubility in polar solvents, but the salts are otherwise equivalent to their respective free base forms for purposes of the invention.

Compounds of formula I may be produced by processes known to those skilled in the art as shown in the examples below. Typically, the claimed compounds wherein X is -S- or -O- can be prepared as shown in the following reaction scheme: 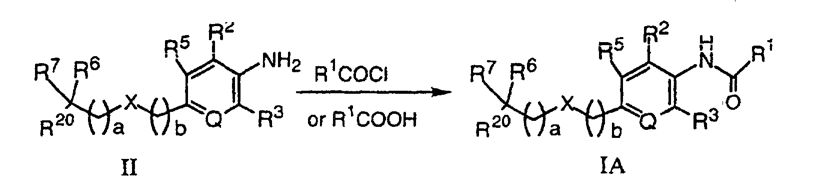 wherein an amine of formula II is reacted with an acid chloride or carbamoyl chloride in the presence of a base, or with a carboxylic acid in the presence of standard amide coupling agents such as EDC and DMAP. Starting materials of formula II can be prepared using known methods.

The compounds of formula I exhibit selective neuropeptide Y5 antagonizing activity, which has been correlated with pharmacological activity for treating eating disorders such as obesity and hyperphagia.

The compounds of formula I display pharmacological activity in test procedures designated to indicate neuropeptide Y5 receptor antagonist activity. The compounds are non-toxic at pharmaceutically therapeutic doses. Following are descriptions of the test procedures.

### cAMP Assay

CHO cells expressing the various NPY receptor subtypes were maintained in Ham's F-12 media (Gibco-BRL) supplemented with 10% FCS (ICN), 1% penicillin-streptomycin, 1% non-essential amino acids and 200 µg/ml Geneticin® (GibcoBRL #11811-031) under a humidified 5% CO₂ atmosphere. Similarly, HEK-293 cells expressing the various NPY receptor subtypes were maintained in Dulbecco's modified Eagle's media (Gibco-BRL) supplemented with 10% FCS (ICN), 1% penicillin-streptomycin and 200 µg/ml Geneticin® (GibcoBRL #11811-031) under a humidified 5% CO₂ atmosphere. Two days prior to assay, cells were released from T-175 tissue culture flasks using cell dissociation solution (1X; non-enzymatic [Sigma #C-5914]) and seeded into 96-well, flat-bottom tissue culture plates at a density of 15,000 to 20,000 cells per well. After approximately 48 hours, the cell monolayers were rinsed with Hank's balanced salt solution (HBSS) then preincubated with approximately 150 µl/well of assay buffer (HBSS supplemented with 4 mM MgCl₂, 10 mM HEPES, 0.2% BSA [HH]) containing 1 mM 3-isobutyl-1-methylxanthine ([IBMX] Sigma #I-5879) with or without the antagonist compound of interest at 37°C. After 20 minutes the 1 mM IBMX-HH assay buffer (± antagonist compound) was removed and replaced with assay buffer containing 1.5 µM (CHO cells) or 5 µM (HEK-293 cells) forskolin (Sigma #F-6886) and various concentrations of NPY in the presence or absence of one concentration of the antagonist compound of interest. At the end of 10 minutes, the media were removed and the cell monolayers treated with 75 µl ethanol. The tissue culture plates were agitated on a platform shaker for 15 minutes, after which the plates were transferred to a warm water bath in order to evaporate the ethanol. Upon bringing all wells to dryness, the cell residues were resolubilized with 250 µl FlashPlate® assay buffer. The amount of cAMP in each well was quantified using the [¹²⁵I]-cAMP FlashPlate® kit (NEN #SMP-001) and according to the protocol provided by the manufacturer. Data were expressed as either pmol cAMP/ml or as percent of control. All data points were determined in triplicate and EC₅₀'s (nM) were calculated using a nonlinear (sigmoidal) regression equation (GraphPad Prism™). The K_{B} of the antagonist compound was estimated using the following formula:

K_{B} = [B] / (1 - {[A'] / [A]})

where [A] is the EC₅₀ of the agonist (NPY) in the absence of antagonist,
[A'] is the EC₅₀ of the agonist (NPY) in the presence of antagonist, and
[B] is the concentration of the antagonist.

### NPY Receptor Binding Assay

Human NPY receptors were expressed in CHO cells. Binding assays were performed in 50 mM HEPES, pH. 7.2, 2.5 mM CaCl₂, 1 mM MgCl₂ and 0.1% BSA containing 5-10 ug of membrane protein and 0.1 nM ¹²⁵I-peptide YY (for NPY1, NPY2 and NPY5 receptors) or 0.1 nM ¹²⁵I-pancreatic polypeptide (NPY4 receptor) in a total volume of 200 ul. Non-specific binding was determined in the presence of 1 uM NPY. The reaction mixtures were incubated for 90 minutes at 30° C (NPY1 receptor) or at room temperature (NPY2, NPY4 and NPY5 receptors), then filtered through Millipore MAFC glass fiber filter plates which had been pre-soaked in 0.5% polyethyleneimine. The filters were washed with phosphate-buffered saline, and radioactivity was measured in a Packard TopCount scintillation counter.

For the compounds of this invention, a range of neuropeptide Y5 receptor binding activity from about 0.1 to about 1000nM was observed. Compounds of this invention preferably have a binding activity in the range of about 0.1 to 250 nM, more preferably about 0.1 to 100 nM, and most preferably about 0.1 to 10 nM.

For preparing pharmaceutical compositions from the compounds of formula I, pharmaceutically acceptable, inert carriers are admixed with the active compounds. The pharmaceutically acceptable carriers may be either solid or liquid. Solid form preparations include powders, tablets, dispersible granules, capsules, cachets and suppositories. A solid carrier can be one or more substances which may also act as dilutents, flavoring agents, solubilizers, lubricants, suspending agents, binders or tablet disintegrating agents; it may also be an encapsulating material.

Liquid form preparations include solutions, suspensions and emulsions. As an example may be mentioned water or water-propylene glycol solutions for parenteral injection.

Also included are solid form preparations which are intended to be converted, shortly before use, to liquid form preparations for either oral or parentertal administration. Such liquid forms include solutions, suspensions and emulsions. These particular solid form preparations are most conveniently provided in unit dose form and as such are used to provide a single liquid dosage unit.

The invention also contemplates alternative delivery systems including, but not necessarily limited to, transdermal delivery. The transdermal compositions can take the form of creams, lotions and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation such as packeted tablets, capsules and powders in vials or ampules. The unit dosage form can also be a capsule, cachet or tablet itself, or it may be the appropriate number of any of these in a packaged form.

The quantity of active compound in a unit dose preparation may be varied or adjusted from about 0.5 mg to 500 mg, preferably about 0.5 to 100 mg, according to the particular application and the potency of the active ingredient and the intended treatment. The composition may, if desired, also contain other therapeutic agents.

The daily dosage is about 0.01 to about 20 mg/kg. The dosage may be varied depending on the requirement of the patient, the severity of the condition being treated and the particular compound being employed. Determination of the proper dosage for a particular situation is within the skill of those in the medical art. For convenience, the total daily dosage may be divided and administered in portions throughout the day or by means providing continuous delivery.

The invention disclosed herein is exemplified by the following examples which should not be construed to limit the scope of the disclosure. Alternative mechanistic pathways and analogous structures may be apparent to those skilled in the art.

In the examples, the following abbreviations are used: phenyl (Ph), acetyl (Ac), ether (Et₂O), ethyl acetate (EtOAc), dimethylformamide (DMF) and ethanol (EtOH). Room temperature is RT.

### Preparation 1

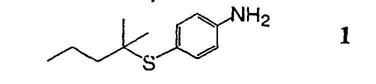

To a stirred mixture of 4-aminothiophenol (12.52 g, 0.100 mol) and 2-methyl-1-pentene (25.30 g, 0.300 mol) in anhydrous Et₂O (100 ml) was added concentrated H₂SO₄ (15.3 ml, 0.300 mol) cautiously. The clear solution was stirred for 45 min, then poured into cold sat'd NaHCO₃ (200 ml). The resultant white solid was collected, washed with cold water several times and dried *in vacuo* to afford **1** (100%). ¹H NMR (400 MHz, CDCl₃) δ 7.33 (d, 2H, *J*= 8.7 Hz, ArH), 6.68 (d, 2H, *J*= 8.6 Hz, ArH), 1.47 (m, 4H, CH₂CH₂CH₃), 1.24 (s, 9H, (CH₃)₃C), 0.97 (t, 3H, *J* = 7.0 Hz, CH₂CH₃).

### Preparation 2

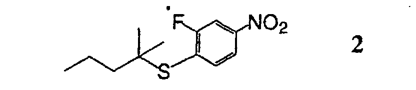

A mixture of 3,4-difluoronitrobenzene (1.0 ml, 9.03 mmol) and Na₂S•9H₂O (3.25 g, 13.5 mmol) in DMF (10 ml) was stirred at RT for 20 h, then poured into cold water. The whole was extracted with CH₂Cl₂ (3x100 ml). The combined organic layers were dried (Na₂SO₄), filtered and concentrated. To the residue was added 2-methyl-1-pentene (2.2 ml, 18.1 mmol) and Et₂O (5.0 ml). To the vigorously stirred mixture was slowly added concentrated H₂SO₄ (1.0 ml). After 1 h the reaction mixture was poured into cold water. The whole was extracted with CH₂Cl₂ (3x100 ml), and the combined organic layers were dried (Na₂SO₄), filtered and evaporated. Purification of the residue by flash column chromatography (1:20 EtOAc/hexanes) afforded **2** (100%). ¹H NMR (CDCl₃, 400 MHz) δ 8.07 (m, 2H, ArH), 7.34 (t, 1H, ArH), 1.59 (m, 4H, CH₃CH₂CH₂), 1.37 (s, 6H, C(CH₃)₂S), 1.01 (m, 3H, CH₃CH₂CH₂).

### Preparation 3

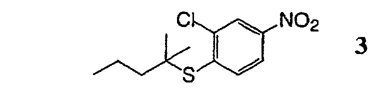

A mixture of S-(1,1-dimethylbutyl)thiouronium 4-toluene-sulfonate (prepared as described: Evans, M. B.et al., *J. Chem. Soc*., (1962), p. 5045) (5.00 g, 15.0 mmol), KOH (2.10 g, 37.5 mmol) and concentrated NH₃ (1 drop) in EtOH (20 ml) was refluxed for 1 h. To the reaction mixture was added 3-chloro-4-fluoronitrobenzene in EtOH (10 ml). The mixture was refluxed for 0.5 h, allowed to cool and poured into cold water. The whole was extracted with CH₂Cl₂ (3x100 ml). The combined organic layers were dried (Na₂SO₄), filtered and concentrated to afford **3** (84%) which was used without further purification. ¹H NMR (CDCl₃, 400 MHz) δ 8.35 (d, 1H, J=2.4 Hz, ArH), 8.09 (dd, 1H, J = 2.4, 8.5 Hz, ArH), 7.78 (d, 1H, J=8.5 Hz, ArH), 1.62 (m, 4H, CH₃CH₂CH₂), 1.40 (s, 6H, C(CH₃)₂S), 0.98 (m, 3H, CH₃CH₂CH₂).

### Preparation 4

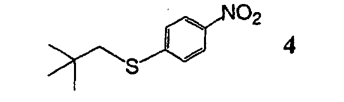

A mixture of 4-nitrothiophenol (500 mg, 3.22 mmol), 1-iodo-2,2-dimethylpropane (0.43 ml, 4.8 mmol), and NaH (80%, 97 mg, 3.2 mmol) in DMF (10 ml) was stirred for 3 days. The reaction mixture was poured into H₂O and the whole was extracted with CH₂Cl₂. The combined organic layers were dried (Na₂SO₄), filtered and evaporated. The residue was subjected to flash chromatography (1:50 EtOAc/hexanes) to afford **4** (190 mg, 26%) as a solid. ¹H NMR (CDCl₃, 400 MHz) δ 8.07 (m, 2H, ArH), 7.31 (m, 2H, ArH), 2.93 (s, 2H, CH₂S), 1.05 (s, 9H, (CH₃)₃).

### Preparation 5

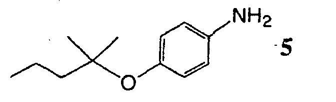

### Step 1:

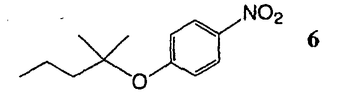

To an ice-cold solution of 2-methyl-2-pentanol (1.5 ml, 15 mmol) in DMF (20 ml) was added NaH (60% dispersion in mineral oil, 600 mg, 15 mmol) under a N₂ atmosphere. The cold bath was removed and the suspension was stirred for 4h. The mixture was cooled in an ice bath, 4-fluoronitrobenzene (1.1 ml, 10 mmol) was added in one portion, and the reaction mixture was stirred at RT. After 18 h, the reaction mixture was poured onto an ice-water slurry (300 ml) and extracted with Et₂O (3x200 ml). The combined Et₂O extracts were washed with H₂O (6x200 ml) and saturated NaCI, dried (MgSO₄), filtered and evaporated to afford an oil (2.4 g). Flash chromatography (3:1 hexanes/CH₂Cl₂) of the crude product gave **6** (1.50 g, 67%) as an oil. ¹H NMR (CDCl₃, 400 MHz) δ 8.22 (2H, m, ArH), 7.09 (2H, m, ArH), 1.77 (2H, m, -OC(CH₃)₂CH₂-), 1.52 (2H, m, -CH₂CH₃), 1.47 (6H, s, -OC(CH₃)₂-), 1.03 (3H, t, *J*= 7.3 Hz, -CH₂CH₃). MS (Cl) *m*/*e* 224 (M+H)⁺.
Step 2: A mixture of **6** (1.40 g, 6.3 mmol) and 10% Pd/C (0.14 g) in CH₃OH (40 ml) was stirred under a balloon of H₂. After 16 h, the catalyst was removed by filtration through celite, and the filter pad was washed with CH₃OH. The combined filtrate and washings were evaporated to afford **5** as an oil (1.21 g, 100%). ¹H NMR (CDCl₃, 400 MHz) δ 6.83 (2H, m, ArH), 6.66 (2H, m, ArH), 1.61 - 1.50 (4H, m, -CH₂CH₂-), 1.25 (6H, s, -OC(CH₃)₂-), 0.98 (3H, t, *J*= 7.1 Hz, -CH₂CH₃).

### Preparation 6

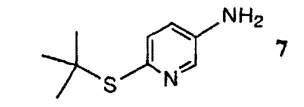

### Step 1:

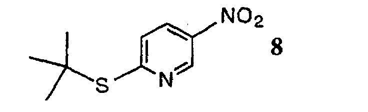

To a solution of 2-chloro-5-nitropyridine (1.00 g, 6.3 mmol) in EtOH (10 ml) was added a solution of potassium 2-methyl-2-propanethiolate prepared from 2-methyl-2-propanethiol (0.71ml, 6.3 mmol) and KOH (0.56 g, 10 mmol) in EtOH (10 ml). The reaction mixture was refluxed for 0.25 h, then cooled in ice. The solid was removed by filtration through celite and the filtrate was evaporated to a syrup, which was dissolved in CH₂Cl₂ (100 ml) and washed with sat'd NH₄Cl. The organic layer was dried (MgSO₄), filtered, and evaporated. Purification of the residue by flash chromatography (1:4 CH₂Cl₂/hexanes) gave **8** (0.80 g, 60%) as a waxy solid. ¹H NMR (CDCl₃, 400 MHz) δ 9.31 (1H, d, *J*= 2.8 Hz, ArH), 8.23 (1H, dd, *J* = 8.9, 2.8 Hz, ArH), 7.28 (1H, d,*J*= 8.9 Hz, ArH), 1.70 (9H, s, -S(CH₃)₃).
Step 2: To a solution of **8** (414 mg, 1.95 mmol) and NiCl₂.6H₂O (950 mg, 4.0 mmol) in CH₃OH (20 ml) was added NaBH₄ (301 mg, 8.0 mmol) in small portions. After 20 min. the reaction mixture was concentrated and the residue was purified by flash chromatography (3:97 CH₃OH/CH₂Cl₂) to give **7** (120 mg, 34%). ¹H NMR (CDCl₃, 400 MHz) δ 8.35 (1H, d, *J*= 2.4 Hz, ArH), 7.43 (1H, d, *J*= 8.3 Hz, ArH), 7.30 (1H, dd,*J* = 8.3, 2.4 Hz, ArH), 6.9 (2H, bs, NH₂), 1.43 (9H, s, -S(CH₃)₃).

### Preparation 7

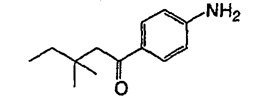

Step 1: A mixture of 3,3-dimethylpentanoic acid (11.00 g, 84.0 mmol; Synthesis (1985), 493) and SOCl₂ (80.0 g, 678 mmol) was refluxed for 2 h. The reaction mixture was concentrated *in vacuo* to afford the acid chloride as a colorless oil (10.0 g, 80%). ¹H NMR (CDCl₃, 400 MHz) δ 2.83 (2H, s, CH₂CO), 1.39 (2H, q, *J* = 7.3 Hz, CH₃CH₂), 1.02 (6H, s, C(CH₃)₂), 0.86 (3H, t, *J* = 7.3 Hz, CH₃CH₂).

### Step 2:

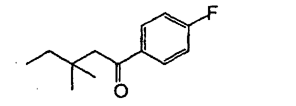

To an ice-cold solution of the product of Step 1 (6.00 g, 41.0 mmol) in dry Et₂O (40 ml) was slowly added 1.0 M 4-fluorophenylmagnesium bromide in THF (37 ml, 37 mmol). The reaction mixture was stirred at 0 °C for 3 h, then poured into 1N HCI solution (100 ml). The whole was extracted with EtOAc (3x100 ml) and the combined organic layers were dried (Na₂SO₄), filtered and evaporated. Purification of the residue by flash chromatography (hexane) afforded the product (7.00 g, 82%) as colorless oil. ¹H NMR (CDCl₃, 400 MHz) δ 7.90 (2H, m, ArH), 7.05 (2H, m, ArH), 2.80 (2H, s, CH₂CO), 1.4 (2H, q, *J*= 8.0 Hz, CH₃CH₂), 0.87 (6H, s, (CH₃)₂C), 0.85 (3H, t, *J* = 7.6 Hz, CH₃CH₂). MS (ES) *m*/*e* 209 (M+H)⁺. Step 3: To a solution of the product of Step 2 (2.00 g, 9.60 mmol) in DMSO (20.0 ml) in a sealed tube was added NaN₃ (6.24 g, 96.0 mmol). The vigorously stirred reaction mixture was heated at 140 °C for 5 days, then allowed to cool to RT and poured into 1N NaOH (100 ml). The whole was extracted with EtOAc (3x100 ml). The combined organic layers were dried (Na₂SO₄), filtered, and concentrated. Flash chromatography of the residue (1:4 EtOAc/hexane) afforded Preparation 7 (0.66g, 33%) as a light brown oil. ¹H NMR (CDCl₃, 400 MHz) δ 7.80 (2H, d, *J*= 8.8 Hz, ArH), 6.70 (2H, d, *J*= 8.8 Hz, ArH), 2.74 (2H, s, CH₂CO), 1.40 (2H, q, *J* = 7.6 Hz, CH₂CH₃), 0.98 (6H, s, (CH₃)₂C), 0.86 (3H, t, *J*= 7.6 Hz, CH₃CH₂). MS (FAB) *m*/*e* 206 (M+H)⁺.

### Preparation 8

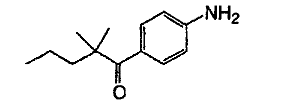

Using 2,2-dimethylpentanoic acid as the starting material and the three-step procedure described for Preparation 7, the title compound was prepared: ¹H NMR (CDCl₃, 400 MHz) δ 7.76 (2H, m), 6.61 (2H, m), 4.05 (2H, bs), 1.76 (2H, m), 1.30 (6H, s), 1.20 (4H, m), 0.83 (3H, t, J= 7.8 Hz). MS *m*/*e* 206 (M+H)⁺.

### Preparation 9

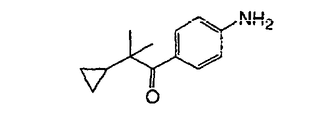

### Step 1:

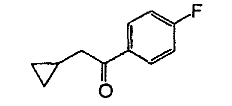

To an ice-cold solution of cyclopropylacetonitrile (4.7 g, 58 mmol) in anhydrous Et₂O (30ml) was added 2M 4-fluorophenylmagnesium bromide in Et₂O (25 ml, 50 mmol), and the reaction mixture was stirred at 0 °C for 2 h. The reaction mixture was allowed to warm to RT and stirred for an additional 2 h. The pH was adjusted to 3 by addition of 1 N HCI and the whole was extracted with Et₂O (4x50 ml). The combined Et₂O layers were washed with saturated Na₂CO₃ and NaCI, dried (MgSO₄), filtered and concentrated. Flash chromatography (2:98 EtOAc/hexane) of the residue afforded the product (5.02g, 56%). ¹H NMR (CDCl₃, 400 MHz) δ 7.98(2H, m), 7.13 (2H, t), 2.85 (2H, d), 1.15 (1H, m), 0.61 (2H, m), 0.19 (2H, m). MS *m*/*e* 179 (M+H)⁺.

### Step 2:

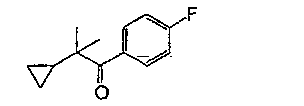

To a stirred, ice-cold solution of the product of Step 1 (5.0 g, 28 mmol) in anhydrous THF (100 ml) under N₂ was added KH (16.0 g, 35% in mineral oil, 140 mmol), and the reaction mixture was stirred for 0.5 h. Then CH₃I (16 ml, 280 mmol) was added dropwise to the ice-cold reaction mixture. After stirring at RT for 4h, the reaction mixture was cooled in an ice-bath and sat'd NH₄Cl was cautiously added. The whole was extracted with EtOAc (3x100 ml), washed with H₂O and sat'd NaCl, dried (MgSO₄), filtered, and concentrated. Flash chromatography (hexanes, then 1:9 EtOAc/hexanes) afforded the product (3.96 g, 68%). ¹H NMR (CDCl₃, 400 MHz) δ 7.84 (2H, m), 7.07 (2H, m), 1.14 (6H, s), 1.13 (1H, m), 0.51 (2H, m), 0.42 (2H, m).
Step 3: Using the procedure of Preparation 7, Step 3, reaction of the product of Step 2 with NaN₃ afforded Preparation 9. ¹H NMR (CDCl₃, 400 MHz) δ 7.86 (2H, m), 6.67 (2H, m), 4.42 (2H, bs), 1.16 (1H, m), 1.15 (6H, s), 0.49 (2H, m), 0.42 (2H, m).

### Preparation 10

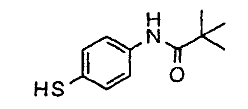

### Step 1:

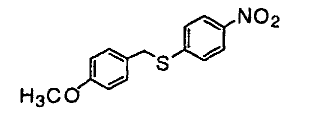

A mixture of 4-fluoronitrobenzene (10.0 g, 70.9 mmol), 4-methoxybenzyl-mercaptan (14.8 mL, 106 mmol), and K₂CO₃ (19.6 g, 142 mmol) in acetone (150 mL) was refluxed for 4h. The cooled reaction mixture was poured into H₂O and extracted with CH₂Cl₂ (3x100 mL). The combined organic extracts were dried (Na₂SO₄), filtered, and concentrated to afford an oil (17.1 g, 87%) that was used without further purification. ¹H NMR (CDCl₃, 400 MHz) δ 8.18 (m, 2H, ArH), 7.38 (m, 4H, ArH), 6.94 (m, 2H, ArH), 4.28 (s, 2H, -CH₂-), 3.87 (s, 3H, CH₃O-).
Step 2: Reduction of the product from Step 1 (17 g, 62 mmol) with NiCl₂.6H₂O/ NaBH₄ according to the procedure of Preparation 6, step 2, gave the product aniline (6.67 g, 44%). ¹H Nmr (CDCl₃, 400 MHz) δ 7.18 (m, 4H, ArH), 6.82 (m, 2H, ArH), 6.61 (m, 2H, ArH), 3.95 (s, 2H, -CH₂-), 3.85 (s, 3H, CH₃O-).

### Step 3:

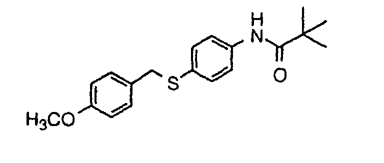

A mixture of the product of Step 2 (6.67 g, 27.2 mmol), trimethylacetyl chloride (5.0 mL, 40 mmol), and DMAP (6.64 g, 54.4 mmol) in CH₂Cl₂ (100 mL) was stirred for 0.3 h. Then CH₂Cl₂ (200 mL) was added and the mixture was washed with 1 M HCl. The organic layer was dried (Na₂SO₄), filtered, and evaporated. Recrystallization of the residue from Et₂O/hexane/CH₂Cl₂ afforded the product (4.6 g, 51%) as a white solid. MS (Cl) *m*/*e* 330 (M+H)⁺.
Step 4: To a stirred, ice-cold mixture of the product of Step 3 (500 mg, 1.46 mmol) in CH₂Cl₂ (25 mL), was added CF₃COOH (6 mL) and Hg(OAc)₂ (465 mg, 1.46 mmol). After 1.3 h, the reaction mixture was poured into H₂O, aqueous Na₂S was added, and the mixture was extracted with 1:2 EtOAc/hexanes. The organic layer was dried (Na₂SO₄), filtered, and evaporated. The residue was subjected to flash chromatography (1:2 EtOAc/hexanes) to give the product (305 mg, 100%). ¹H NMR (CDCl₃, 400 MHz) δ 7.51 (m, 2H, ArH), 7.33 (m, 2H, ArH), 1.49 (s, 3H, (CH₃)₃-).

### Example 1

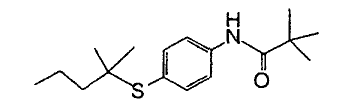

To a mixture of Preparation 1 (10.00 g, 47.7 mmol) and pyridine (7.70 ml, 95.5 mmol) in CH₂Cl₂ (100 ml) was added trimethylacetyl chloride (8.80 ml, 71.6 mmol). The reaction mixture was stirred at RT for 0.5 h, then poured into 2 M HCI (100 ml). The mixture was extracted with CH₂Cl₂ (3x100 ml), dried (Na₂SO₄), filtered, and concentrated. The residue was subjected to flash column chromatography (1:10 EtOAc/hexanes) to afford the title compound (76%). ¹H NMR (CDCl₃, 400 M Hz) δ 7.50-7.39 (m, 4H, ArH), 7.32 (S, 1H, NH), 1.50-1.35 (m, 4H, CH₂CH₂), 1.29 (s, 9H, (CH₃)₃C), 1.17 (s, 6H, (CH₃)₂C), 0.88 (t, 3H, CH₃CH₂). MS (Cl) *m*/*e* 294 (M+H)⁺.

Using appropriate starting materials and essentially the same procedure the following compounds can be prepared (Table 1).

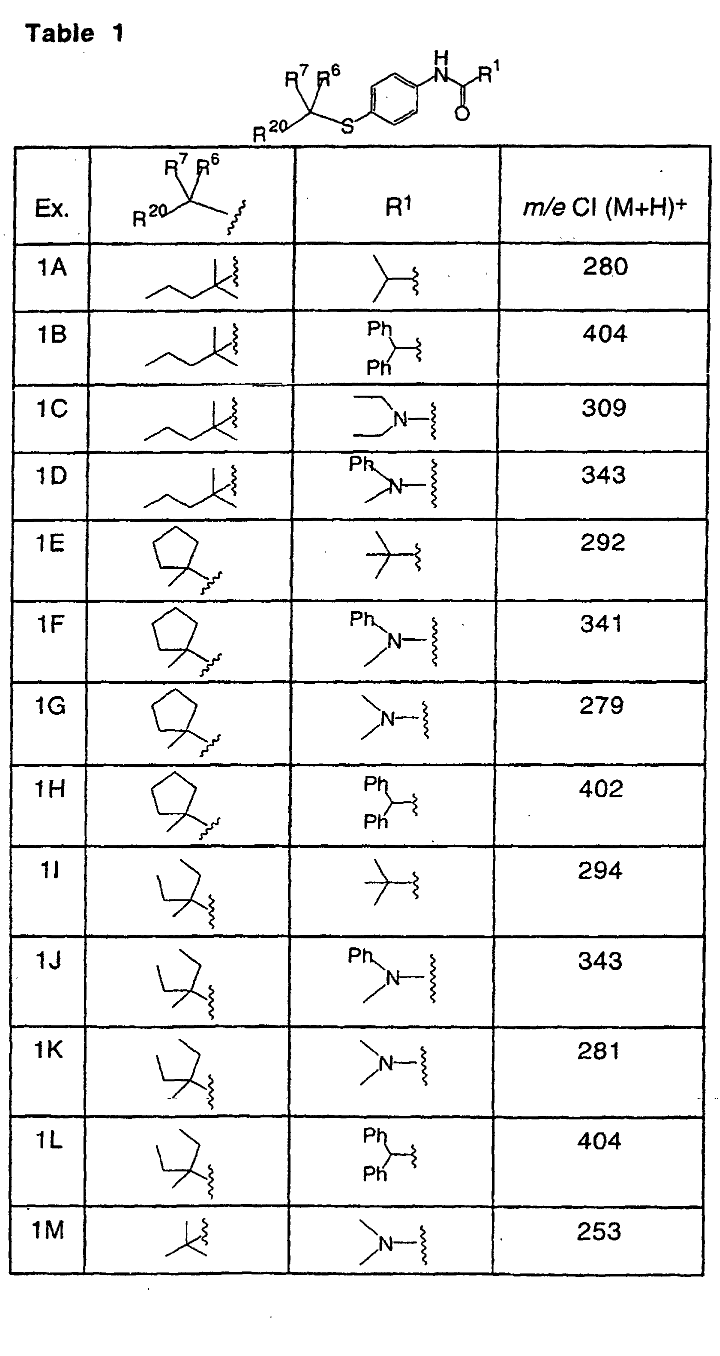

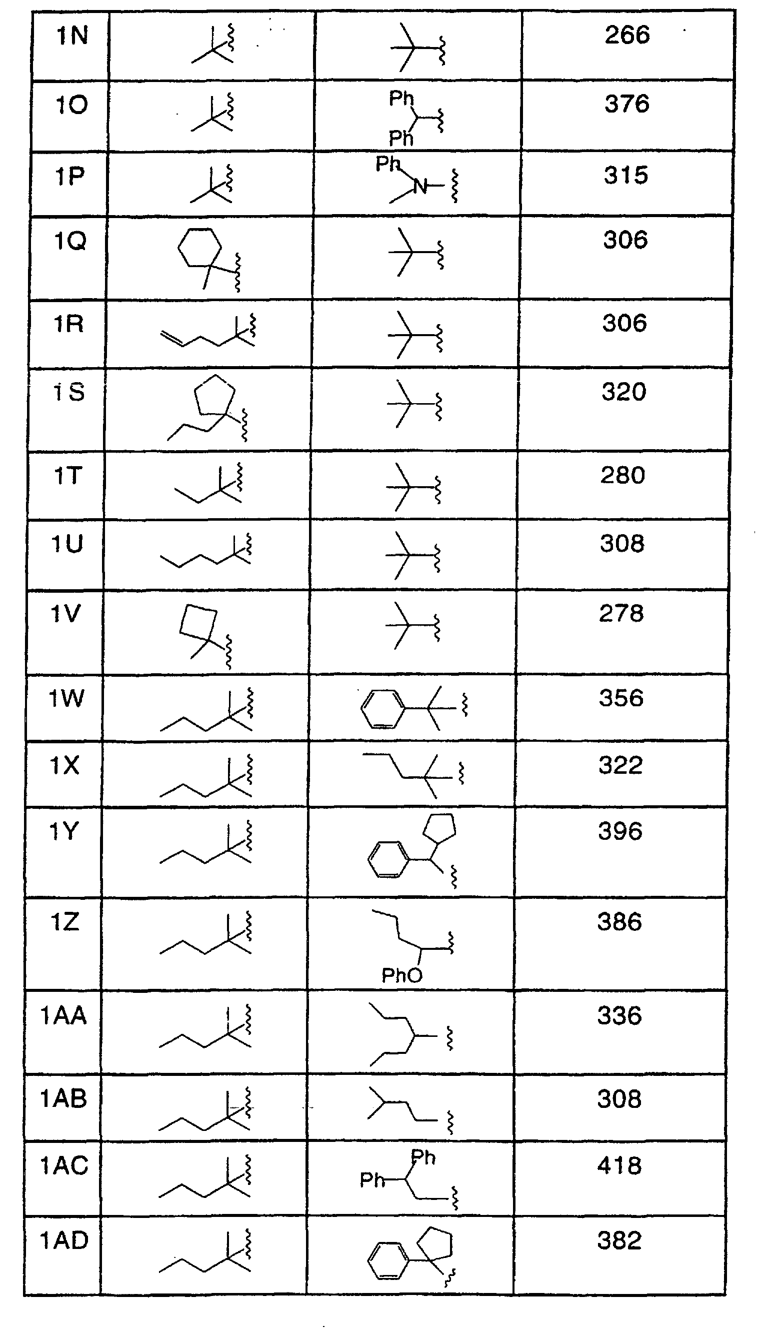

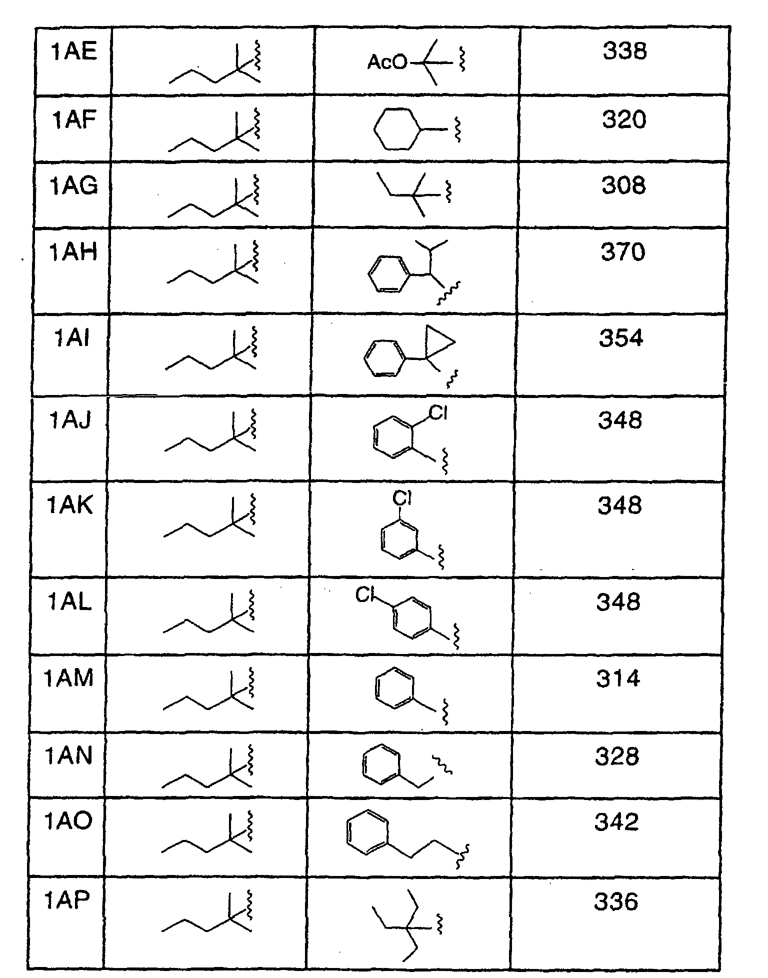

### Example 2

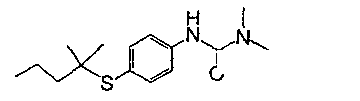

A mixture of Preparation 1 (1.03 g, 4.92 mmol), Et₃N (3.40 ml, 24.6 mmol) and triphosgene (0.585 g, 1.97 mmol) in toluene (60 ml) was refluxed for 2 h, then allowed to cool to RT. (CH₃)₂NH (2.0 M in THF) (4.90 ml, 9.84 mmol) was added. The reaction mixture was allowed to stir at RT for 1.5 h, then poured into cold water. The whole was extracted with CH₂Cl₂ (3x100 ml), dried (Na₂SO₄), filtered and concentrated. Purification of the residue by flash chromatography (1:1 EtOAc/hexanes) afforded the title compound (1.03 g, 74%) as a white solid. ¹H NMR (CDCl₃, 400 M Hz) δ 7.43 (m, 4H, ArH), 6.39 (s, 1H, NHCO), 3.07 (s, 6H, N(CH₃)₃), 1.45 (m, 4H, CH₂CH₂CMe₂S), 1.23 (s, 6H, (CH₃)₂CS), 0.93 (t, 3H, J=6.88 Hz, CH₃CH₂). MS (Cl) *m*/*e* 281 (M+H)⁺.

Using appropriate starting materials and essentially the same procedure the following compounds can be prepared (Table 2).

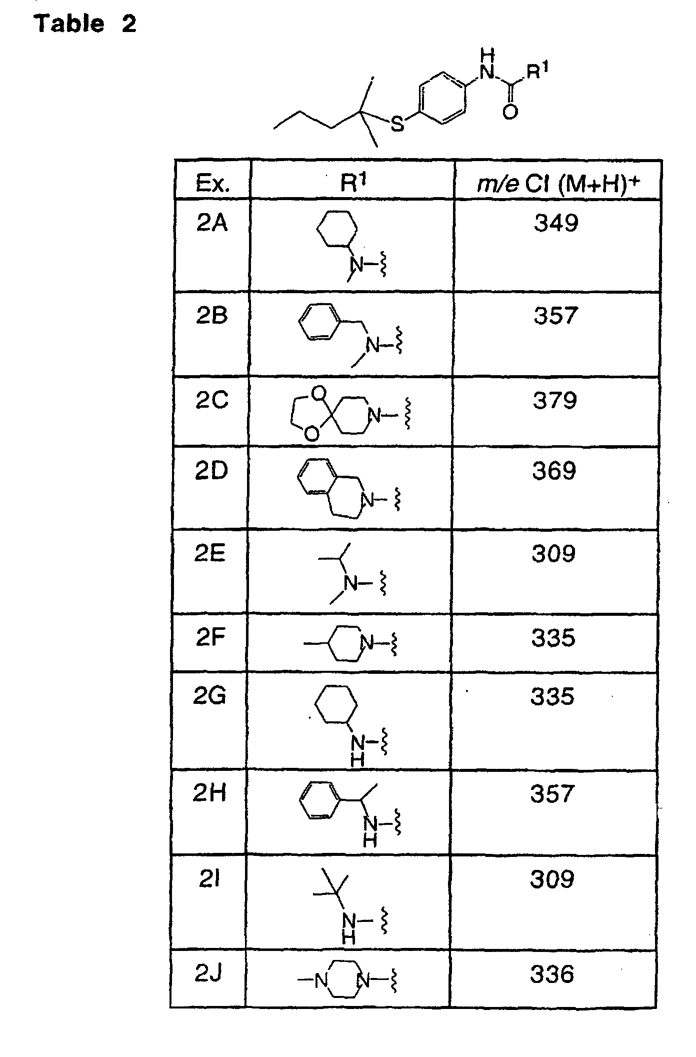

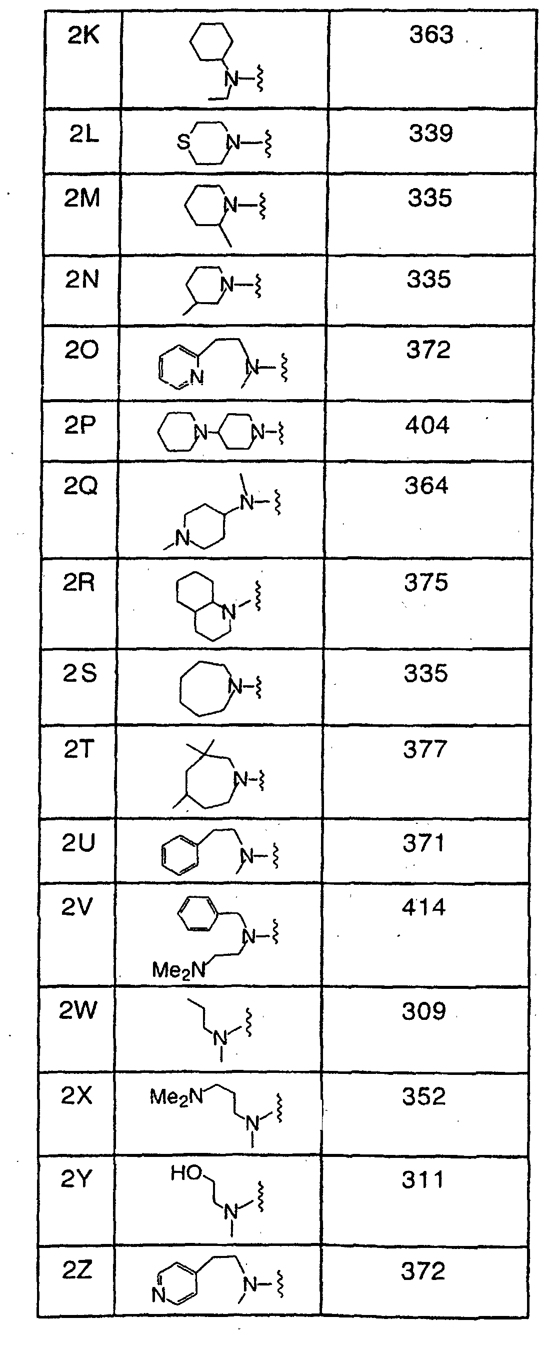

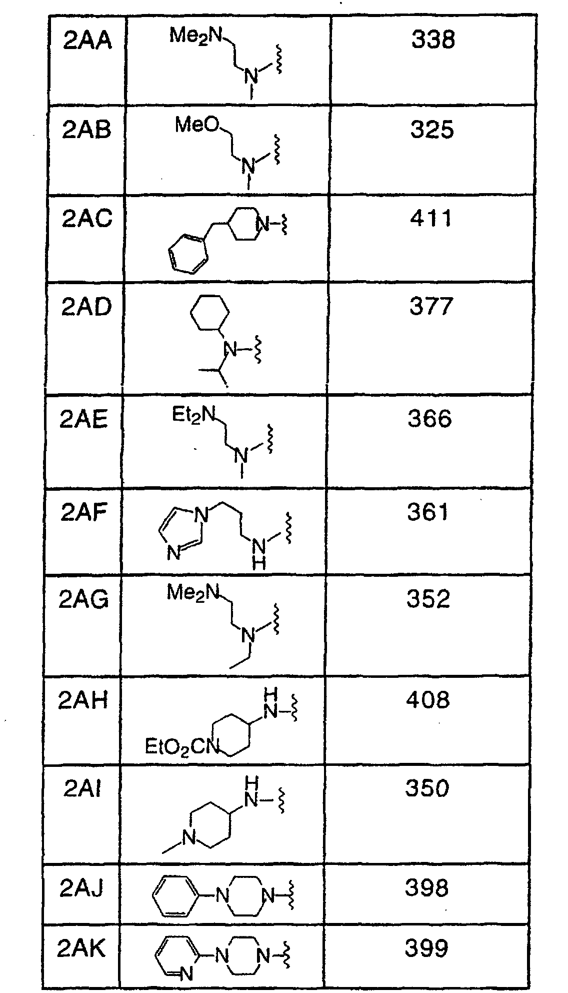

### Example 3

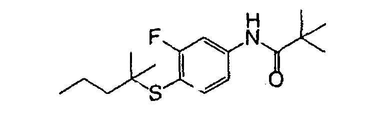

To an ice-cold mixture of Preparation 2 (2.32 g, 9.03 mmol) and NiCl₂•6H₂O (4.83 g, 20.3 mmol) in CH₃OH (100 ml) was added NaBH₄ (1.53 g, 40.6 mmol) in portions. After 1.5 h, the reaction mixture was poured into water and the whole was extracted with CH₂Cl₂ (3x100 ml). The combined organic extracts were dried (Na₂SO₄), filtered, and concentrated in vacuo to give the desired 3-fluoro-4-(1',1'-dimethylbutylthio)aniline (56%). A mixture of the aniline (60 mg, 0.264 mmol), pyridine (0.11 ml, 1.32 mmol), and (CH₃)₃CCOCl (0.065 ml, 0.528 mmol) in CH₂Cl₂ (1.0 ml), was stirred overnight, then subjected to plc (1:6 EtOAc/hexanes) to give the title compound (41%). ¹H NMR (CDCl₃, 400 M Hz) δ 7.65 (m, 1H, ArH), 7.45 (m, 2H, ArH & NH), 7.20 (m, 1H, ArH), 1.52(m, 4H, CH₃CH₂CH₂), 1.37(s, 9H, C(CH₃)₃), 1.27(s, 6H, C(CH₃)₂S), 0.96 (t, 3H, J=6.8 Hz, CH₃CH₂CH₂). MS (Cl) *m*/*e* 312 (M+H)⁺.
Similarly prepared were compounds of the formula:

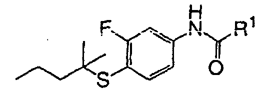

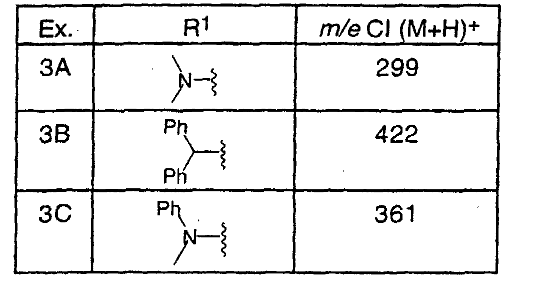

Using the compound of Preparation 3 and the appropriate acid chloride or carbamyl chloride, the procedure of Example 3 afforded the following compounds:

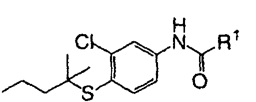

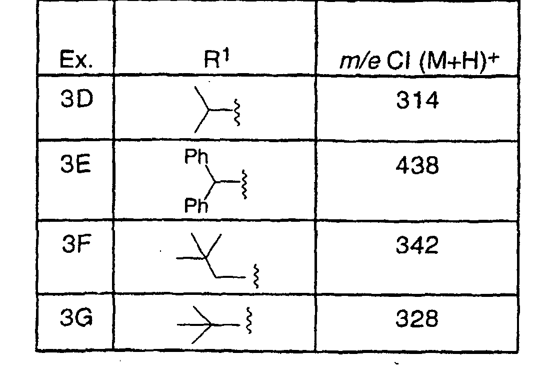

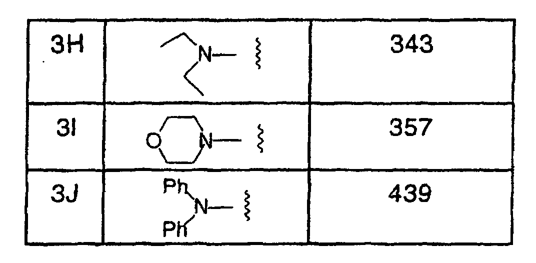

### Example 4

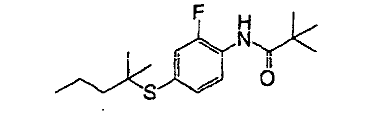

Step 1: A mixture of 2,4-difluoronitrobenzene (2.6 ml, 23.3 mmol), *p*-methoxybenzyl mercaptan (2.00 g, 11.7 mmol), K₂CO₃ (6.47 g, 46.8 mmol) in acetone (50 ml) was refluxed for 20 h. The reaction mixture was poured into cold water. The whole was extracted with CH₂Cl₂ (3x100 ml), and the combined organic layers were dried (Na₂SO₄), filtered, and concentrated. The residue was purified by flash chromatography (1:30→1:20 EtOAc/hexanes) to give the desired 2-fluoro-4-(4'-methoxybenzylmercapto)nitrobenzene containing a small amount of 4-fluoro-2-(4'-methoxybenzylmercapto)nitrobenzene.
MS (Cl) *m*/*e* 294 (M+H)⁺.
Step 2: To a vigorously stirred ice-cold mixture of 2-fluoro-4-(4'-methoxy-benzylmercapto)nitrobenzene and NiCl₂•6H₂O (6.08 g, 25.6 mmol) in CH₃OH was added NaBH₄ (1.93 g, 51.1 mmol) in portions.
The reaction mixture was stirred for 1 h at 0 °C, then poured into cold water. The whole was extracted with CH₂Cl₂ (3x100 ml) and the combined organic layers were dried (Na₂SO₄), filtered, and evaporated to give 2-fluoro-4-(4'-methoxybenzylmercapto)aniline.
MS (Cl) *m*/*e* 264 (M+H)⁺.
Step 3: A mixture of the product of step 2, pyridine (3.1 ml, 38.4 mmol) and (CH₃)₃CCOCl (3.2 ml, 25.6 mmol) in CH₂Cl₂ (100 ml) was stirred for 2 h, then poured into cold water. The whole was extracted with CH₂Cl₂ (3x100 ml) and the combined organic layers were dried (Na₂SO₄), filtered and concentrated to afford 2-fluoro-4-(4'-methoxybenzylmercapto)phenyl-2,2-dimethyipropanamide. MS (Cl) *m*/*e* 348 (M+H)⁺.
Step 4: A solution of the product of Step 3 in CF₃CO₂H (20 ml) was heated at 80 °C for 28 h, then concentrated. The residue (963 mg) was dissolved in Et₂O (2 ml). 2-Methyl-1-pentene (2.0 ml) and concentrated H₂SO₄ (0.5 ml) were added with stirring. After 20 min. the reaction mixture was poured into CH₂Cl₂ (200 ml), and washed with water and sat'd NaCl. The organic layer was dried (Na₂SO₄), filtered and concentrated. The residue was purified by plc (1:10 EtOAc/hexanes) to afford the title compound in 16% overall yield starting from 2,4-difluoronitrobenzene. ¹H NMR (CDCl₃, 400 MHz) δ 8.37 (t, 1H, J=8.5 Hz, ArH), 7.71 (s, 1H, NH), 7.30 (m, 2H, ArH), 1.50 (m, 4H, CH₃CH₂CH₂), 1.37 (s, 9H, C(CH₃)₃), 1.25 (s, 6H, C(CH₃)₂S), 0.95 (t, 3H, J=7.0 Hz, CH₃CH₂CH₂). MS(CI) *m*/*e* 312 (M+H)⁺.

### Example 5

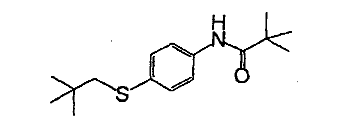

To a refluxing suspension of FeSO₄.7H₂O (3.95 g, 14.2 mmol) and Fe powder (397 mg, 7.1 mmol) in 1:1 H₂O/EtOH (100 ml) was added a hot solution of Preparation 4 (319 mg, 1.42 mmol) in EtOH (10 ml). The suspension was refluxed for 6h, allowed to cool, and filtered through celite. The filtrate was extracted with CH₂Cl₂ and the organic extract was dried (Na₂SO₄), filtered, and concentrated to afford the aniline (185 mg). A portion of the aniline (30 mg, 0.15 mmol), trimethylacetyl chloride (47 mg, 0.38 mmol), pyridine (62 mg, 0.77 mmol), and 4-dimethylaminopyridine (19 mg, 0.15 mmol) in CH₂Cl₂ (2 ml) was stirred overnight. The reaction mixture was subjected to flash chromatography (1:10 EtOAc/hexanes) to afford the title compound (37 mg, 95%) as white solid. MS (CI) *m*/*e* 280 (M+H)⁺.

### Example 6

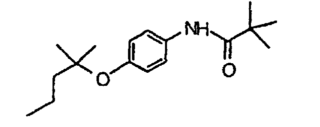

Using the procedure of Example 1, the reaction of Preparation 5 (97 mg, 0.5 mmol) and trimethylacetyl chloride (0.12 ml, 1.0 mmol) afforded the title compound (137 mg, 99%) as a white solid. ¹H NMR (CDCl₃, 400 MHz) δ 7.43 (2H, m, ArH), 6.97 (2H, m, ArH), 1.64 - 1.48 (4H, m, -CH₂CH₂-), 1.34 (9H, s, -C(CH₃)₃), 1.28 (6H, s, -OC(CH₃)₂-), 0.97 (3H, t, *J*= 7.1 Hz, -CH₂CH₃). MS (Cl) *m*/*e* 278 (M+H)⁺.

Using appropriate starting materials and essentially the same procedure, the following compounds can be prepared:

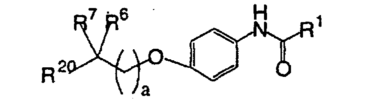

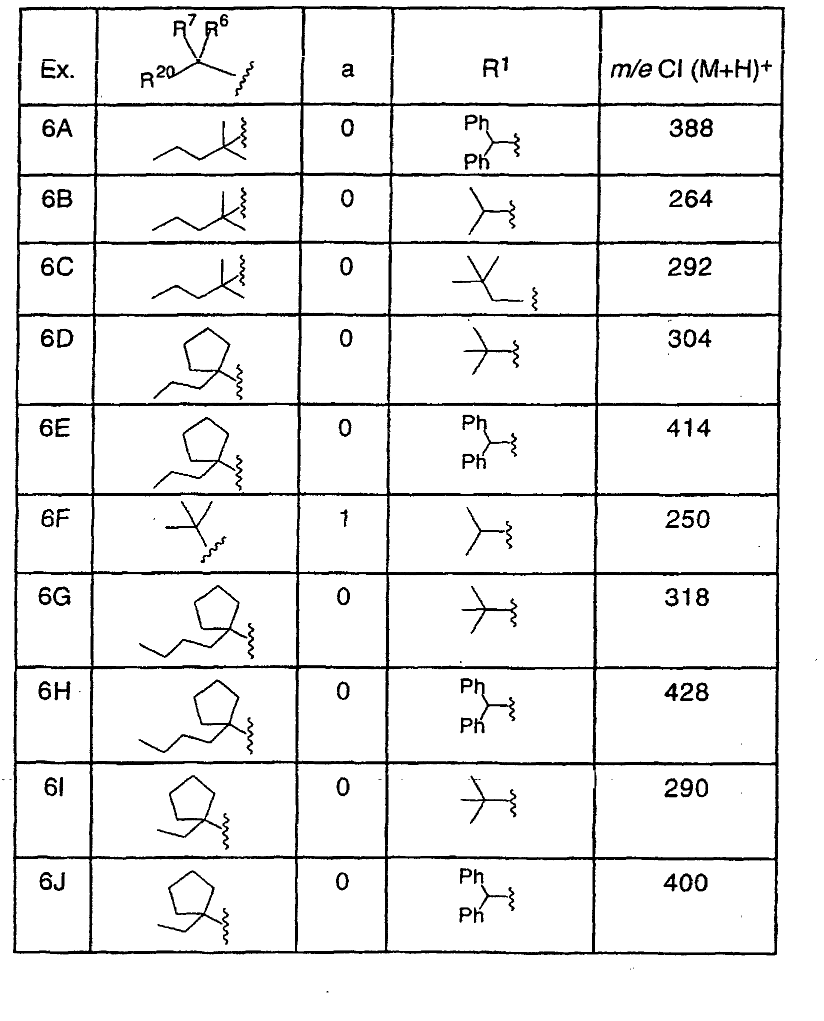

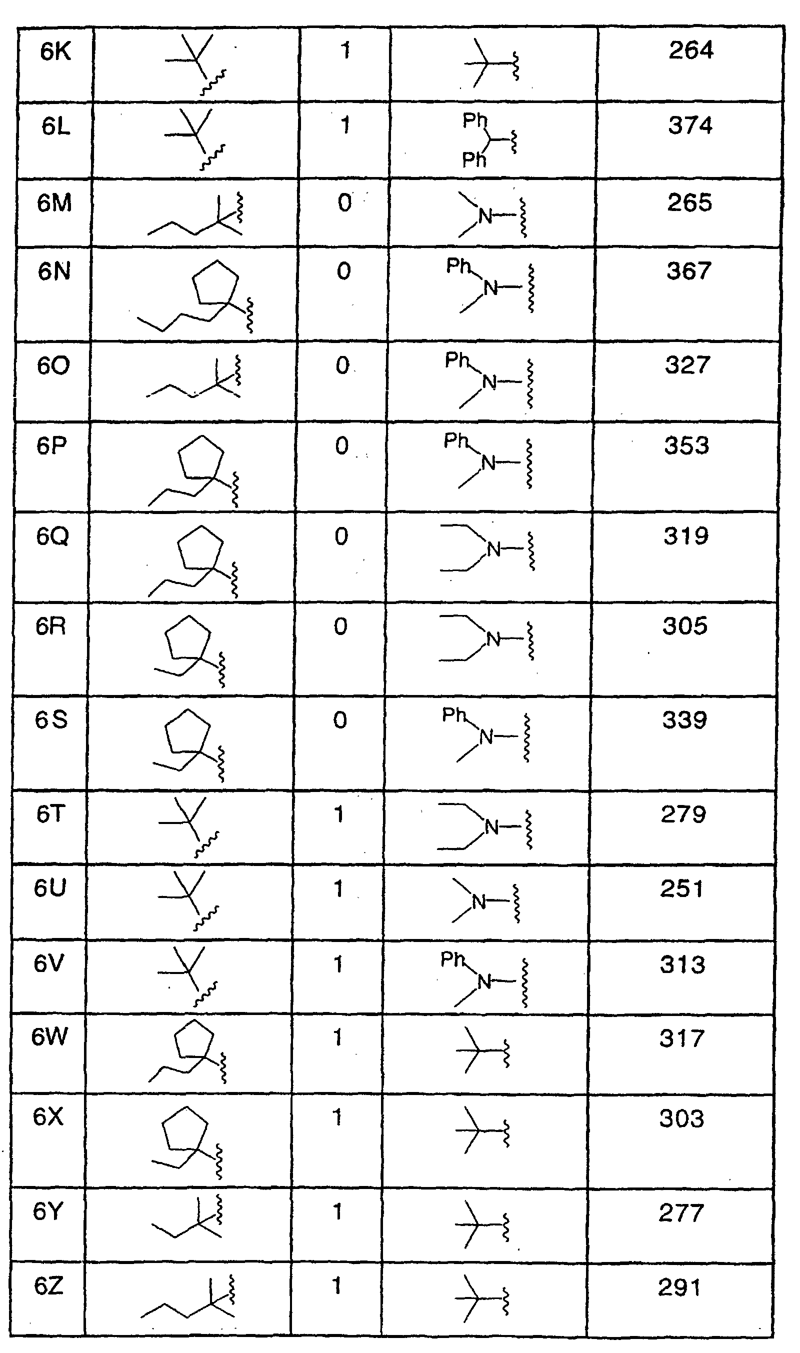

The following compounds can also be prepared using appropriate starting materials and similar methods:

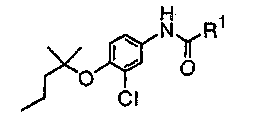

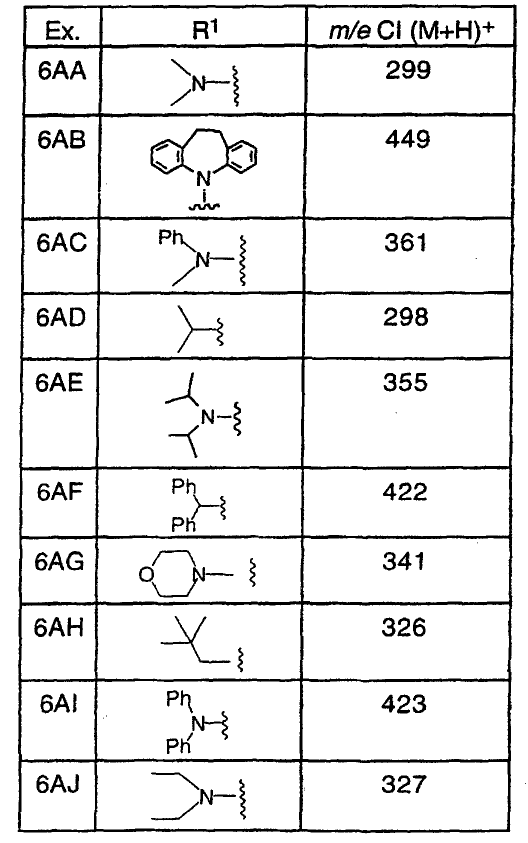

### Example 7

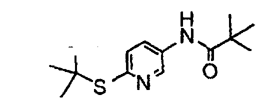

Using the procedure of Example 1, the reaction of the product of Preparation 6 (21 mg, 0.11 mmol) and trimethylacetyl chloride (25 µl, 0.20 mmol) afforded the title compound (20 mg, 65%) as a white solid. ¹H NMR (CDCl₃, 400 MHz) δ 8.59 (1H, s, ArH), 8.17 (1H, d, *J*=8.6 Hz, ArH), 7.47 (1H, d,*J* = 8.6 Hz, ArH), 1.49 (9H, s, -CO(CH₃)₃), 1.30 (9H, s, -S(CH₃)₃). MS (CI) *m*/*e* 267 (M+H)⁺.

Using appropriate starting materials and essentially the same procedure the following compounds can be prepared:
7A) 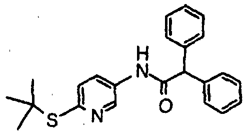 MS (Cl) *m*/*e* 377 (M+H)⁺.
7B) 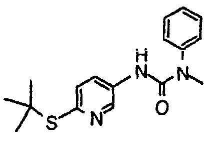 MS (CI) *m*/*e* 316 (M+H)⁺.

### Example 8

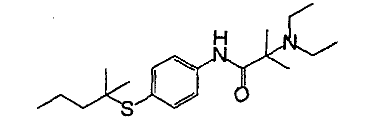

### Step 1:

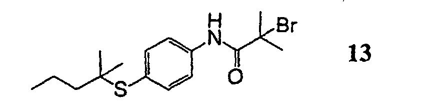

To a mixture of Preparation **1** (2.00 g, 9.55 mmol) and pyridine (1.50 ml, 19.1 mmol) in CH₂Cl₂ (100 ml) was added 2-bromoisobutyryl bromide (1.80 ml, 14.3 mmol). The reaction mixture was stirred at RT for 15 min, then poured into 1N HCl and extracted with CH₂Cl₂ (3x100 ml). The combined organic layers were dried (Na₂SO₄), filtered, and concentrated. The residue was subjected to flash chromatography (1:10 EtOAc/hexanes) to afford **13** (99%) as a white solid. ¹H NMR (CDCl₃, 400 MHz) δ 8.55 (s, 1H, NHCO), 7.55 (m, 4H, ArH), 2.10 (s, 6H, (CH₃)₂CBr), 1.51 (m, CH₂CH₂), 1.26 (s, 6H, (CH₃)₂CS), 0.96 (t, 3H, CH₃CH₂). MS (CI) *m*/*e* 358 (M+H)⁺.
Step 2: To a stirred solution of Et₂NH (58 µl, 0.558 mmol) in THF (2.0 ml) was added NaH (8.0 mg, 0.307 mmol), followed by **13** (100 mg, 0.279 mmol). The reaction mixture was stirred at RT for 30 min. and then subjected directly to plc (1:15 EtOAc/hexanes) to afford the title compound (61%). ¹H NMR (CDCl₃, 400 M Hz) δ 9.63 (s, 1H, NHCO), 7.52 (m, 4H, ArH), 2.62 (q, 4H, J=7.15 Hz, N(CH₂CH₃)₂), 1.48 (m, 4H, CH₂CH₂CMe₂S), 1.36 (s, 6H, (CH₃)₂CN), 1.24 (s, 6H, (CH₃)₂CS), 1.17 (t, 6H, J=7.09 Hz, N(CH₂CH₃)₂), 0.94 (t, 3H, J=6.88 Hz, CH₃(CH₂)₂CMe₂S). MS (Cl) *m*/*e* 351 (M+H)⁺.

Using the same procedure and the appropriate amine, the following compounds were also prepared:

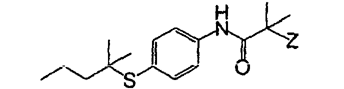

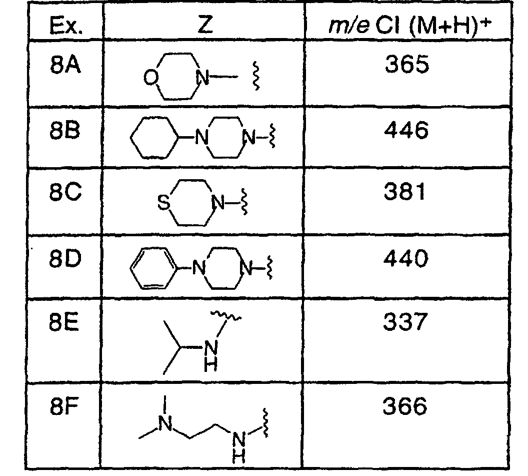

### Example 9

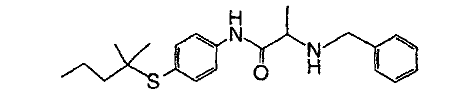

### Step 1:

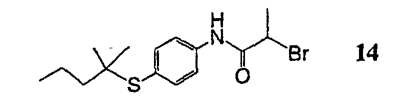

Using the product of Preparation **1** (210 mg, 1.0 mmol) and 2-bromopropionyl bromide (0.10 ml, 1.0 mmol) as starting materials, the procedure described for **13** (Example 8, Step 1) afforded **14** (218 mg, 64%). ¹H NMR (400 MHz, CDCl₃) δ 8.13 (1H, bs, NH), 7.55 (4H, m, ArH), 4.61 (1H, q, *J*= 7 Hz, -CHCH₃), 2.03 (3H, d, *J*=7 Hz, -CHCH₃), 1.60-1.45 (4H, m, CH₂CH₂CH₃), 1.26 (6H, s, (CH₃)₂C), 0.97 (t, 3H, *J*=7 Hz, CH₂CH₃).
Step 2: A mixture of **14** (102 mg, 0.30 mmol), benzylamine (65 µl, 0.59 mmol), and K₂CO₃ (83 mg, 0.60 mmol) in DMSO (1 ml) was stirred. After 2 h, H₂O (10 ml) was added and the whole was extracted with CH₂Cl₂.
The combined organic layers were washed with sat'd NaCl, dried (MgSO₄), filtered and evaporated. The residue was subjected to plc (1:1 EtOAc/ hexanes) to afford the title compound (70 mg, 62%) as a glass. ¹H NMR (400 MHz, CDCl₃) δ 9.60 (1H, bs, NH), 7.62 (2H, m, ArH), 7.52 (2H, m, ArH), 7.48-7.30 (5H, m, ArH), 3.90 (2H, s, CH₂Ph), 3.51 (1H, m, -CHCH₃), 1.60-1.45 (7H, m, -CHCH₃, CH₂CH₂CH₃), 1.26 (6H, s, (CH₃)₂C), 0.97 (t, 3H, *J*=7 Hz, CH₂CH₃). MS (CI) *m*/*e* 371 (M+H)+.

Using the appropriate amine and essentially the same procedures, the following compounds can also be prepared:

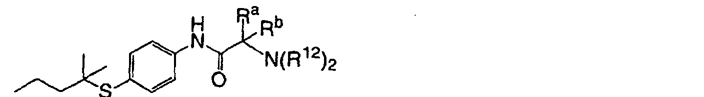

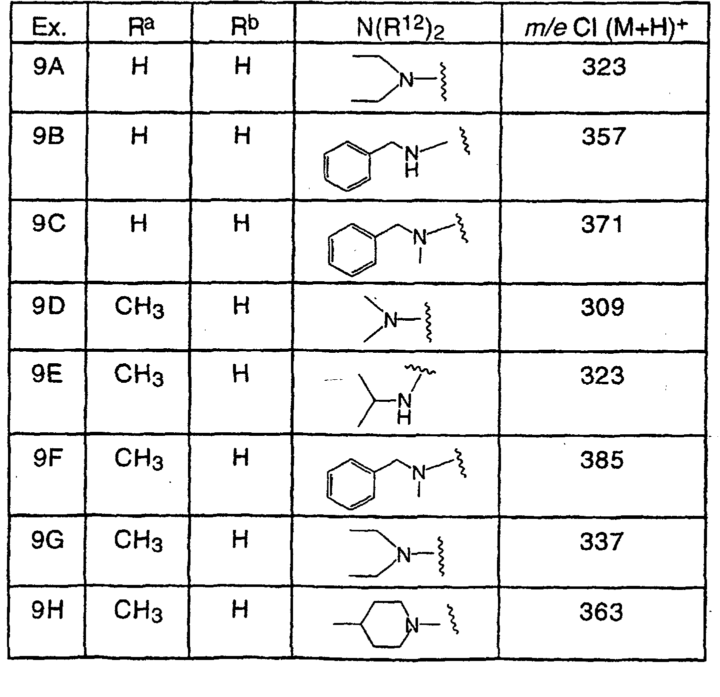

### Example 10

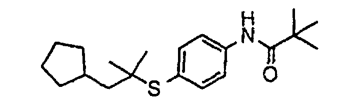

To a solution of Preparation 10 (50 mg, 0.24 mmol) and 3-cyclopentyl-2-methylprop-1-ene (59 mg, 0.48 mmol) in Et₂O (0.5 ml) was added conc. H₂SO₄ (26 µl, 0.48 mmol). The reaction mixture was stirred for 18 h, then subjected to plc (1:6 EtOAc/hexanes} to give the product (28 mg, 35%). ¹H NMR (CDCl₃, 400 MHz) δ 7.57 (m, 4H, ArH), 7.45 (s, 1H, NH), 2.13 (m, 1H, aliphatic H), 1.96 (m, 2H, aliphatic H), 1.62 (m, 4H, aliphatic H), 1.41 (s, 9H, (CH₃)₃C-), 1.31 (s, 6H, (CH₃)₂C), 1.19 (m, 4H, aliphatic). MS (Cl) *m*/*e* 334 (M+H)⁺.

Using appropriate starting materials and essentially the same procedure the following compounds are prepared.
10A) 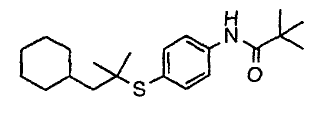 MS (CI) *m*/*e* 348 (M+H)⁺.
10B) 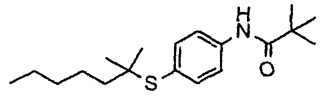 MS (CI) *m*/*e* 322 (M+H)⁺.

### Example 11

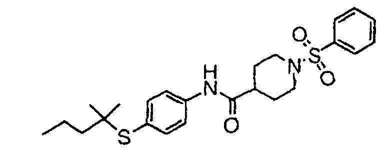

### Step 1:

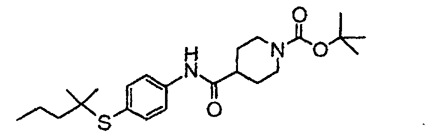

To a mixture of Preparation 1 (2.00 g, 9.56 mmol) and N-t-butoxycarbonylpiperidine-4-carboxylic acid (2.40 g, 10.5 mmol) in DMF (50 ml) was added DMAP (0.082 g, 0.67 mmol) and EDC (1.83 g, 11.6 mmol). The reaction mixture was stirred at RT for 16 h, then partitioned between H₂O (300 ml) and EtOAc (300 ml). The organic layer was washed with H₂O, dried (MgSO₄), filtered, and evaporated. The residue was subjected to flash chromatography (1:5 EtOAc/ hexanes) to afford the product (2.16 g, 54%) as a white solid. ¹H NMR (CDCl₃, 400 MHz) δ 7.50 (4H, m, ArH), 7.26 (1H, s, -NH), 4.23 (2H, m, -CH₂), 2.83 (2H, m, -CH₂), 2.42 (1H, m, -CH), 1.93 (2H, m, -CH₂), 1.78 (2H, m, -CH₂), 1.50 (9H, s, -C(CH₃)₃), 1.50-1.42 (4H, m, -(CH₂)₂-), 1.23 (6H, s, (CH₃)₂C-), 0.94 (3H, t, *J*= 7.3 Hz, CH₃).

### Step 2:

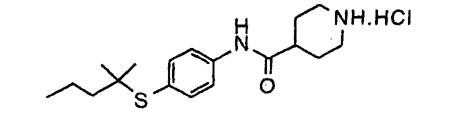

To the product of Step 1 (2.16 g, 5.1 mmol) was added 4M HCl in 1,4-dioxane (70 ml) and the reaction mixture was stirred for 0.5 h. The mixture was concentrated under reduced pressure to afford a white solid (1.80 g) that was used without further purification. ¹H NMR (CDCl₃ + CD₃OD, 400 MHz) δ 7.45 (4H, m, ArH), 3.32 (2H, m, -CH₂), 3.06 (2H, m, -CH₂), 2.71 (1H, m, -CH), 2.02 (2H, m, -CH₂), 1.91 (2H, m, -CH₂ 1.45-1.32 (4H, m, -(CH₂)₂-), 1.14 (6H, s, (CH₃)₂C-), 0.84 (3H, t, *J*= 7.3 Hz, CH₃).
Step 3: To a mixture of the product of Step 2 (0.10 g, 0.28 mmol) and Et₃N (0.06 ml, 0.43 mmol) in CH₂Cl₂ (1.5 ml) was added benzenesulfonyl chloride (63 mg, 0.36 mmol). The reaction mixture was stirred for 3 days, then diluted with CH₂Cl₂. The mixture was washed with 10% NH₄OH, 1M HCl, and saturated NaCl, then dried (MgSO₄), filtered and evaporated. The residue was subjected to plc (1:99 CH₃OH/CH₂Cl₂) to afford the product (90 mg, 70%) as a white solid. Anal. calcd for C₂₄H₃₂N₂O₃S₂: C, 62.58; H, 7.00; N, 6.08; S, 13.92. Found: C, 62.20; H, 7.05; N, 6.07; S, 13.72%. MS (FAB) *m*/*e* 461 (M+H)⁺.

Using the appropriate sulfonyl chloride starting material and the procedure of Step 3, the following compounds were prepared:

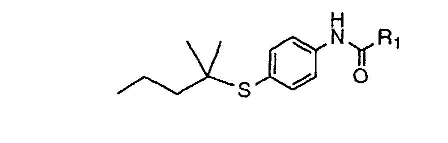

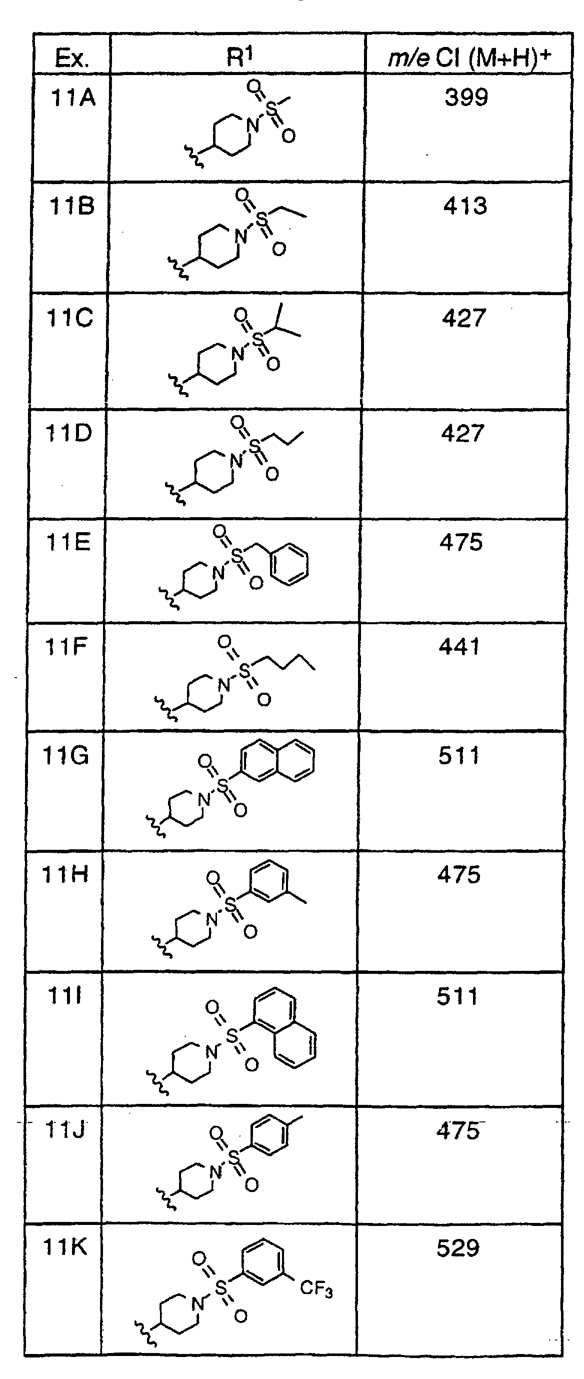

### Example 12

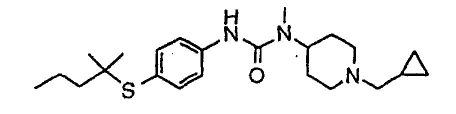

### Step 1:

To a mixture of N-t-butoxycarbonyl-4-piperidone (10.0 g, 0.050 mol) and aqueous CH₃NH₂ (40% w/w, 10 ml) in 1,2-dichloroethane (125 ml) was added NaBH(OAc)₃ (16.0 g, 0.075 mol). The reaction mixture was stirred overnight, then 1M NaOH (250 ml) was added and the whole was extracted with Et₂O (700 ml). The organic layer was washed with sat'd NaCl, dried (MgSO₄), filtered, and concentrated to give the product (10.5 g, 97%) as an oil that was used directly in Step 2. ¹H NMR (CDCl₃, 400 MHz) δ 4.09 (2H, m), 2.86 (2H, m), 2.55 (1H, m), 2.50 (3H, s), 1.90 (2H, m), 1.51 (9H, s), 1.30 (2H, m).

### Step 2:

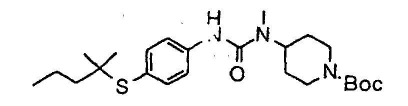

Using the procedure of Example 2, the product of Step 1, triphosgene, and the product of Preparation 1 were reacted to give the product. ¹H NMR (CDCl₃, 400 MHz) δ 7.32 (4H, m, ArH), 6.32 (1H, s, NH), 4.35 (1H, m, CH), 4.15 (2H, m, CH₂), 2.81 (3H, s, NCH₃), 2.73 (2H, m, CH₂), 1.65-1.32 (8H, m, GH₂x4), 1.90 (2H, m), 1.40 (9H, s, C(CH₃)₃), 1.13 (6H, s, (CH₃)₂), 0.83 (3H, t, *J*= 6.9 Hz, CH₃).

### Step 3:

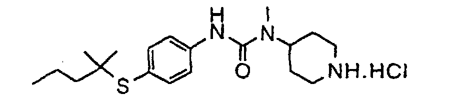

Using the procedure of Example 11, Step 2, the product of Step 2 was reacted with 4M HCl in 1,4-dioxane to give the product. ¹H NMR (CD₃OD, 400 MHz) δ 7.38 (4H, m, ArH), 4.38 (1H, m, CH), 3.50 (2H, m, CH₂), 3.12 (2H, m, CH₂), 2.96 (3H, s, NCH₃), 2.03 (2H, m, CH₂), 1.93 (2H, m, CH₂), 1.55-1.38 (4H, m, CH₂x2), 1.18 (6H, s, (CH₃)₂), 0.91 (3H, t, *J*= 7.2 Hz, CH₃).
Step 4: To a suspension of the product of Step 3 (200 mg, 0.52 mmol) and NaBH(OAc)₃ (155 mg, 0.73 mmol) in 1,2-dichloroethane (2.5 ml) was added cyclopropane carboxaldehyde (0.12 ml, 1.6 mmol). After stirring for 16 h, the reaction mixture was added to 1M NaOH (10 ml) and extracted with CH₂Cl₂ (20 ml). The organic layer was dried (MgSO₄), filtered and evaporated. The residue was subjected to plc (1:9 CH₃OH/CH₂Cl₂ saturated with conc. NH₄OH) to afford the product (166 mg, 79%) as a white solid. Anal. calcd for C₂₃H₃₇N₃OS: C, 68.44; H, 9.24; N, 10.41; S, 7.94. Found: C, 68.09; H, 9.18; N, 10.36; S, 7.56%. MS (Cl) *m*/*e* 404 (M+H)⁺.

Treatment of the product with excess 1M HCl in Et₂O followed by evaporation of the Et₂O under reduced pressure gave the HCl salt. Anal. calcd for C₂₃H₃₈N₃OSCl·0.5H₂O: C, 61.38; H, 8.96; N, 9.34; S, 7.12. Found: C, 61.72; H, 8.65; N, 9.30; S, 6.81%.

Using the appropriate ketone or aldehyde starting material and the procedure of step 4, the following compounds were prepared:

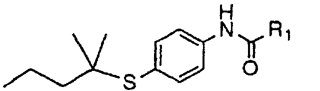

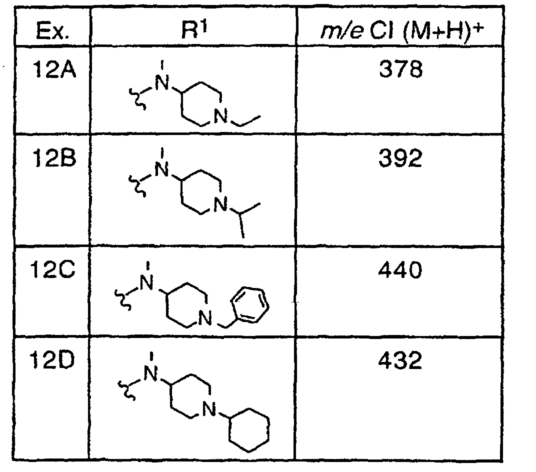

### Example 13

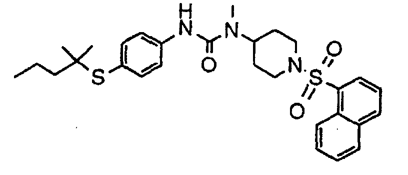

Using the procedure of Example 11, Step 3, reaction of the product of Example 12, Step 3 with 1-naphthalenesulfonyl chloride afforded the product. Anal. calcd for C₂₃H₃₇N₃OS·0.25H₂O: C, 64.03; H, 6.90; N, 7.70; S, 11.88. Found: C, 63.75; H, 6.77; N, 7.70; S, 12.05%. MS (Cl) *m*/*e* 540 (M+H)⁺.

Using the appropriate sulfonyl chloride starting material, the following compounds were prepared:
13A) 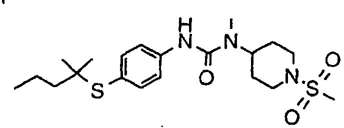 MS (Cl) *m*/*e* 428 (M+H)⁺.
13B) 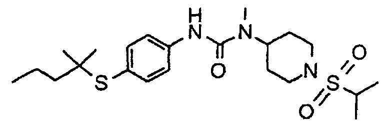 MS (Cl) *m*/*e* 456 (M+H)⁺.
13C) 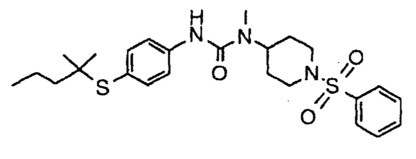 MS (Cl) *m*/*e* 490 (M+H)⁺.

### Example 14

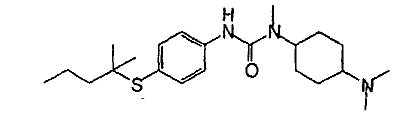

### Step 1:

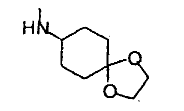

To a stirred mixture of 1,4-cyclohexanedione monoethylene ketal (4.68 g, 30 mmol) and 40% w/w aq. CH₃NH₂ (6.0 ml) in 1,2-dichloroethane (75 ml), was added Na(OAc)₃BH (9.6 g, 45 mmol) in portions. The reaction mixture was vigorously stirred for 16 h, then 1 N NaOH (75 ml) was added. The organic layer was washed with sat'd NaCl, dried (MgSO₄), filtered and evaporated to give an oil (4.60 g, 90%) that was used without further purification. ¹H NMR (CDCl₃, 400 MHz) δ 3.97 (4H, s), 2.47 (1H, m), 2.46 (3H, s), 1.91 (2H, m), 1.80 (2H, m), 1.59 (2H, m), 1.45 (2H, m).

### Step 2:

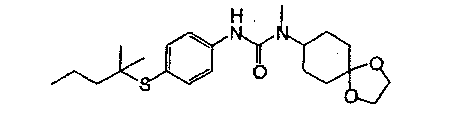

Using the procedure of Example 2, reaction of the product of Step 1 with the isocyanate derived from Preparation 1 afforded the product. MS *m*/*e* 407 (M+H)⁺.

### Step 3:

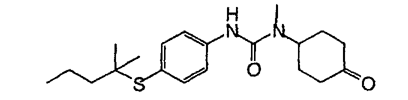

A mixture of the product of Step 2 (1.73 g, 2.8 mmol) and 3M HCl (10 ml) in THF (20 ml) was stirred at RT for 1 h. The reaction mixture was neutralized with 1M NaOH and extracted with CH₂Cl₂ (2x50 ml). The combined organic layers were dried (MgSO₄), filtered and evaporated. Flash chromatography (1:99 CH₃OH/CH₂Cl₂) of the residue afforded the product (0.90 g, 88%). MS *m*/*e* 363 (M+H)⁺.
Step 4: To a stirred mixture of the product of Step 3 (100 mg, 0.28 mmol) and 40% w/w (CH₃)₂NH (0.09 ml, 0.9 mmol) in CH₂Cl₂ (1 ml) was added Na(OAc)₃BH (88 mg, 0.42 mmol). After 16 h, 1M NaOH (5 ml) was added and the whole was extracted with CH₂Cl₂ (2x10 ml). The combined organic layers were dried (MgSO₄), filtered, and concentrated. Purification of the residue by preparative tic (1:7:92 conc. NH₄OH/CH₃OH/CH₂Cl₂) afforded the less polar *cis* isomer, 14A (48 mg, 45%) and the more polar *trans* isomer, 14B (31 mg, 29%).
14A, *Cis* isomer 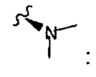 ¹H NMR (CDCl₃₊ CD₃OD, 400 MHz) δ 7.22 (4H, m), 4.08 (1H, m), 2.79 (3H, s), 2.13 (6H, s), 2.08 (1H, m), 1.83 (2H, m), 1.60 (2H, m), 1.40 - 1.23 (8H, m), 1.03 (6H, s), 0.74 (3H, t, *J*= 7.3 Hz). MS *m*/*e* 392 (M+H)⁺.
14B, *Trans* isomer 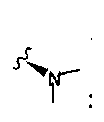 ¹H NMR (CDCl₃+ CD₃OD, 400 MHz) δ 7.22 (4H, m), 3.92 (1H, m), 2.72 (3H, s), 2.13 (6H, s), 1.96 (1H, m), 1.93 (2H, m), 1.63 (2H, m), 1.45 -1.22 (8H, m), 1.04 (6H, s), 0.74 (3H, t, *J*= 7.2 Hz). MS *m*/*e* 392 (M+H)⁺.

Using the appropriate amine and essentially the same procedure outlined in Example 14, Step 4, the following compounds were prepared.

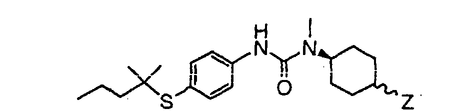

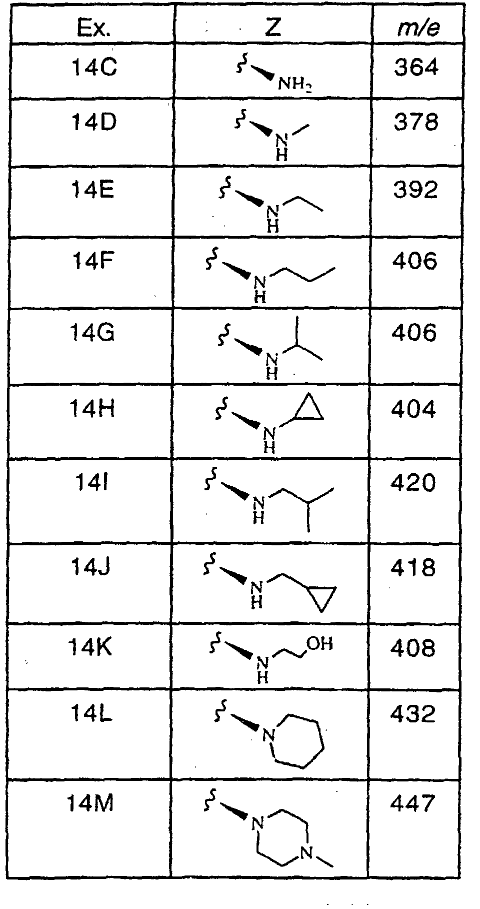

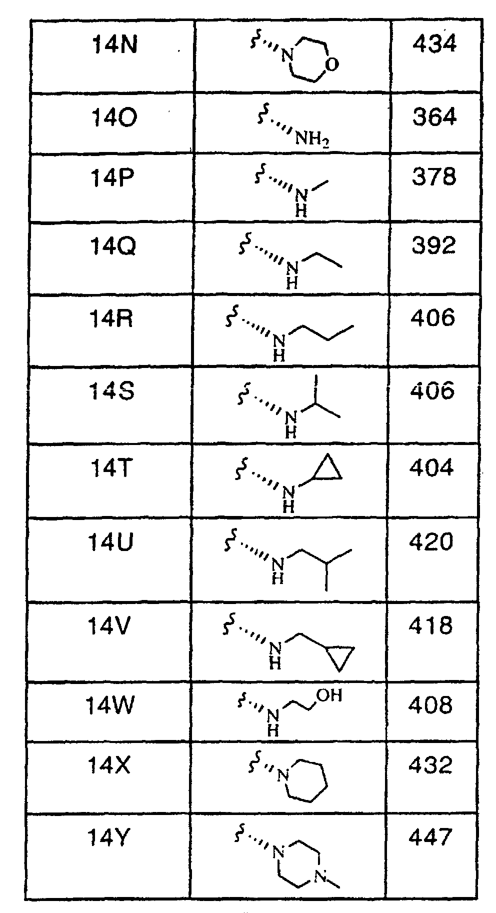

### Example 15

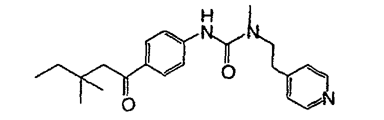

Using the procedure of Example 2, Preparation 7, *i*Pr₂NEt, triphosgene, and 4-(2-methy(amino)ethylpyridine were reacted to give the product. ¹H NMR (CDCl₃, 400 MHz) δ 8.50 (2H, s, ArH), 7.85 (2H, m, ArH), 7.41 (2H, m, ArH), 7.19 (2H, m, ArH), 6.60 (1H, s, NH), 3.60 (2H, t, *J* = 6.8 Hz, CH₂N), 2.97 (3H, s, CH₃N), 2.95 (2H, t, *J*= 7.2 Hz, NCH₂CH₂), 2.77 (2H, s, CH₂CO), 1.40 (2H, q, *J*= 7.6 Hz, CH₃CH₂), 1.03 (6H, s, (CH₃)₂C), 0.85 (3H, t, *J*= 7.6 Hz, CH₃CH₂). MS (ES) *m*/*e* 368 (M+H)⁺.

Reaction of Preparation 7,8, or 9, triphosgene, and the appropriate amine by essentially the same procedure afforded the following compounds: 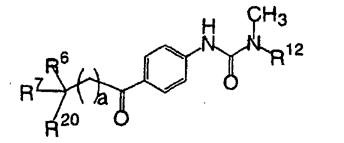 wherein a, R⁶, R⁷, R²⁰ and R¹² are as defined in the table

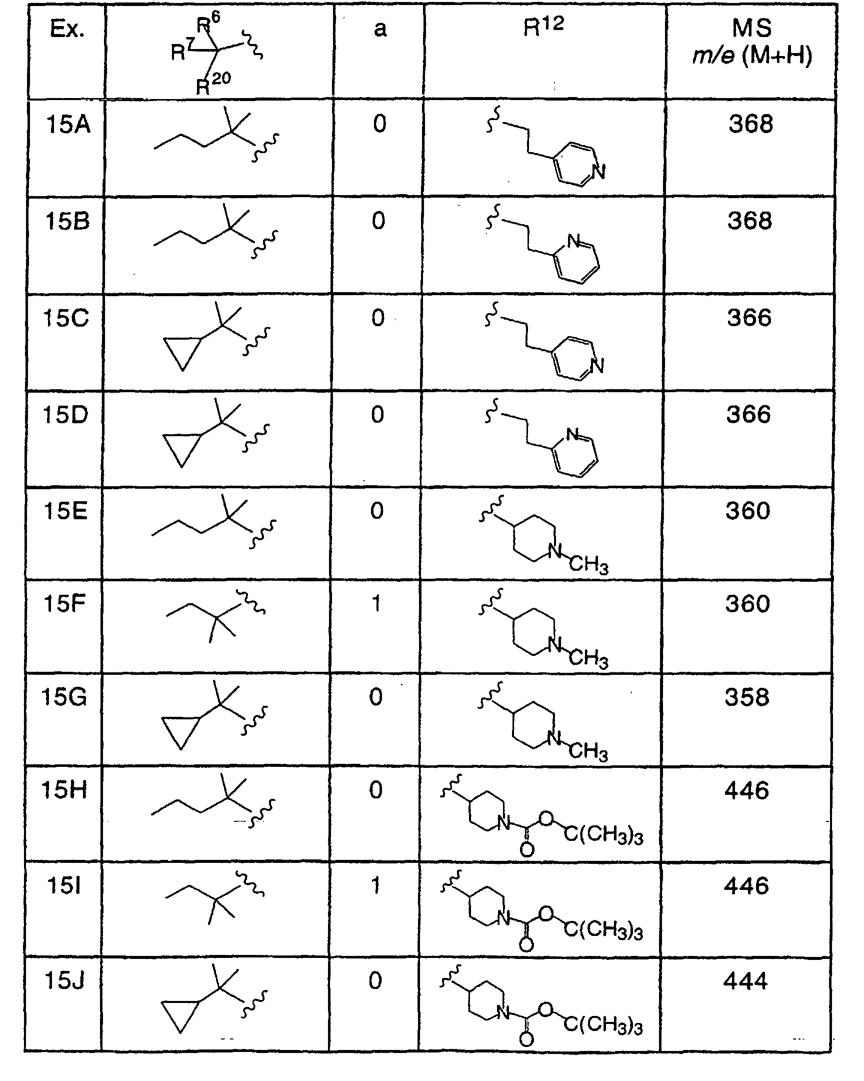

### Example 16

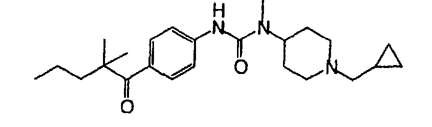

Step 1: Using the procedure described in Example 11, Step 2, the compound of Example 15H was treated with HCl to obtain the hydrochloride. MS *m*/*e* 346 (M-Cl)⁺.
Step 2: Using the procedure described in Example 12, Step 4, cyclopropane carboxaldehyde was reacted with the product of Step 1 to obtain the title compound. MS *m*/*e* 400 (M+H)⁺.

Using the appropriate starting materials and essentially the same procedure, the following compounds were prepared:
**16A** 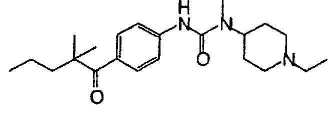 MS *m*/*e* 374 (M+H)⁺
**16B** 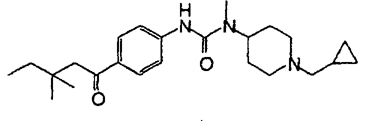 MS *m*/*e* 400 (M+H)⁺
**16C** 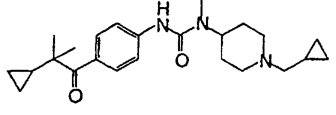 MS *m*/*e* 398 (M+H)⁺

### Example 17

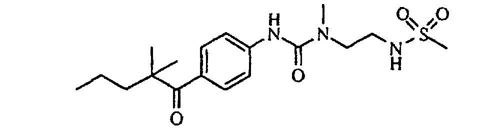

Step 1: To a mixture of t-butyldiphenylchlorosilane (9.3 g, 34 mmol) and Et₃N (5.12 g, 51 mmol) in CH₃CN (50 ml) was slowly added N-methylethylenediamine (5.0 g, 67 mmol). The reaction mixture was stirred for 2 h. After removal of CH₃CN, the residue was dissolved in CH₂Cl₂ and washed with sat'd NaHCO₃ and H₂O. The organic layer was dried (MgSO₄), filtered and evaporated to give a colorless oil (10.2 g) which was used directly in Step 2.

### Step 2:

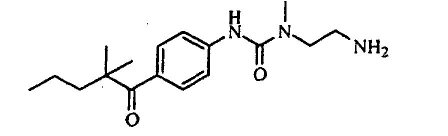

Using the procedure of Example 2, reaction of Preparation 8, triphosgene, and the product of Step 1 afforded the product. MS *m*/*e* 306.1 (M+H)⁺.
Step 3: To a solution of the product of Step 2 (95 mg, 0.31 mmol) and Et₃N (63 mg, 0.62 mmol) in CH₂Cl₂ (2 ml) was added CH₃SO₂Cl (72 mg, 0.63 mmol) dropwise. After 5 min, the reaction mixture was subjected to preparative TLC (CH₂Cl₂/CH₃OH/conc. NH₄OH 10:1:0.1) to afford the product (70 mg, 59%). ¹HNMR (CDCl₃, 400 MHz) δ 7.76 (2H, m, ArH), 7.47 (2H, m, ArH), 7.20 (1H, s, NH), 5.90 (1H, bs, NH), 3.50 (2H, m, CH₂CH₂), 3.30 (2H, m, CH₂CH₂), 2.98 (3H, s, CH₃), 2.97 (s, 3H, CH₃), 1.70 (m, 2H, CH₂), 1.05-1.4 (8H, m, (CH₃)₂ & CH₂), 0.9 (t, 3H, *J*= 7.3 Hz, CH₃). MS *m*/*e* 384.1 (M+H)⁺.

### Example 18

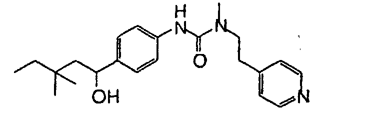

To a solution of Example 15 (330 mg, 0.90 mmol) in CH₃OH (10 ml) at RT was added NaBH₄ (68 mg, 18 mmol) in portions. The reaction was stirred at room temperature for 2 h, then poured into sat.'d NaHCO₃. The whole was extracted with CH₂Cl₂ (3x50 ml), the combined organic layers were washed with H₂O and brine, dried (Na₂SO₄), filtered and concentrated. The crude product (230 mg, 69%) was used in the next step without further purification. ¹H NMR (CDCl₃, 400 MHz) δ 8.50 (2H, br, ArH), 7.10 - 7.30 (6H, m, ArH), 6.30 (1H, s, NH), 4.75 (1H, m, HOCH), 3.60 (2H, t, *J* = 7.2 Hz, CH₂N), 2.93 (3H, s, CH₃N), 2.88 (2H, t, *J* = 7.6 Hz, NCH₂CH₂), 2.22 (1H, br, OH), 1.70 (1H, m, HOCHCHaHb), 1.50 (1H, m, HOCHCHaHb), 1.31 (m, 2H, CH₃CH₂), 0.89 (6H, s, (CH₃)₂C), 0.80 (3H, t, *J* = 7.2 Hz, CH₃CH₂).

Use of the appropriate starting material and essentially the same procedure afforded the following compounds: 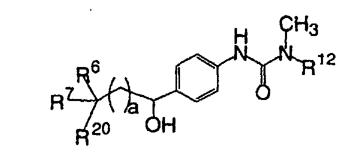 wherein a, R⁶, R⁷, R²⁰ and R¹² are as defined in the table

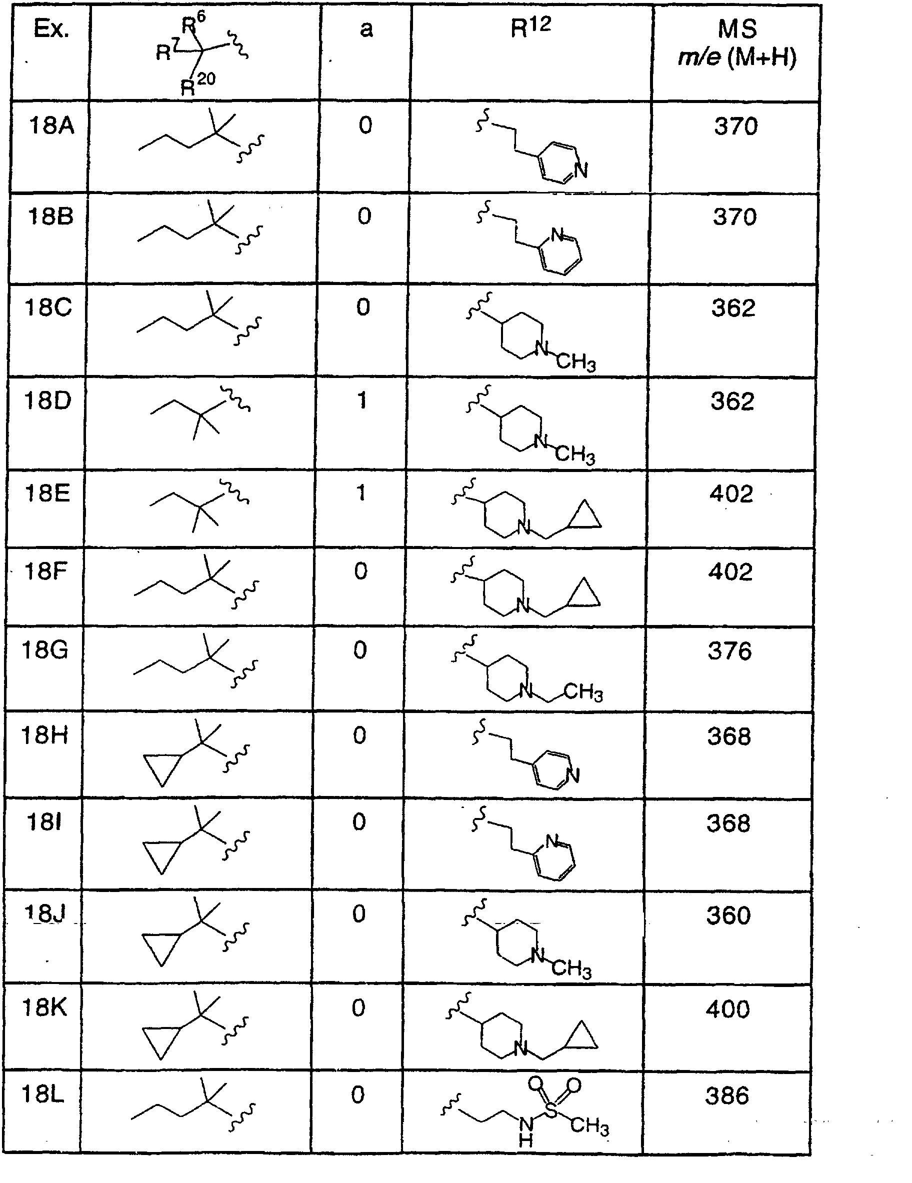

### Example 19

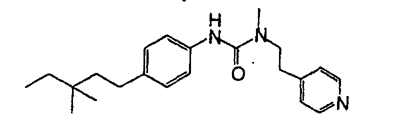

To a solution of Example 18 (230 mg, 0.62 mmol) in dry CH₂Cl₂ (20 ml) was added Et₃SiH (723 mg, 6.2 mmol) and CF₃CO₂H (142 mg, 1.2 mmol). The reaction mixture was stirred at RT for 16 h, then concentrated. The residue was subjected to preparative TLC (1:10 (2M NH₃ in CH₃OH)/CH₂Cl₂) to afford the product (180 mg, 82%) as colorless oil. ¹H NMR (CDCl₃, 400 MHz) δ 8.50 (2H, m, ArH), 7.0 - 7.25 (6H, m, ArH), 6.20 (1H, s, NH), 3.60 (2H, t, *J* = 7.2 Hz, CH₂N), 2.94 (3H, s, CH₃N), 2.85 (2H, t, *J* = 7.2 Hz, CH₂CH₂N), 2.45 (2H, m, CH₂CH₂CMe₂), 1.40 (2H, m, CH₂CH₂CMe₂), 1.30 (2H, q, *J* = 7.2 Hz, CH₃CH₂), 0.88 (6H, s, C(CH₃)₂), 0.80 (3H, t, *J*=7.6 Hz, CH₃CH₂). MS (ES) *m*/*e* 354 (M+H)⁺.

Using appropriate starting materials and essentially the same procedure the following compounds were prepared: 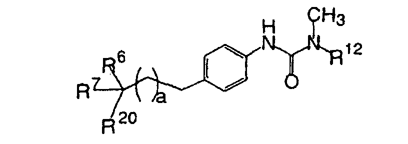 wherein a, R⁶, R⁷, R²⁰ and R¹² are as defined in the table

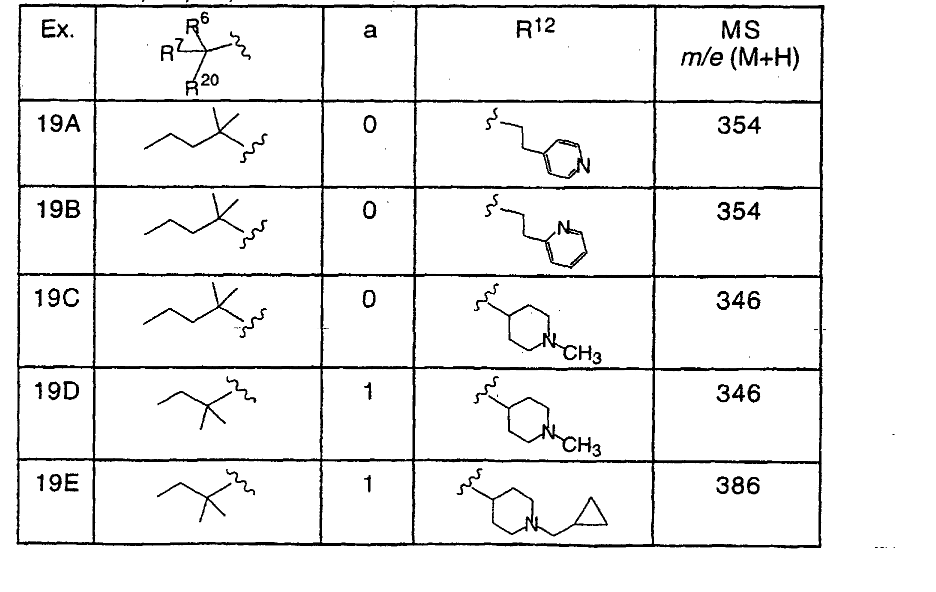

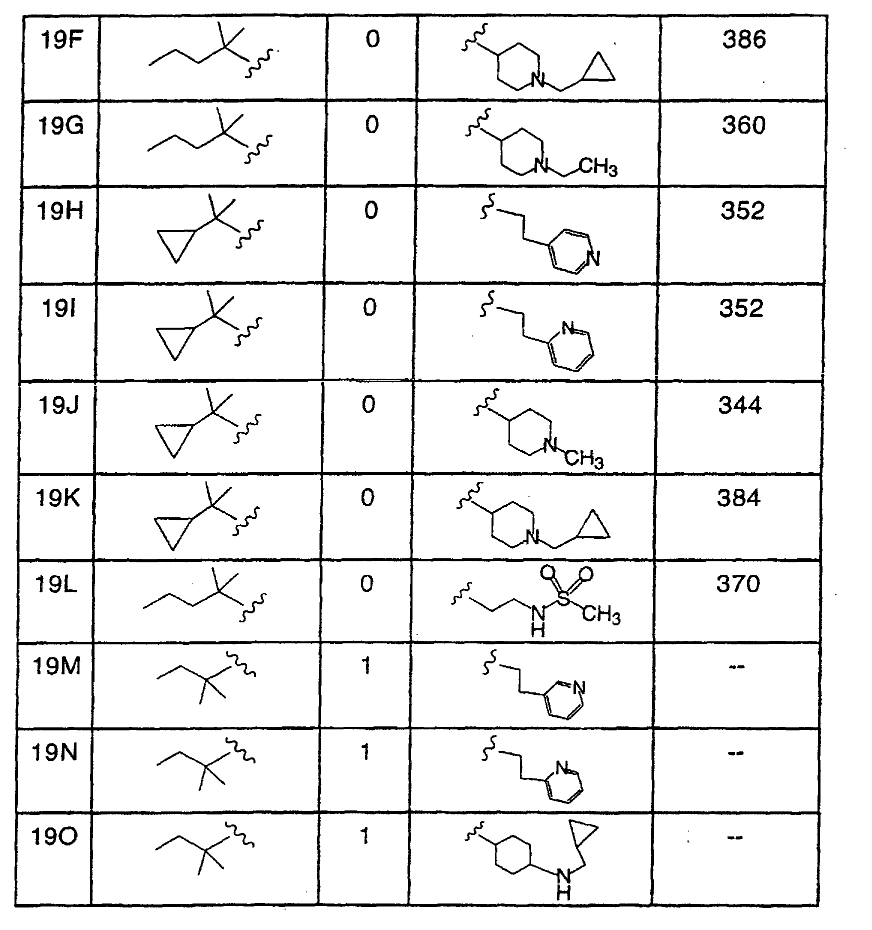

### Example 20

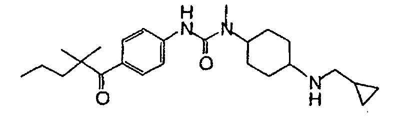

### Step 1:

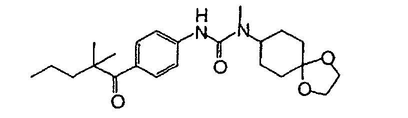

Reaction of Preparation 8 with the product of Example 14, Step 1 according to the procedure of Example 2 afforded the product. ¹H NMR (CDCl₃, 400 MHz) δ 7.76 (2H, m), 7.42 (2H, m), 6.48 (1H, s), 4.28 (1H, m), 3.95 (4H, s), 2.91 (3H, s), 1.75 (10H, m), 1.30 (6H, s), 1.21 (2H, m), 0.83 (3H, t). MS *m*/*e* 403 (M+H)⁺.

### Step 2:

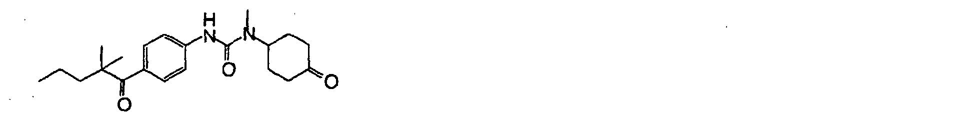

Reaction of the product of Step 1 with HCl by the procedure of Example 14, Step 3, afforded the product. ¹H NMR (CDCl₃, 400 MHz) δ 7.77 (2H, m), 7.44 (2H, m), 6.58 (1H, s), 4.81 (1H, m), 2.91 (3H, s), 2.57 (2H, m), 2.46 (2H, m), 2.03 (2H, m), 1.90 (2H, m), 1.75 (2H, m), 1.30 (6H, s), 1.21 (2H, m), 0.83 (3H, t). MS *m*/*e* 359 (M+H)⁺.
Step 3: Reaction of the product of Step 2 with cyclopropanemethylamine by the procedure of Example 14, Step 4, afforded the product as a mixture of *cis* and *trans* isomers that was separated by preparative tlc (1:9 (2M NH₃ in CH₃OH)/CH₂Cl₂).
**20A,** less polar*Cis* isomer 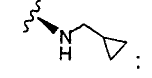 ¹H NMR (CDCl₃, 400 MHz) δ 7.75 (2H, m), 7.44 (2H, m), 6.58 (1H, s), 4.14 (1H, m), 2.94 (3H, s), 2.93 (1H, m), 2.46 (2H, m), 1.85 (4H, m), 1.74 (2H, m), 1.59 (2H, m), 1.46 (2H, m), 1.29 (6H, s), 1.20 (2H, m), 0.98 (1H, m), 0.82 (3H, t, *J*=7.2 Hz), 0.51 (2H, m), 0.14 (2H, m). MS *m*/*e* 414 (M+H)⁺.
**20B**, more polar *trans* isomer 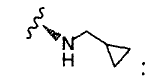 ¹H NMR (CDCl₃, 400 MHz) δ 7.75 (2H, m), 7.45 (2H, m), 6.64 (1H, s), 4.16 (1H, m), 2.89 (3H, s), 2.50 (3H, m), 2.05 (2H, m), 1.74 (4H, m,), 1.51 (2H, m), 1.38 (2H, m), 1.29 (6H, s), 1.23 (2H, m), 0.98 (1H, m), 0.82 (3H, t, *J*= 7.3 Hz), 0.49 (2H, m), 0.15 (2H, m). MS *m*/*e* 414 (M+H)⁺.

Similarly prepared from the product of Step 2 were:
**20C** 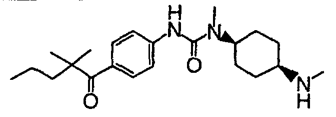 ¹H NMR (CDCl₃, 400 MHz) δ 7.77 (2H, m), 7.42 (2H, m), 6.50 (1H, s), 4.14 (1H, m), 2.93 (3H, s), 2.72 (1H, m), 2.39 (3H, s), 1.84 (4H, m), 1.76 (2H, m), 1.59 (2H, m), 1.46 (2H, m), 1.30 (6H, s), 1.21 (2H, m), 0.82 (3H, t, *J* = 7.2 Hz). MS *m*/*e* 374 (M+H)⁺.
**20D** 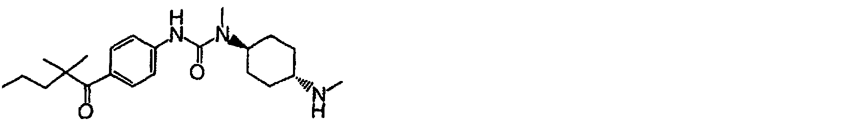 ¹H NMR (CDCl₃, 400 MHz) δ 7.76 (2H, m), 7.42 (2H, m), 6.48 (1H, s), 4.17 (1H, m), 2.89 (3H, s), 2.44 (3H, s), 2.35 (1H, m), 2.05 (2H, m), 1.75 (4H, m), 1.50 (2H, m), 1.46 (2H, m), 1.30 (6H, s), 1.21 (2H, m), 0.83 (3H, t, *J* = 7.3 Hz). MS *m*/*e* 374 (M+H)⁺.
**20E** 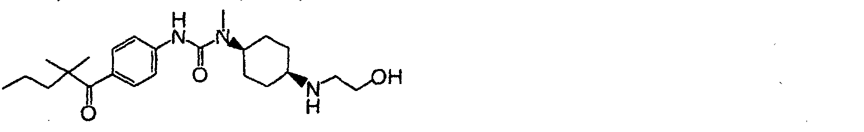 ¹H NMR (CDCl₃, 400 MHz) δ 7.75 (2H, m), 7.43 (2H, m), 6.51 (1H, s), 4.14 (1H, m), 3.70 (2H, m), 2.93 (3H, s), 2.80 (2H, m), 2.52 (1H, m), 1.88 (4H, m), 1.75 (2H, m), 1.71 (2H, m), 1.51 (2H, m), 1.30 (6H, s), 1.21 (2H, m), 0.83 (3H, t, *J*=7.3 Hz). MS *m*/*e* 404 (M+H)⁺.
**20F** 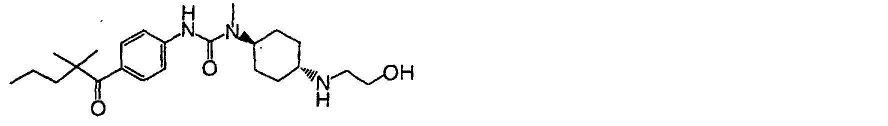 ¹H NMR (CDCl₃, 400 MHz) δ 7.75 (2H, m), 7.43 (2H, m), 6.54 (1H, s), 4.14 (1H, m), 3.70 (2H, m), 2.88 (3H, s), 2.86 (2H, m), 2.51 (3H, m), 2.08 (2H, m), 1.75 (4H, m), 1.51 (2H, m), 1.30 (6H, s), 1.21 (2H, m), 0.83 (3H, t, *J*=7.3 Hz). MS *m*/*e* 404 (M+H)⁺.

### Example 21

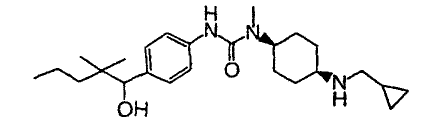

Reduction of Example 20A with NaBH₄ by the procedure of Example 18 afforded the title compound. ¹H NMR (CDCl₃, 400 MHz) δ 7.34 (2H, m), 7.20 (2H, m), 6.32 (1H, s), 4.40 (1H, s), 4.16 (1H, m), 2.93 (4H, s), 2.46 (2H, m), 1.88 (4H, m), 1.60 (2H, m), 1.49 (2H, m), 1.31 (4H, m), 0.99 (1H, m), 0.87 (6H, s), 0.79 (3H, m), 0.51 (2H, m), 0.15 (2H, m). MS *m*/*e* 416 (M+H)⁺.

Similarly prepared was:
**21A** 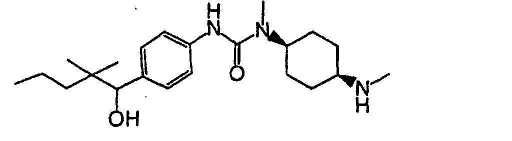 ¹H NMR (CDCl₃, 400 MHz) δ 7.34 (2H, m), 7.21 (2H, m), 6.31 (1H, s), 4.41 (1H, s), 4.18 (1H, m), 2.93 (3H, s), 2.78 (1H, m), 2.43 (3H, s), 1.87 (4H, m), 1.62 (2H, m), 1.49 (2H, m), 1.31 (4H, m), 0.87 (6H, s), 0.79 (3H, m). MS *m*/*e* 376 (M+H)⁺.

### Example 22

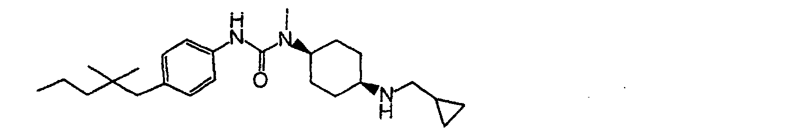

Reduction of Example 20 with Et₃SiH/TFA by the procedure of Example 19 afforded the title compound. ¹H NMR (CDCl₃, 400 MHz) δ 7.28 (2H, m), 7.10 (2H, m), 6.26 (1H, s), 4.16 (1H, m), 2.93 (3H, s), 2.89 (1H, m), 2.47 (2H, m), 2.43 (2H, s), 1.87 (4H, m), 1.60 (2H, m), 1.48 (2H, m), 1.31 (2H, m), 1.16 (2H, m), 0.99 (1H, m), 0.88 (3H, t, *J* = 7.3 Hz), 0.81 (6H, s). 0.50 (2H, m), 0.15 (2H, m). MS *m*/*e* 400 (M+H)⁺.

Similarly, the following compound was prepared:
**22A** 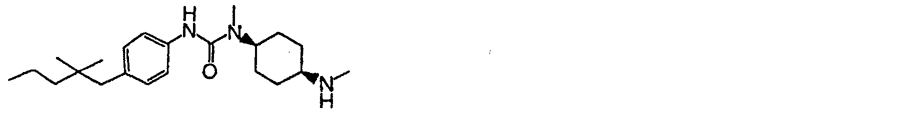 ¹H NMR (CDC₃, 400 MHz) δ 7.27 (2H, m), 7.01 (2H, m), 6.26 (1H, s), 4.18 (1H, m), 2.92 (3H, s), 2.77 (1H, m), 2.43 (5H, s), 1.88 (4H, m), 1.66 (2H, m), 1.50 (2H, t), 1.30 (2H, m), 1.11 (2H, m), 0.88 (3H, s ), 0.81 (6H, s). MS *m*/*e* 360 (M+H)⁺.

### Example 23

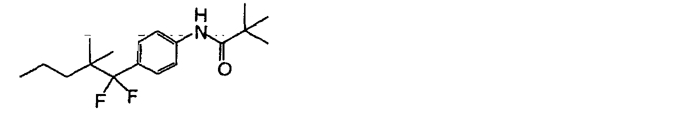

### Step 1:

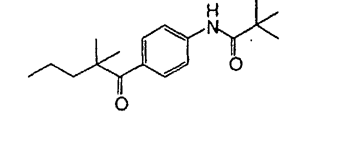

Using the procedure of Example 1, reaction of Preparation 8, trimethyl acetyl chloride and pyridine gave the product. ¹H NMR (CDCl₃, 400 MHz) δ 7.76 (d, 2H, J=8.8 Hz, ArH), 7.57 (d, 2H, J=8.4 Hz, ArH), 7.41 (s, 1H, NH), 1.75 (m, 2H, CH₃CH₂CH₂), 1.32 (s, 9H, (CH₃)₃C), 1.30 (s, 6H, (CH₃)₂C), 1.26 (m, 2H, CH₂CH₃), 0.84 (t, 3H, *J*=7.2 Hz, CH₃CH₂). MS *m*/*e* 290 (M+H)⁺.
Step 2: To a solution of the product of Step 1 (100 mg, 0.346 mmol) in dry CH₂Cl₂ (1.0 ml) was added (diethylamino)sulfur trifluoride (557 mg, 3.46 mmol). The reaction mixture was heated at 80 °C overnight, then allowed to cool to RT. The crude mixture was subjected to plc (1:6 EtOAc/hexanes) to give the product (25.0 mg, 23%) as a colorless oil. ¹H NMR (CDCl₃, 400 MHz) δ 7.37 (m, 2H, ArH), 7.26 (m, 2H, ArH), 1.32 (m, 13H, (CH₃)₃C & CH₃CH₂CH₂), 0.98 (s, 6H, (CH₃)₂C), 0.87 (m, 3H, CH₃CH₂). MS *m*/*e* 312 (M+H)⁺.

## Claims

1. A compound having the structural formula 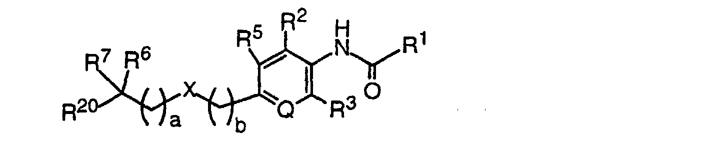 or a pharmaceutically acceptable salt thereof, wherein
a and b are independently 0, 1 or 2, provided that the sum of a and b is 0 to 3;
Q is 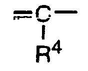 or -N= ;
X is -O-, -S-, -SO-, -SO₂-, -CH(OR⁸)-, -C(O)-, -C(R²³)₂-, -C(R²⁵)=C(R²⁵)-, -C≡C- or 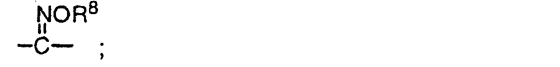 R¹ is R¹⁵-aryl, R²⁴-heteroayyl, -NHR¹², -N(R¹²)₂, -(C₁-C₉)alkyl-OC(O)R⁸, aryloxy(C₁-C₉)alkyl, 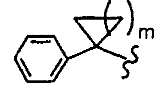 wherein m is 1-4, or 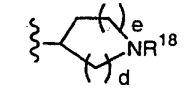 wherein d and e are independently 0, 1 or 2;
R², R³, R⁴ and R⁵ are independently selected from the group consisting of H, C₁-C₅ straight or branched alkyl, (C₃-C₁₂)cycloalkyl, R¹⁴-(C₃-C₁₂)cycloalkyl, halogen, -OR⁸, -N(R⁸)₂, -CO₂R⁸ and CF₃;
R⁶ and R⁷ are independently selected from the group consisting of H, (C₁-C₉)alkyl, (C₁-C₉)alkenyl, hydroxy-(C₁-C₉)alkyl, amino-(C₁-C₉)-alkyl, (C₁-C₉)alkoxy-(C₁-C₉)alkyl, (C₃-C₁₂)cycloalkyl and (C₃-C₁₂)-cycloalkyl-(C₁-C₆)alkyl, or R⁶ and R⁷, together with the carbon to which they are attached, form a 3, 4, 5, 6 or 7-membered carbocyclic ring, or a 4, 5, 6 or 7-membered heterocyclic ring, wherein 1, 2 or 3 ring members are independently selected from the group consisting of O, S and N;
R⁸ is independently selected from the group consisting of H, (C₁-C₆)alkyl, (C₃-C₁₂)cycloalkyl, R¹⁵-aryl and R²⁴-heteroaryl;
R⁹ is (C₁-C₆)alkyl, (C₃-C₁₂)cycloalkyl, R¹⁵-aryl or R²⁴-heteroaryl;
R¹¹ is independently selected from the group consisting of H, (C₁-C₆)alkyl and (C₃-C₁₂)cycloalkyl;
R¹² is independently selected from the group consisting of straight or branched (C₁-C₉)alkyl, hydroxy(C₂-C₉)alkyl, (C₁-C₉)alkoxy(C₂-C₉)-alkyl, N(R¹¹)(R¹⁹)-(C₂-C₉)-alkyl, HS(C₂-C₉)-alkyl, (C₁-C₉)-alkylthio-(C₂-C₉)-alkyl, (C₃-C₁₂)-cycloalkyl, R¹⁴-(C₃-C₁₂) cycloalkyl, R¹⁵-aryl,
R²⁴-heteroaryl, R¹⁵-aryl(C₁-C₆)-alkyl, R²⁴-heteroaryl(C₁-C₆)-alkyl, 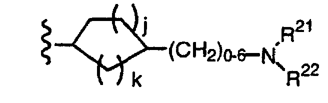 wherein j and k are independently 0, 1 or 2, and 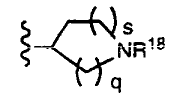 wherein q is 1 or 2, and s is 0, 1 or 2; or two R¹² groups, together with the nitrogen to which they are attached, form a ring of the formula 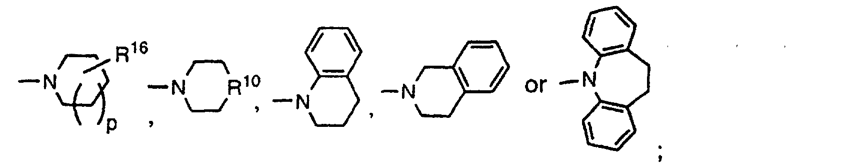 wherein p is 0, 1 or 2;
R¹⁰ is -NR¹⁸-, -O- or -S-;
R¹³ is 1 to 3 substituents independently selected from the group consisting of hydrogen, (C₁-C₆)alkyl, halogen, (C₁-C₆)alkoxy and CF₃;
R¹⁴ is 1 to 3 substituents independently selected from the group consisting of (C₁-C₆)alkyl, benzyl, R¹³-aryl and R¹³-heteroaryl;
R¹⁵ is 1 to 3 substituents independently selected from the group consisting of hydrogen, (C₁-C₆)alkyl, halo, polyhalo(C₁-C₆)alkyl, R¹⁷O-, -N(R¹⁷)₂, -S(R¹⁷), R¹⁷O-(C₁-C₆)alkyl, (R¹⁷)₂N-(C₁-C₆)alkyl, formyl, -C(O)R¹⁷, -COOR¹⁷, -CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷- and tri(C₁-C₆)-alkylsilyl;
R¹⁶ is 1-3 substituents independently selected from the group consisting of H, (C₁-C₆)alkyl, (C₃-C₁₂)cycloalkyl, (C₃-C₁₂)spirocycloalkyl, (C₃-C₄)spiro-alkylenedioxy, R¹⁵-aryl, R²⁴-heteroaryl, benzyl, N-piperidinyl, -COR⁸, -C(O)NR⁸R⁹, -NR⁸R⁹ and -NR⁸C(O)R⁹, or when two R¹⁶ groups are attached to adjacent ring carbon atoms, together with said carbon atoms, they can form a (C₅-C₇)cycloalkyl ring;
R¹⁷ is independently selected from the group consisting of H, (C₁-C₆)alkyl, (C₃-C₁₂)cycloalkyl, (C₃-C₁₂)cycloalkyl(C₁-C₆)alkyl, R¹³-aryl and R¹³-heteroaryl;
R¹⁸ is independently selected from the group consisting of H, (C₁-C₆)alkyl, (C₃-C₁₂)cycloalkyl, (C₃-C₁₂)cycloalkyl-(C₁-C₆)alkyl, R¹⁵-aryl, R²⁴-heteroaryl, -CO₂R⁹, -C(O)N(R⁸)₂, -COR⁸ and -SO₂R⁹;
R¹⁹ is H, (C₃-C₁₂)cycloalkyl-(C₁-C₆)alkyl, R¹⁵-aryl, R²⁴-heteroaryl, -CO₂R⁹, -C(O)N(R⁸)₂, -COR⁸ or -SO₂R⁹;
R²⁰ is (C₁-C₆)alkyl, (C₃-C₁₂)cycloalkyl, (C₃-C₁₂)cycloalkyl-(C₁-C₆)alkyl, hydroxy(C₁-C₆)alkyl, oxo(C₁-C₆)alkyl or polyhalo(C₁-C₆)alkyl;
R²¹ and R²² are independently selected from the group consisting of H, (C₁-C₆)alkyl, (C₃-C₁₂)cycloalkyl-(C₁-C₆)alkyl, hydroxy(C₁-C₆)alkyl, R¹⁵-aryl, R²⁴-heteroaryl, R¹⁵-aryl(C₁-C₆)alkyl or R²⁴-heteroaryl(C₁-C₆)-alkyl;
R²³ is independently selected from the group consisting of H, halogen, (C₁-C₆)alkyl, (C₃-C₁₂)cycloalkyl, R¹⁵-aryl, and R²⁴-heteroaryl;
R²⁴ is 1 to 2 substituents independently selected from the group consisting of hydrogen, (C₁-C₆)alkyl, halo, polyhalo(C₁-C₆)alkyl, R¹⁷O-, -N(R¹⁷)₂, -S(R¹⁷), R¹⁷O-(C₁-C₆)alkyl, (R¹⁷)₂N-(C₁-C₆)alkyl, formyl, -C(O)R¹⁷, -COOR¹⁷, -CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷- and tri(C₁-C₆)-alkylsilyl; and
R²⁵ is independently selected from the group consisting of hydrogen, halogen, (C₁-C₆)-alkyl and polyhalo(C₁-C₆)alkyl.

2. A compound of claim 1 wherein Q is

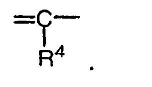

3. A compound of claim 1 wherein R³ and R⁴ are each H; and R² and R⁵ are independently selected from the group consisting of hydrogen and halogen.

4. A compound of claim 1 wherein X is -S-; -C(O)-; -CH(OR⁸)- or -C(R²³)₂-.

5. A compound of claim 4 wherein X is -C(R²³)₂- and R²³ is hydrogen.

6. A compound of claim 1 wherein a is 1 or 2 and b is 0.

7. A compound of claim 1 wherein R¹ is -NHR¹² or -N(R¹²)₂.

8. A compound of claim 7 wherein R¹ is 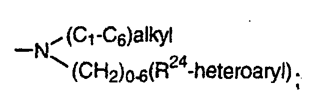 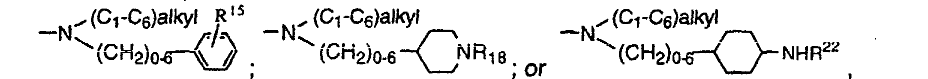 wherein R¹⁸ is (C₁-C₆)alkyl or -SO₂R⁹; R⁹ is (C₁-C₆)alkyl or aryl; and R²² is (C₁-C₆)alkyl or (C₃-C₁₂)cycloalkyl(C₁-C₆)alkyl.

9. A compound of claim 1 wherein R⁶ and R⁷ are each (C₁-C₆)alkyl, or R⁶ and R⁷, together with the carbon to which they are attached, form a C₃-C₆ carbocyclic ring.

10. A compound of claim 1 selected from the group consisting of those having the structural formula 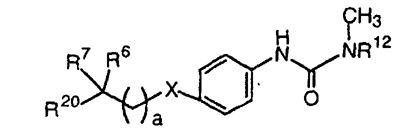 wherein R²⁰, R⁶, R⁷, a, X, and R¹² are as defined in the following table:

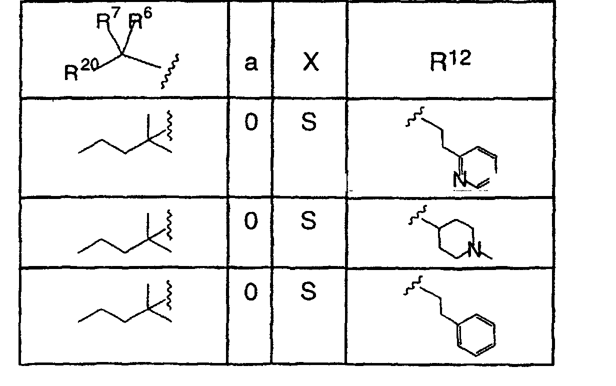

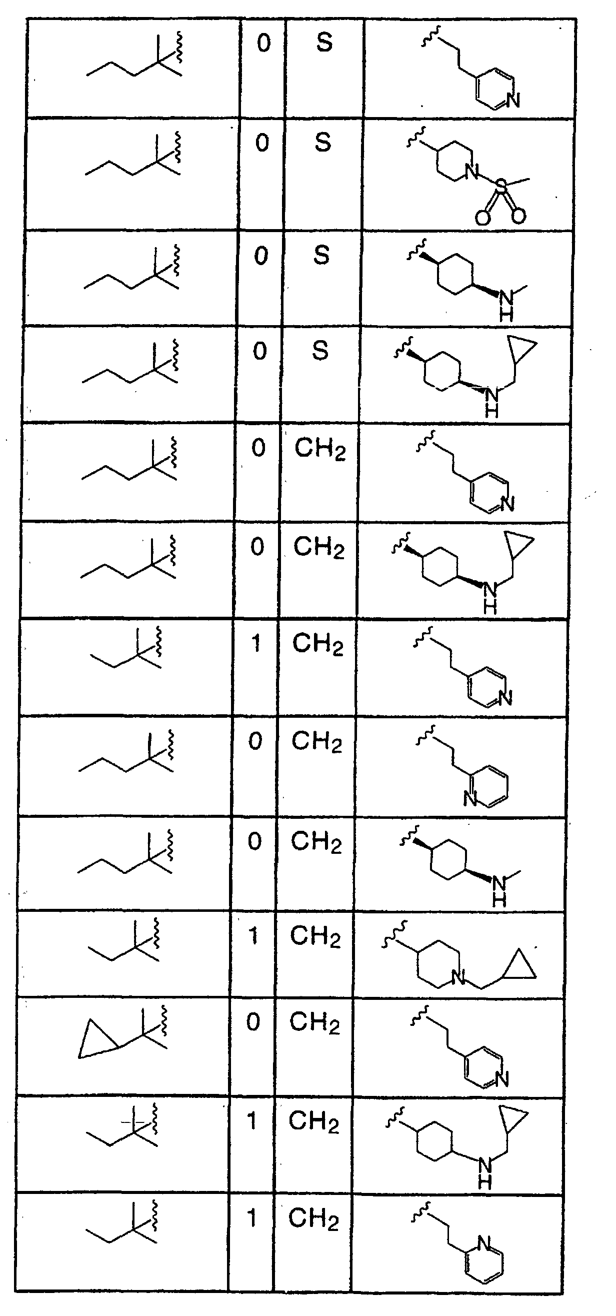

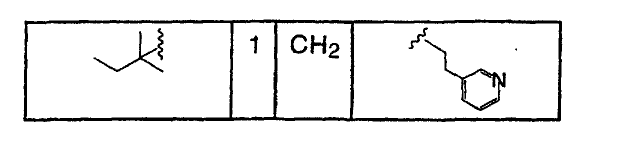

11. A compound selected from the group consisting of those having the structural formula 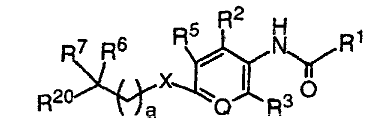 wherein R²⁰, R⁶, R⁷, a, X, R⁵, R² , R³, Q and R¹ are as defined in the following table:

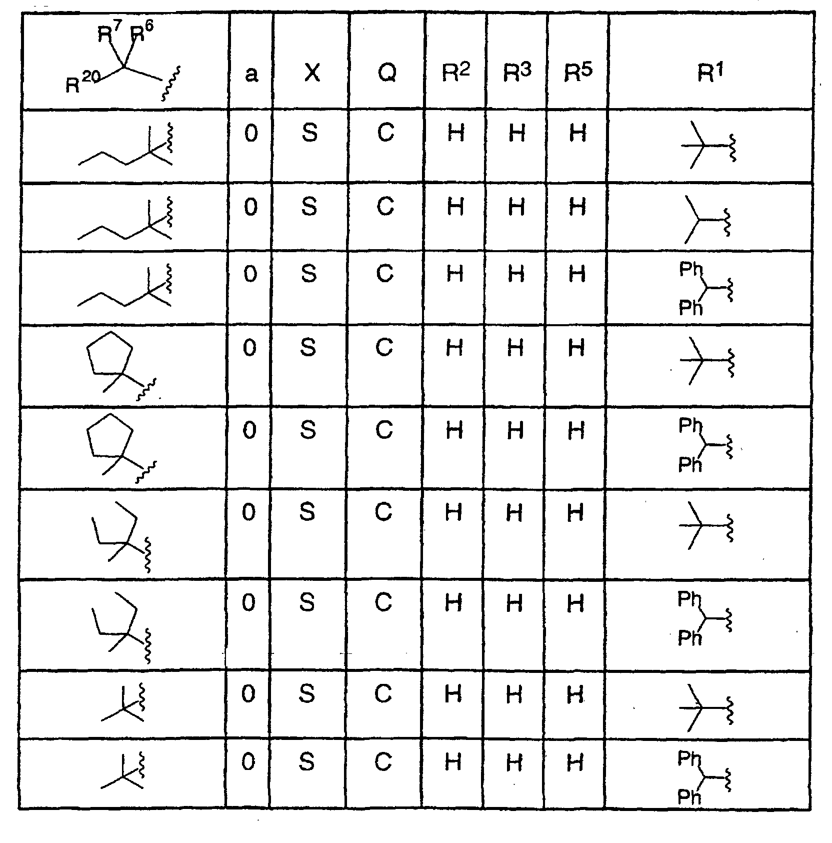

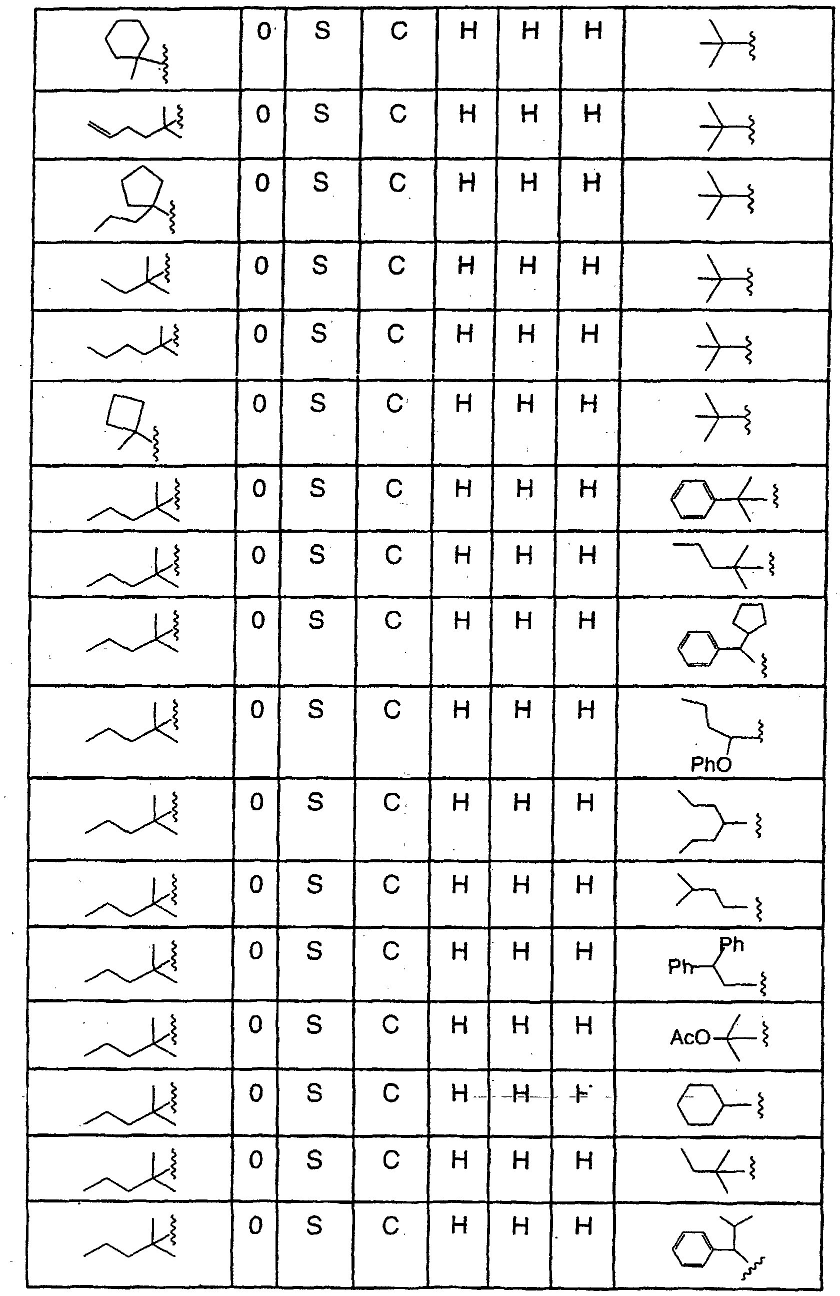

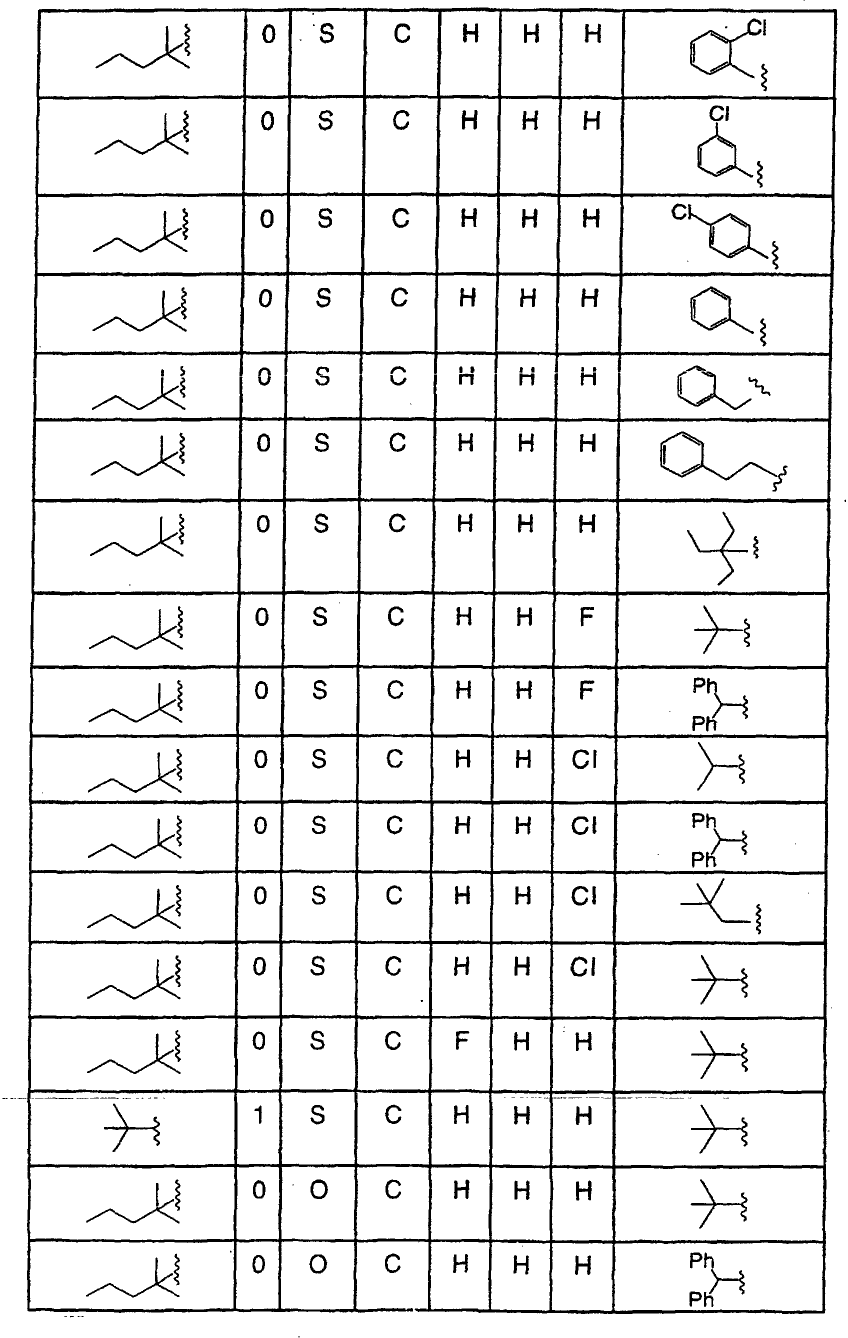

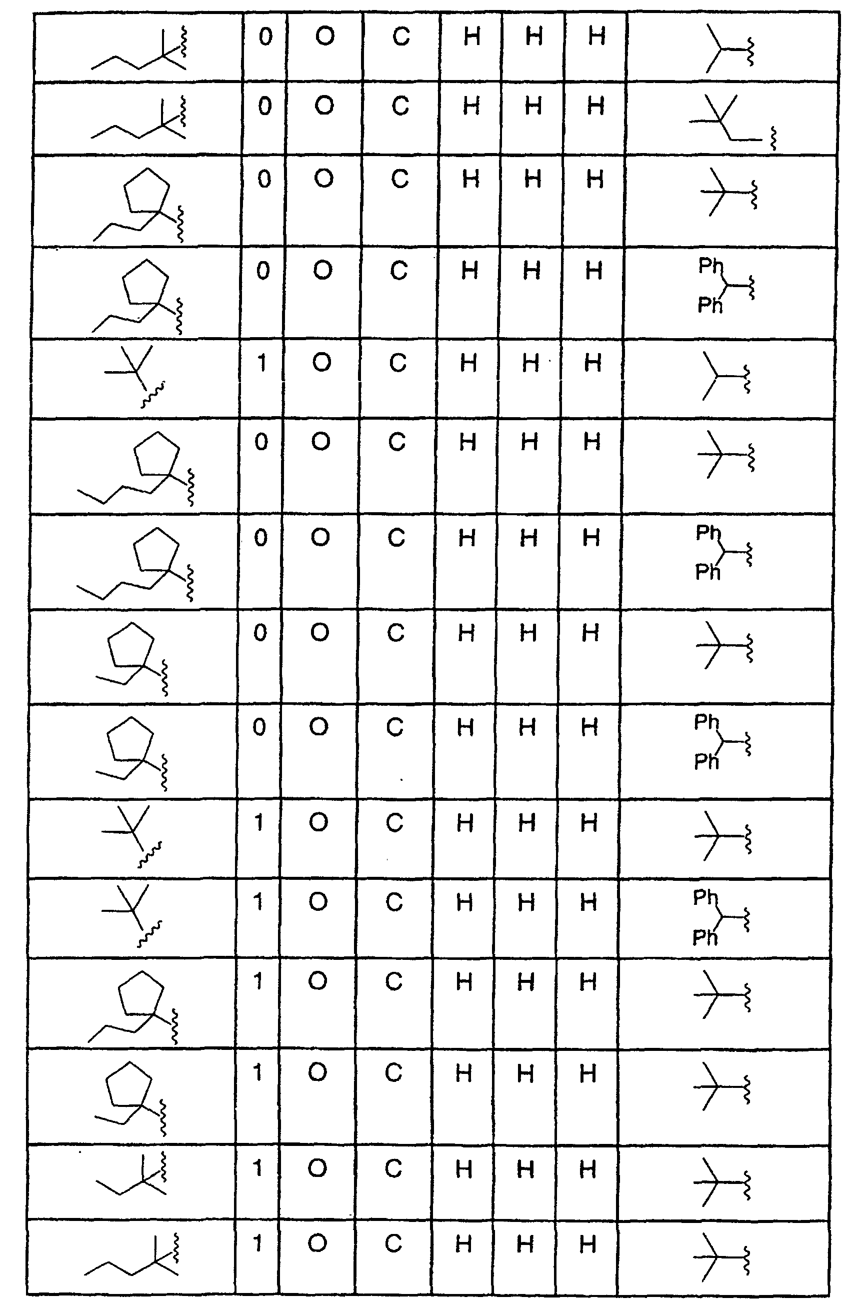

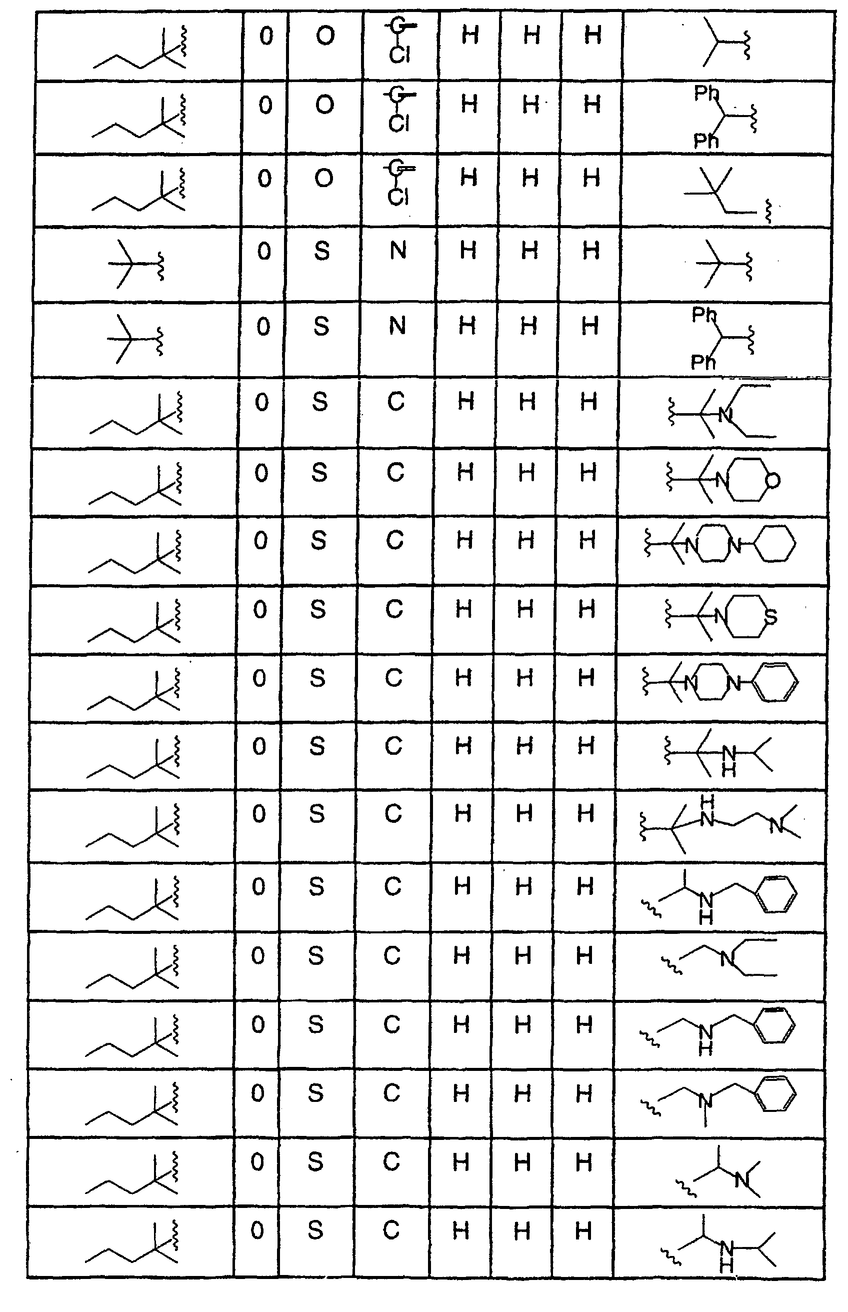

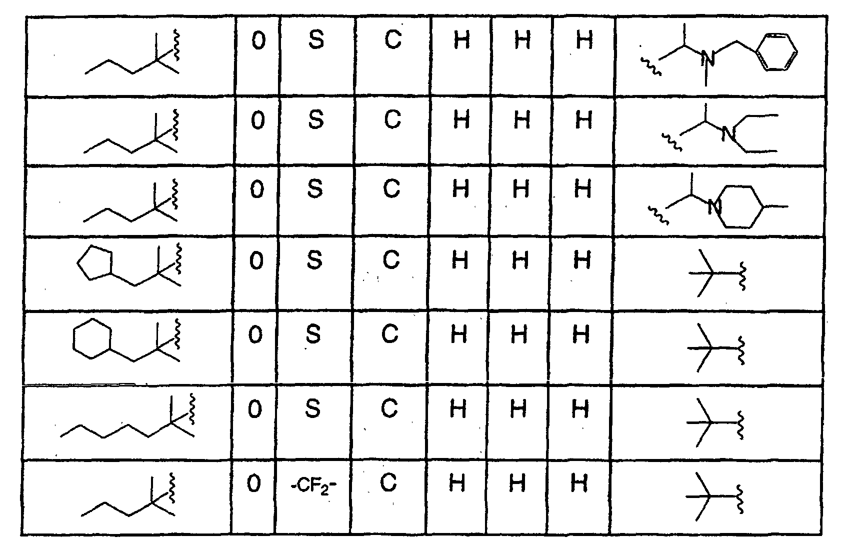

12. A pharmaceutical composition comprising a compound as defined in claim 1 in combination with a pharmaceutically acceptable carrier.

13. The use of a compound of claim 1 for the preparation of a medicament for treating an eating disorder or diabetes.

## Patentansprüche

1. Verbindung der Strukturformel 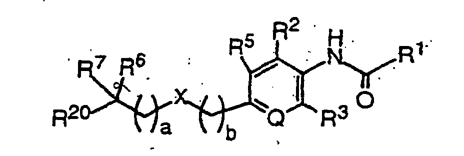 oder ein pharmazeutisches akzeptables Salz davon, wobei a und b unabhängig 0, 1 oder 2 sind, vorausgesetzt, dass die Summe von a und b 0 bis 3 ist;
Q 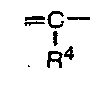 oder -N= ist;
X -O-, -S-, -SO-, -SO₂-, -CH(OR⁸)-, -C(O)-, -C(R²³)₂-, -C(R²⁵)=C(R²⁵)-, -C≡C- oder 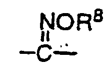 ist;
R¹ R¹⁵-Aryl, R²⁴-Heteroaryl, NHR¹², -N(R¹²)₂, -(C₁ bis C₉)Alkyl-OC(O)R⁸, Aryloxy(C₁ bis C₉)Alkyl, 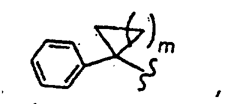 wobei m 1 bis 4 ist, oder 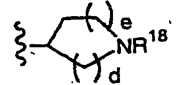 ist, wobei d und e unabhängig 0, 1 oder 2 sind;
R², R³, R⁴ und R⁵ unabhängig ausgewählt sind aus der Gruppe bestehend aus H, geradkettigem oder verzweigtem C₁ bis C₅ Alkyl, (C₃ bis C₁₂)Cycloalkyl, R¹⁴- (C₃ bis C₁₂)Cycloalkyl, Halogen, OR⁸, -N(R⁸)₂, -CO₂R⁸ und CF₃;
R⁶ und R⁷ unabhängig voneinander ausgewählt sind aus der Gruppe bestehend aus H, (C₁ bis C₉)Alkyl, (C₁ bis C₉)Alkenyl, Hydroxy-(C₁ bis C₉)alkyl, Amino-(C₁ bis C₉)alkyl, (C₁ bis C₉)Alkoxy-(C₁ bis C₉)alkyl, (C₃ bis C₁₂)Cycloalkyl und (C₃ bis C₁₂)Cycloalkyl-(C₁ bis C₆) oder R⁶ und R⁷ zusammen mit dem Kohlenstoff, an den sie gebunden sind, einen 3-, 4-, 5-, 6- oder 7-gliedrigen Kohlenstoff-Ring oder einen 4-, 5-, 6- oder 7-gliedrigen heterozyklischen Ring bilden, wobei 1, 2 oder 3 Ringglieder unabhängig voneinander ausgewählt sind aus der Gruppe bestehend aus O, S und N;
R⁸ unabhängig ausgewählt ist aus der Gruppe bestehend aus H, (C₁ bis C₆)Alkyl, (C₃ bis C₁₂)Cycloalkyl, R¹⁵-Aryl und R²⁴-Heteroaryl;
R⁹ (C₁ bis C₆)Alkyl, (C₃ bis C₁₂)Cycloalkyl, R¹⁵-Aryl oder R²⁴-Heteroaryl ist;
R¹¹ unabhängig ausgewählt ist aus der Gruppe bestehend aus H, (C₁ bis C₆)Alkyl und (C₃ bis C₁₂)Cycloalkyl;
R¹² unabhängig ausgewählt ist aus der Gruppe bestehend aus geradkettigem oder verzweigtem (C₁ bis C₉)Alkyl, Hydroxy(C₂ bis C₉)alkyl, (C₁ bis C₉)Alkoxy-(C₂ bis C₉)-alkyl, N(R¹¹)(R¹⁹)-(C₂ bis C₉)-Alkyl, HS(C₂ bis C₉)-Alkyl, (C₁ bis C₉)-Alkylthio(C₂ bis C₉)-alkyl, (C₃ bis C₁₂)Cycloalkyl, R¹⁴-(C₃ bis C₁₂) Cycloalkyl, R¹⁵-Aryl,
R²⁴-Heteroaryl, R¹⁵-Aryl(C₁ bis C₆)-alkyl, R²⁴-Heteroaryl(C₁ bis C₆)-alkyl, 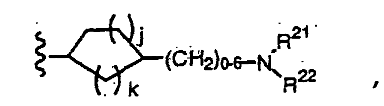 wobei j und k unabhängig 0, 1 oder 2 sind, und 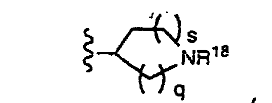 wobei q 1 oder 2, und s 0, 1 oder 2 ist; oder zwei R¹² Gruppen zusammen mit dem Stickstoff, an den sie gebunden sind, einen Ring der Formel 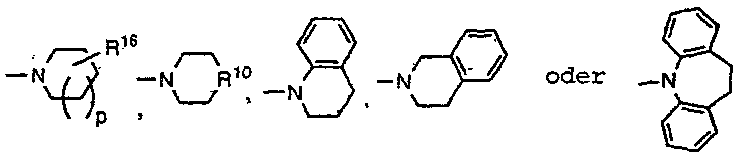 bilden;
wobei p 0, 1 oder 2 ist;
R¹⁰ -NR¹⁸-, -O- oder -S- ist;
R¹³ 1 bis 3 Substituenten unabhängig ausgewählt aus der Gruppe bestehend aus Wasserstoff, (C₁ bis C₆)Alkyl, Halogen, (C₁ bis C₆) Alkoxy und CF₃ ist;
R¹⁴ 1 bis 3 Substituenten unabhängig ausgewählt aus der Gruppe bestehend aus (C₁ bis C₆)Alkyl, Benzyl, R¹³-Aryl und R¹³-Heteroaryl ist;
R¹⁵ 1 bis 3 Substituenten unabhängig ausgewählt aus der Gruppe bestehend aus Wasserstoff, (C₁ bis C₆)Alkyl, Halogen, Polyhalogen (C₁ bis C₆)alkyl, R¹⁷O-, -N(R¹⁷)₂, -S(R¹⁷), R¹⁷O-(C₁ bis C₆)Alkyl, (R¹⁷)₂N-(C₁ bis C₆)Alkyl, Formyl, -C(O)R¹⁷, -COOR¹⁷, CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷- und tri(C₁ bis C₆)Alkylsilyl ist;
R¹⁶ 1 bis 3 Substituenten unabhängig ausgewählt aus der Gruppe bestehend aus H, (C₁ bis C₆)Alkyl, (C₃ bis C₁₂)Cycloalkyl, (C₃ bis C₁₂)Spirocycloalkyl, (C₃ bis C₄)Spiro-alkylendioxy, R¹⁵-Aryl, R²⁴-Heteroaryl, Benzyl, N-Piperidinyl, -COR⁸, -C(O)NR⁸R⁹, -NR⁸R⁹, und -NR⁸C(O)R⁹ ist oder, wenn zwei R¹⁶ Gruppen an benachbarte Ring-Kohlenstoffatome gebunden sind, zusammen mit diesen Kohlenstoffatomen einen (C₅ bis C₇) Cycloalkylring bilden können;
R¹⁷ unabhängig ausgewählt aus der Gruppe bestehend aus H, (C₁ bis C₆)Alkyl, (C₃ bis C₁₂)Cycloalkyl, (C₃ bis C₁₂)Cycloalkyl(C₁ bis C₆)alkyl, R¹³-Aryl und R¹³-Heteroaryl ist;
R¹⁸ unabhängig ausgewählt aus der Gruppe bestehend aus H, (C₁ bis C₆)Alkyl, (C₃ bis C₁₂)Cycloalkyl, (C₃ bis C₁₂)Cycloalkyl(C₁ bis C₆)alkyl, R¹⁵-Aryl, R²⁴-Heteroaryl, -CO₂R⁹, -C(O)N(R⁸)₂, -COR⁸ und -SO₂R⁹ ist;
R¹⁹ H, (C₃ bis C₁₂)Cycloalkyl-(C₁ bis C₆)alkyl, R¹⁵-Aryl, R²⁴-Heteroaryl, -CO₂R⁹, -C(O)N(R⁸)₂, -COR⁸ oder -SO₂R⁹ ist;
R²⁰ (C₁ bis C₆)Alkyl, (C₃ bis C₁₂)Cycloalkyl, (C₃ bis C₁₂)Cycloalkyl-(C₁ bis C₆)alkyl, Hydroxy(C₁ bis C₆)alkyl, Oxo(C₁ bis C₆)alkyl oder Polyhalogen (C₁ bis C₆) alkyl ist;
R²¹ und R²² unabhängig ausgewählt sind aus der Gruppe bestehend aus H, (C₁ bis C₆)Alkyl, (C₃ bis C₁₂)Cycloalkyl-(C₁ bis C₆)alkyl, Hydroxy(C₁ bis C₆)alkyl, R¹⁵-Aryl, R²⁴-Heteroaryl, R¹⁵-Aryl(C₁ bis C₆)alkyl oder R²⁴-Heteroaryl(C₁ bis C₆)-alkyl; R²³ unabhängig ausgewählt ist aus der Gruppe bestehend aus H, Halogen, (C₁ bis C₆)Alkyl, (C₃ bis C₁₂)Cycloalkyl, R¹⁵-Aryl, und R²⁴-Heteroaryl;
R²⁴ 1 bis 2 Substituenten unabhängig ausgewählt aus der Gruppe bestehend aus H, (C₁ bis C₆)Alkyl, Halogen, Polyhalogen(C₁ bis C₆)alkyl, R¹⁷O-, -N(R¹⁷)₂, -S(R¹⁷), R¹⁷O-(C₁ bis C₆)Alkyl, (R¹⁷)₂N-(C₁ bis C₆)Alkyl, Formyl, -C(O)R¹⁷, -COOR¹⁷, -CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷- und tri(C₁ bis C₆)Alkylsilyl ist; und
R²⁵ unabhängig ausgewählt ist aus der Gruppe bestehend aus H, Halogen, (C₁ bis C₆)-Alkyl und Polyhalogen(C₁ bis C₆)alkyl.

2. Verbindung nach Anspruch 1, wobei Q 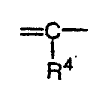 ist.

3. Verbindung nach Anspruch 1, wobei R³ und R⁴ jeweils H sind; und R² und R⁵ unabhängig ausgewählt sind aus der Gruppe bestehend aus Wasserstoff und Halogen.

4. Verbindung nach Anspruch 1, wobei X -S-; -C(O)-; -CH(OR⁸)- oder -C(R²³)₂- ist.

5. Verbindung nach Anspruch 4, wobei X -C(R²³)₂- und R²³ Wasserstoff ist.

6. Verbindung nach Anspruch 1, wobei a 1 oder 2 und b 0 ist.

7. Verbindung nach Anspruch 1, wobei R¹ -NHR¹² oder -N(R¹²)₂ ist.

8. Verbindung nach Anspruch 7, wobei R¹ 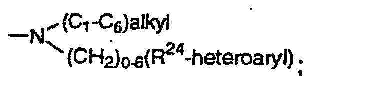 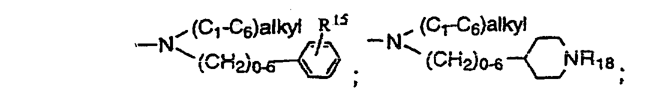 oder 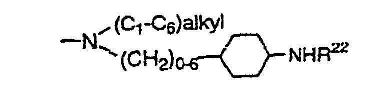 ist,
wobei R¹⁸ (C₁ bis C₆)Alkyl oder -SO₂R⁹ ist; R⁹ (C₁ bis C₆)Alkyl oder Aryl ist; und R²² (C₁ bis C₆)Alkyl oder (C₃ bis C₁₂)Cycloalkyl(C₁ bis C₆)alkyl ist.

9. Verbindung nach Anspruch 1, wobei R⁶ und R⁷ jeweils (C₁ bis C₆)Alkyl sind, oder R⁶ und R⁷ zusammen mit dem Kohlenstoff, an den sie gebunden sind, einen C₃ bis C₆-carbocyclischen Ring bilden.

10. Verbindung nach Anspruch 1, ausgewählt aus der Gruppe bestehend aus solchen Verbindungen, die die Strukturformel 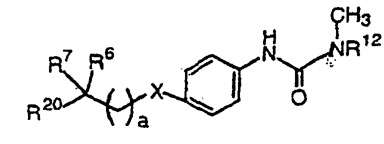 haben, wobei R²⁰, R⁶, R⁷, a, X und R¹² wie in der folgenden Tabelle definiert sind:

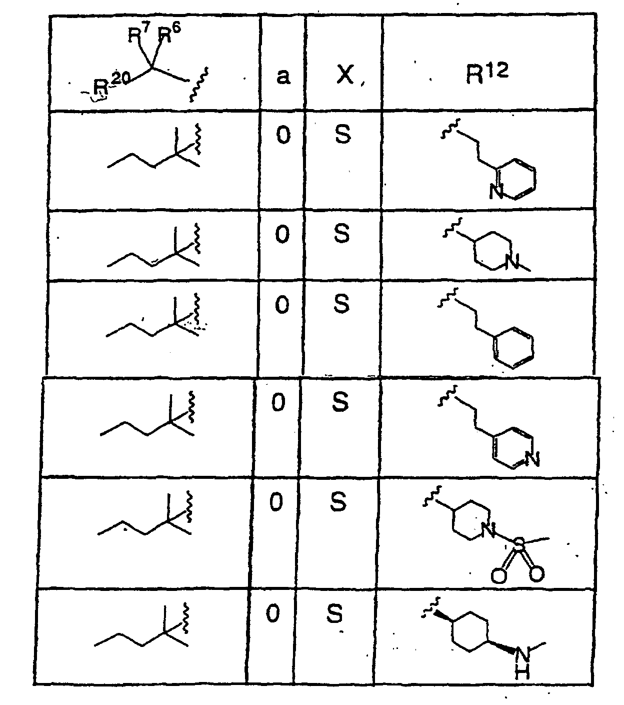

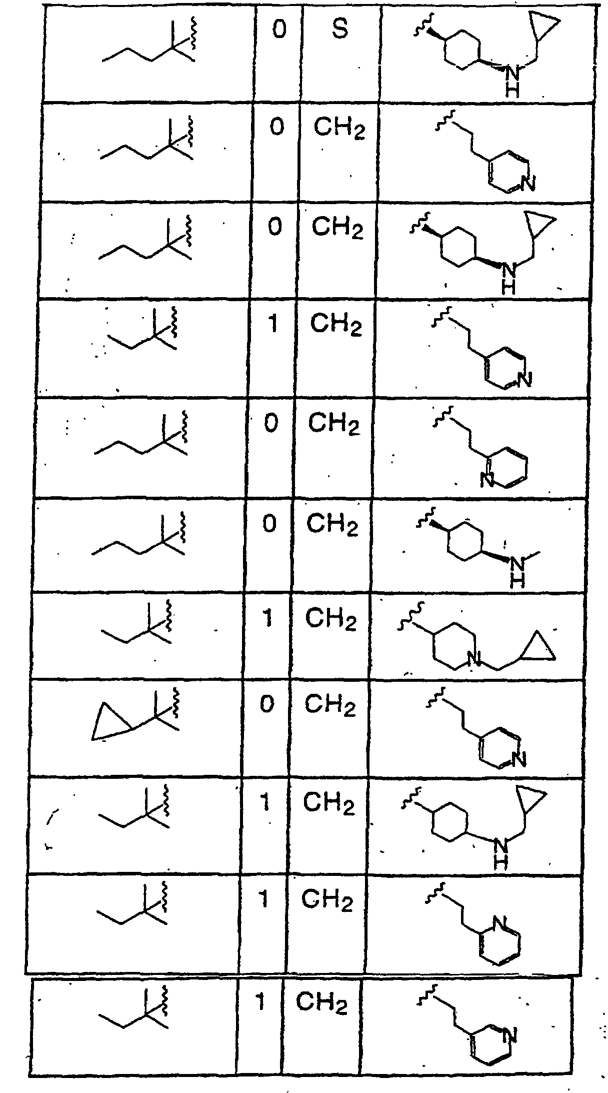

11. Verbindung ausgewählt aus der Gruppe bestehend aus solchen Verbindungen, die die Strukturformel 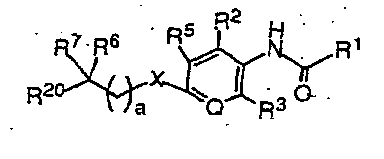 haben, wobei R²⁰, R⁶, R⁷, a, X, R⁵, R², R³, Q und R¹ so sind wie in der folgenden Tabelle definiert:

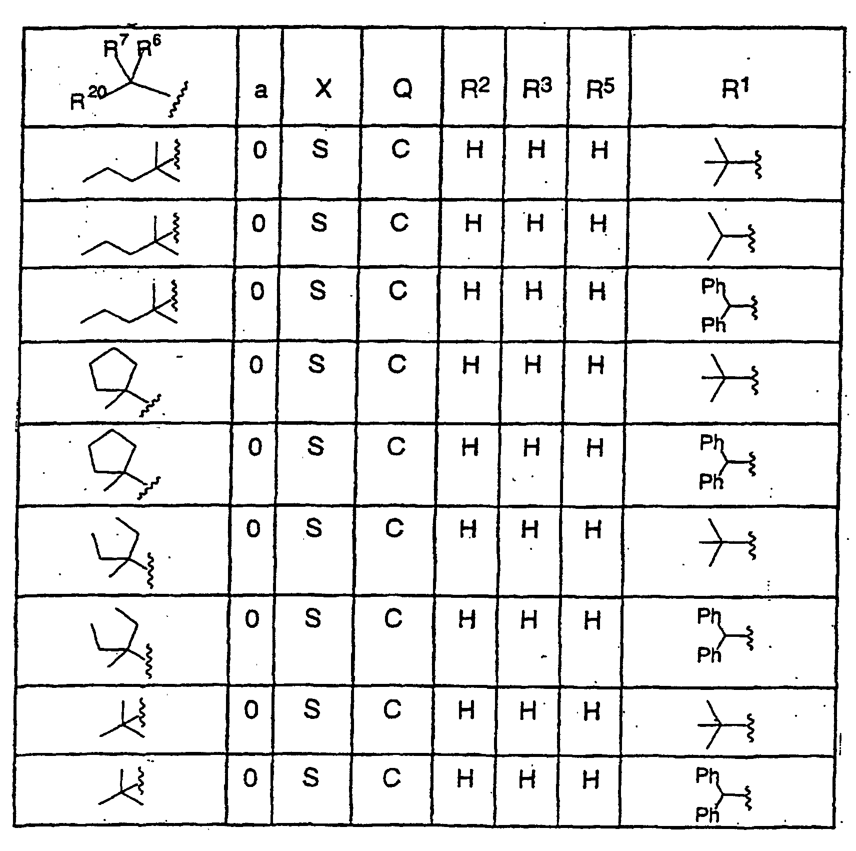

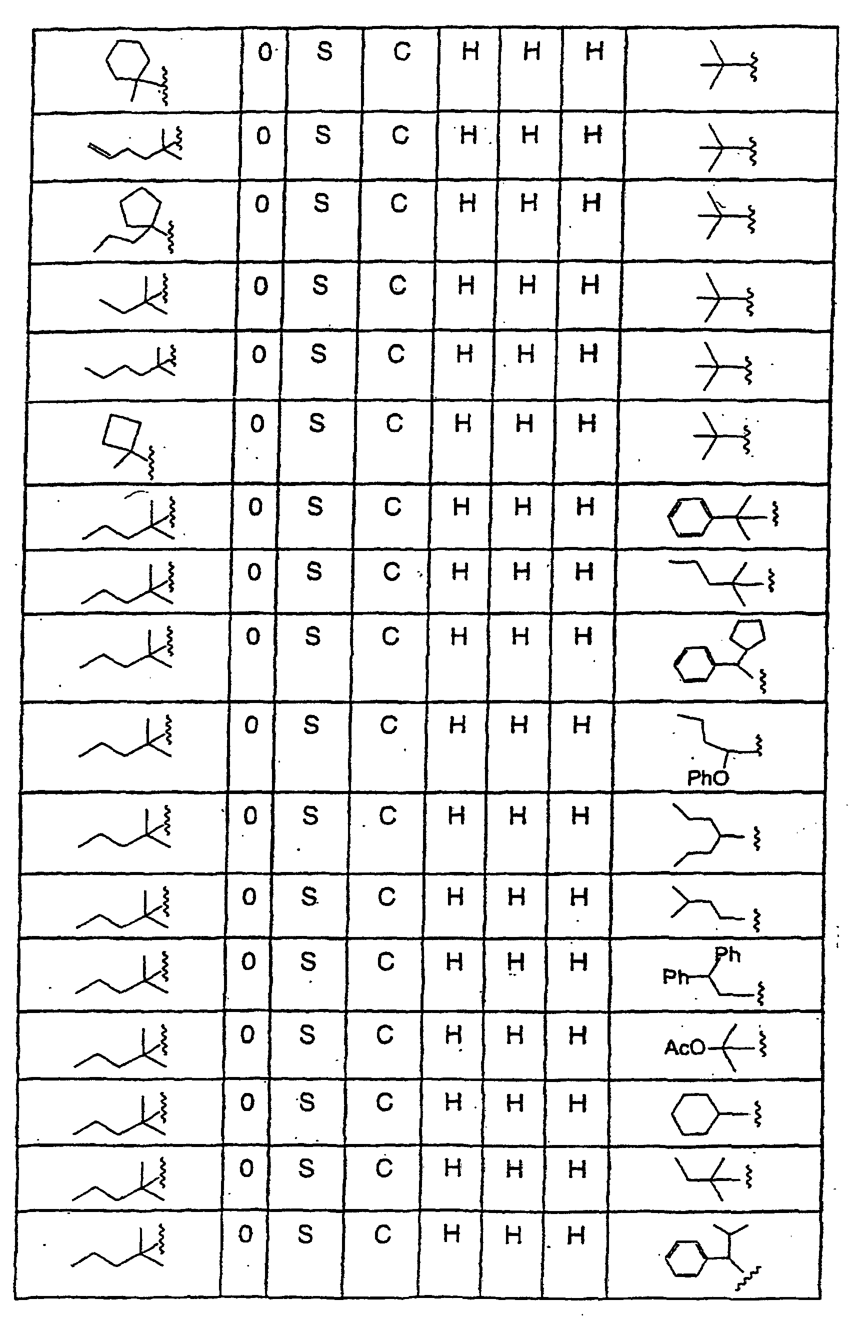

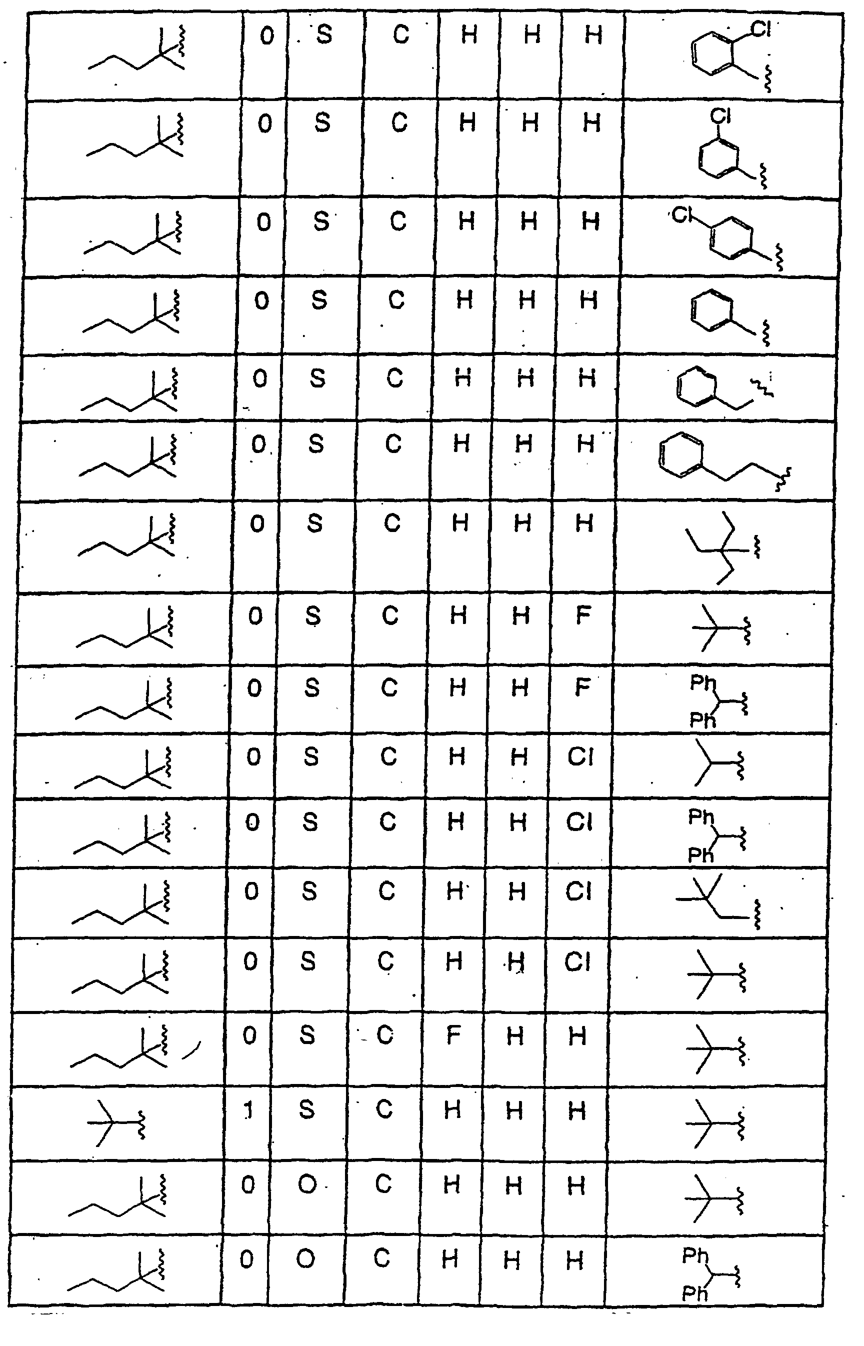

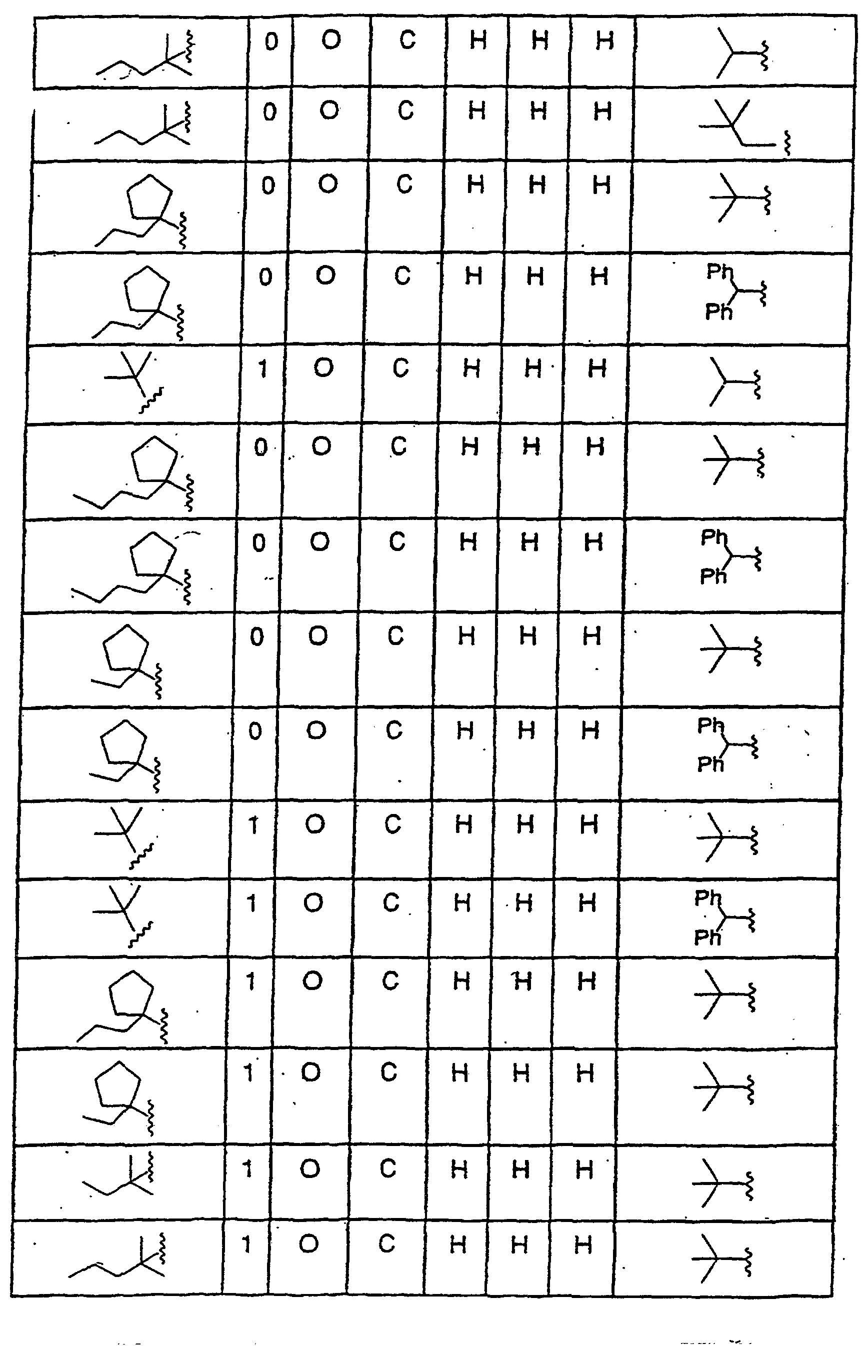

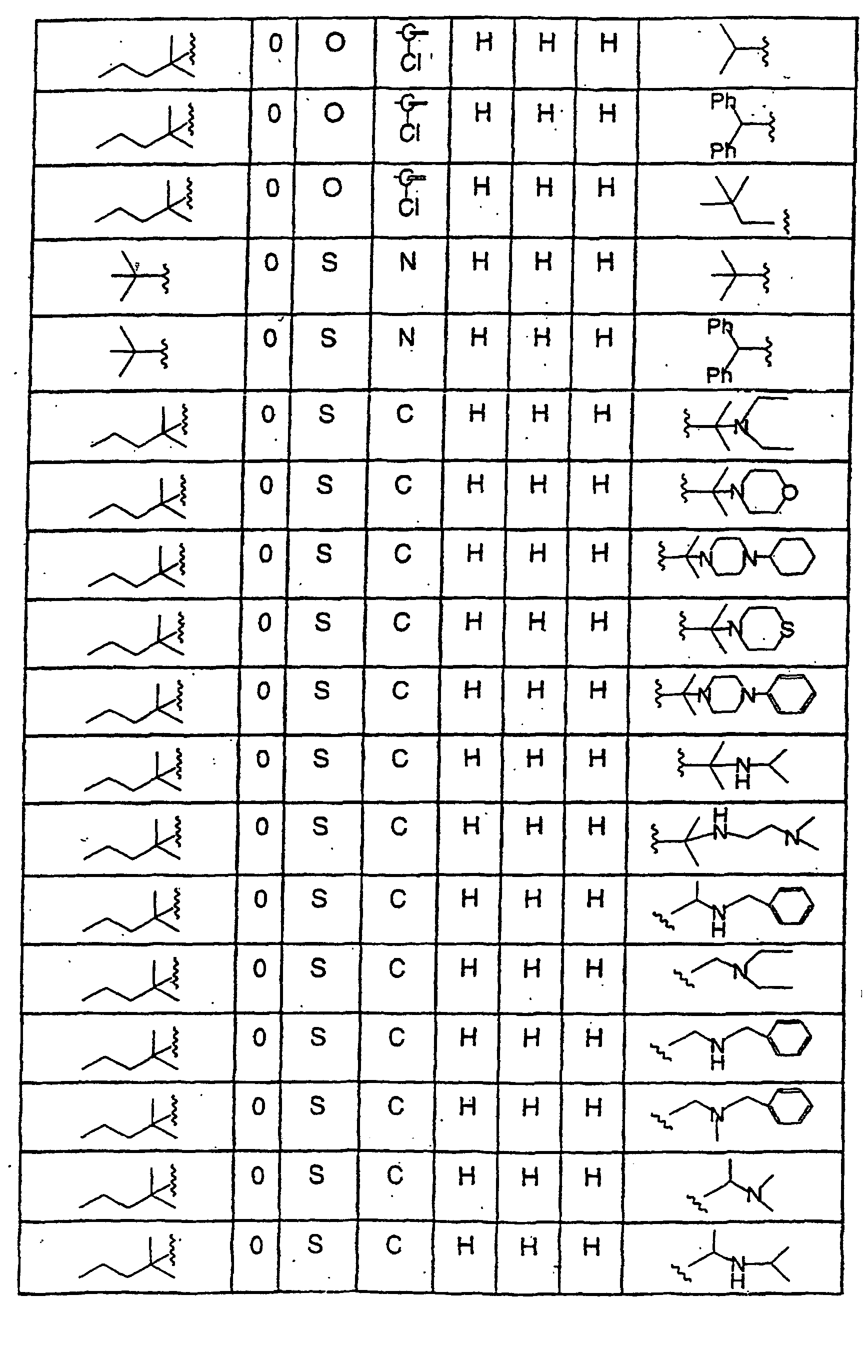

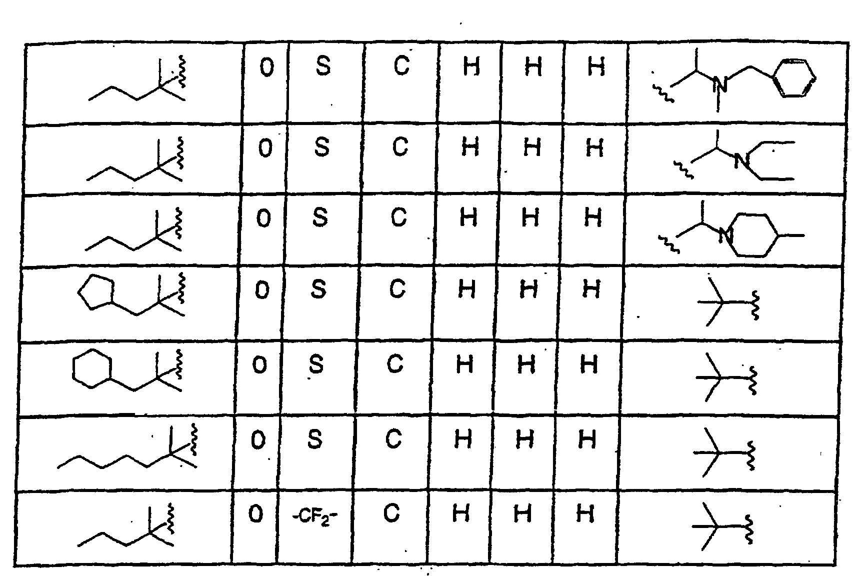

12. Pharmazeutische Zusammensetzung, die eine Verbindung gemäß Anspruch 1 in Kombination mit einem pharmazeutisch akzeptablen Träger enthält.

13. Verwendung einer Verbindung gemäß Anspruch 1 zur Herstellung eines Medikaments zur Behandlung von Essstörungen oder Diabetes.

## Revendications

1. Composé de formule développée : 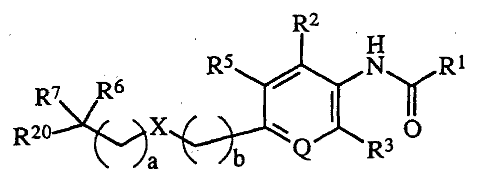 dans laquelle :
a et b représentent chacun indépendamment 0, 1 ou 2, étant entendu que la somme a+b est égale à 0 à 3,
Q représente 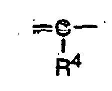 ou -N= ;
X représente -O-, -S-, -SO-, -SO₂-, -CH(OR⁸)-, -C(O)-, -C(R²³)₂-, -C(R²⁵)=C(R²⁵)-, -C≡C- ou 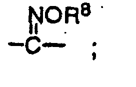 R¹ représente un groupe R¹⁵-aryle, R²⁴-hétéroaryle, -NHR¹²,-N(R¹²)₂,- alkyl(C₁ à C₉)-OC(O)R⁸, aryloxyalkyle(C₁ à C₉), 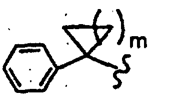 où m est égal à 1 à 4, ou 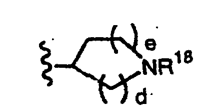 où d et e représentent chacun indépendamment 0,1 ou 2,
R², R³, R⁴ et R⁵ représentent chacun indépendamment un atome d'hydrogène ou d'halogène ou un groupe alkyle en C₁ à C₅, à chaîne droite ou ramifiée, cycloalkyle en C₃ à C₁₂, R¹⁴-cycloalkyle (C₃ à C₁₂),-OR⁸,-N(R⁸)₂,-CO₂R⁸ ou CF₃,
R⁶ et R⁷ représentent chacun indépendamment un atome d'hydrogène ou un groupe alkyle en C₁ à C₉, alcényle en C₂ à C₉, hydroxyalkyle en C₁ à C₉, aminoalkyle en C₁ à C₉, alcoxy(C₁ à C₉)alkyle (C₁ à C₉), cycloalkyle en C₃ à C₁₂ ou cycloalkyl(C₃ à C₁₂)alkyle (C₁ à C₆), ou bien R⁶ et R⁷ forment ensemble, avec l'atome de carbone auquel ils sont liés, un noyau carbocyclique à 3, 4, 5, 6 ou 7 chaînons, ou un noyau hétérocyclique à 4, 5, 6 ou 7 chaînons, dans lequel 1, 2 ou 3 éléments du cycle sont choisis indépendamment parmi O, S et N,
chaque R⁸ représente indépendamment un atome d'hydrogène ou un groupe alkyle en C₁ à C₆, cycloalkyle en C₃ à C₁₂, R¹⁵-aryle ou R²⁴-hétéroaryle,
R⁹ représente un groupe alkyle en C₁ à C₆, cycloalkyle en C₃ à C₁₂, R¹⁵-aryle ou R²⁴-hétéroaryle,
R¹¹ représente un atome d'hydrogène ou un groupe alkyle en C₁ à C₆ ou cycloalkyle en C₃ à C₁₂,
chaque R¹² représente indépendamment un groupe alkyle en C₁ à C₉, à chaîne droite ou ramifiée, hydroxyalkyle en C₂ à C₉, alcoxy(C₁ à C₉)alkyle (C₂ à C₉), N(R¹¹)(R¹⁹)-alkyle (C₂ à C₉), HS-alkyle (C₂ à C₉), alkyle (C₁ à C₉)thio-alkyle (C₂ à C₉), cycloalkyle en C₃ à C₁₂, R¹⁴-cycloalkyle (C₃ à C₁₂), R¹⁵-aryle, R²⁴-hétéroaryle, R¹⁵-arylalkyle (C₁ à C₆), R²⁴-hétéroarylalkyle (C₁ à C₆), 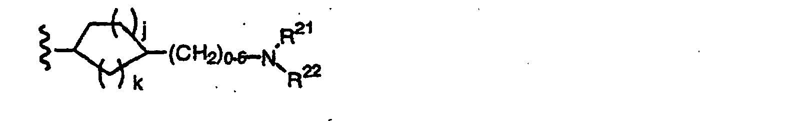 où j et k représentent chacun 0, 1 ou 2, ou 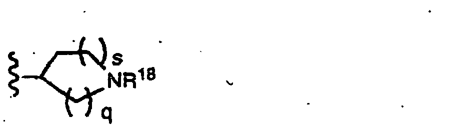 où q est égal à 1 ou 2 et s est égal à 0, 1 ou 2, ou bien deux R¹² forment ensemble, avec l'atome d'azote auquel ils sont liés, un cycle de formule : 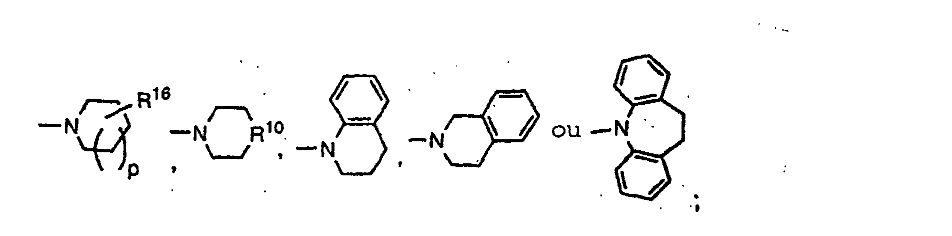 p étant égal à 0,1 ou 2,
R¹⁰ représente -NR¹⁸-, -O- ou -S-,
R¹³ représente 1 à 3 substituants pris indépendamment parmi les atomes d'hydrogène et d'halogène et les groupes alkyle en C₁ à C₆, alcoxy en C₁ à C₆ et CF₃,
R¹⁴ représente 1 à 3 substituants pris indépendamment parmi les groupes alkyle en C₁ à C₆, benzyle, R¹³-aryle et R¹³-hétéroaryle,
R¹⁵ représente 1 à 3 substituants pris indépendamment parmi les atomes d'hydrogène et d'halogène, et les groupes alkyle en C₁ à C₆, polyhalogénoalkyle en C₁ à C₆, R¹⁷O-, -N(R¹⁷)₂, -S(R¹⁷), R¹⁷O-alkyle (C₁ à C₆), (R¹⁷)₂N-alkyle (C₁ à C₆), formyle, -C(O)R¹⁷, -COOR¹⁷, -CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷- et trialkyl(C₁ à C₆)silyle,
R¹⁶ représente 1 à 3 substituants pris indépendamment parmi l'atome d'hydrogène et les groupes alkyle en C₁ à C₆, cycloalkyle en C₃ à C₁₂, spirocycloalkyle en C₃ à C₁₂, spiro-alkylène (C₃ à C₄)dioxy, R¹⁵-aryle, R²⁴-hétéroaryle, benzyle, N-pipéridinyle, -COR⁸, -C(O)NR⁸R⁹, -NR⁸R⁹ et -NR⁸C(O)R⁹, ou bien, lorsque deux groupes R¹⁶ sont reliés à des atomes de carbone adjacents du cycle, ils peuvent former avec lesdits atomes de carbone un noyau cycloalkyle en C₅ à C₇,
chaque R¹⁷ représente indépendamment un atome d'hydrogène ou un groupe alkyle en C₁ à C₆, cycloalkyle en C₃ à C₁₂, cycloalkyl(C₃ à C₁₂)alkyl(C₁ à C₆), R¹³-aryle ou R¹³-hétéroaryle,
R¹⁸ représente un atome d'hydrogène ou un groupe alkyle en C₁ à C₆, cycloalkyle en C₃ à C₁₂, cycloalkyl(C₃ à C₁₂)alkyle (C₁ à C₆), R¹⁵-aryle, R²⁴-hétéroaryle, -CO₂R⁹, -C(O)N(R⁸)₂, -COR⁸ ou -SO₂R⁹,
R¹⁹ représente un atome d'hydrogène ou un groupe cycloalkyl(C₃ à C₁₂)alkyle (C₁ à C₆), R¹⁵-aryle, R²⁴-hétéroaryle, -CO₂R⁹, -C(O)N(R⁸)₂, -COR⁸ ou -SO₂R⁹,
R²⁰ représente un groupe alkyle en C₁ à C₆, cycloalkyle en C₃ à C₁₂, cycloalkyl(C₃ à C₁₂)alkyle (C₁ à C₆), hydroxyalkyle en C₁ à C₆, oxoalkyle en C₁ à C₆ ou polyhalogénoalkyle en C₁ à C₆,
R²¹ et R²² représentent chacun indépendamment un atome d'hydrogène ou un groupe alkyle en C₁ à C₆, cycloalkyl(C₃ à C₁₂)alkyle (C₁ à C₆), hydroxyalkyle en C₁ à C₆, R¹⁵-aryle, R²⁴-hétéroaryle, R¹⁵-arylalkyle (C₁ à C₆) ou R²⁴-hétéroaryl-alkyle (C₁ à C₆),
chaque R²³ représente indépendamment un atome d'hydrogène ou d'halogène, ou un groupe alkyle en C₁ à C₆, cycloalkyle en C₃ à C₁₂, R¹⁵-aryle ou R²⁴-hétéroaryle,
R²⁴ représente 1 ou 2 substituants pris indépendamment parmi les atomes d'hydrogène et d'halogène, et les groupes alkyle en C₁ à C₆, polyhalogénoalkyle en C₁ à C₆, R¹⁷O-, -N(R¹⁷)₂, -SR¹⁷, R¹⁷O-alkyle (C₁ à C₆), (R¹⁷)₂N-alkyle (C₁ à C₆), formyle,
-C(O)R¹⁷, -COOR¹⁷, -CON(R¹⁷)₂, -OC(O)N(R¹⁷)₂, -N(R¹⁷)C(O)N(R¹⁷)₂, -NR¹⁷C(O)R¹⁷, -NR¹⁷C(O)OR¹⁴, R¹⁷S(O)-, R¹⁷SO₂-, R¹⁷SO₂NR¹⁷-
et trialkyl(C₁ à C₆)silyle, et
chaque R²⁵ représente indépendamment un atome d'hydrogène ou d'halogène, ou un groupe alkyle en C₁ à C₆ ou polyhalogénoalkyle en C₁ à C₆,
ou un sel pharmaceutiquement acceptable d'un tel composé.

2. Composé selon la revendication 1, dans lequel Q représente

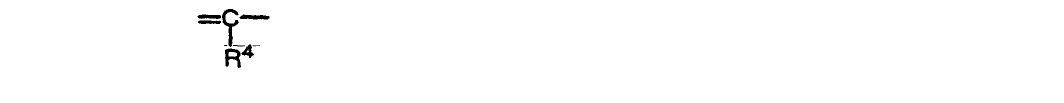

3. Composé selon la revendication 1, dans lequel R³ et R⁴ représentent chacun un atome d'hydrogène, et R² et R⁵ représentent chacun indépendamment un atome d'hydrogène ou d'halogène.

4. Composé selon la revendication 1, dans lequel X représente -S-, -C(O)-, -CH(OR⁸)- ou -C(R²³)₂-.

5. Composé selon la revendication 4, dans lequel X représente -C(R²³)₂- et R²³ représente un atome d'hydrogène.

6. Composé selon la revendication 1, dans lequel a est égal à 1 ou 2 et b est égal à 0.

7. Composé selon la revendication 1, dans lequel R¹ représente -NHR¹² ou -N(R¹²)₂.

8. Composé selon la revendication 7, dans lequel R¹ représente 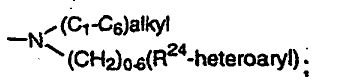 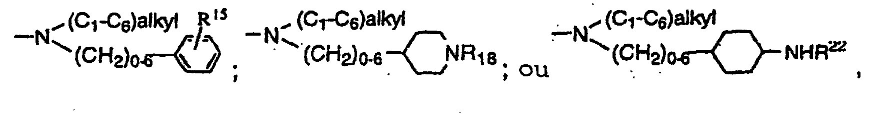 où R¹⁸ représente un groupe alkyle en C₁ à C₆ ou -SO₂R⁹, R⁹ désignant un groupe alkyle en C₁ à C₆ ou un groupe aryle, et R²² représente un groupe alkyle en C₁ à C₆ ou cycloalkyl(C₃ à C₁₂)alkyle (C₁ à C₆).

9. Composé selon la revendication 1, dans lequel R⁶ et R⁷ représentent chacun un groupe alkyle en C₁ à C₆, ou bien R⁶ et R⁷ forment ensemble, avec l'atome de carbone auquel ils sont liés, un noyau carbocyclique en C₃ à C₆.

10. Composé selon la revendication 1, qui est choisi parmi les composés de formule développée : 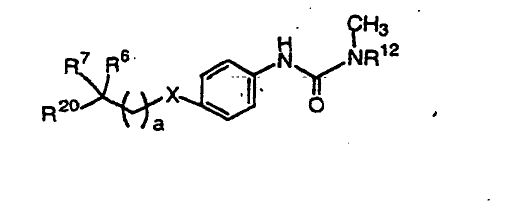 dans laquelle R²⁰, R⁶, R⁷, a, X et R¹² ont les significations indiquées dans le tableau suivant :

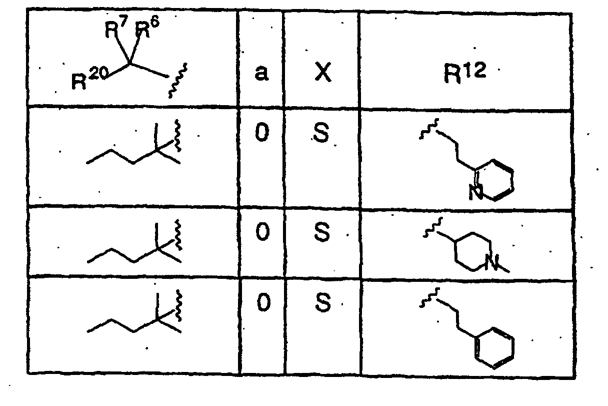

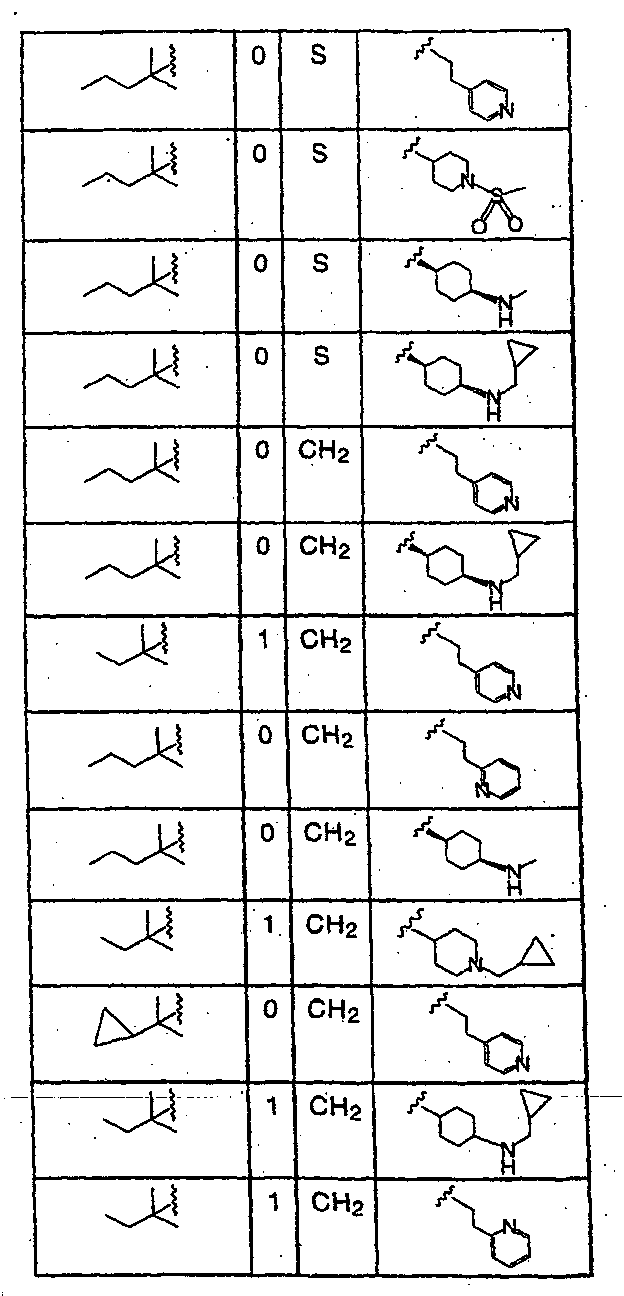

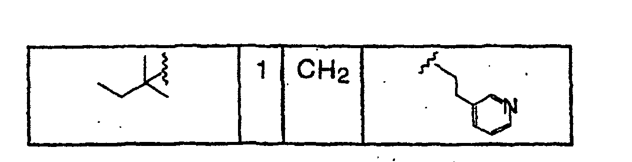

11. Composé qui est choisi parmi les composés de formule développée : 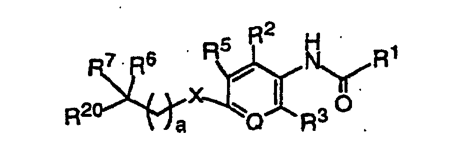 dans laquelle R²⁰, R⁶, R⁷, a, X, R⁵, R², R³ Q et R¹ ont les significations indiquées dans le tableau suivant :

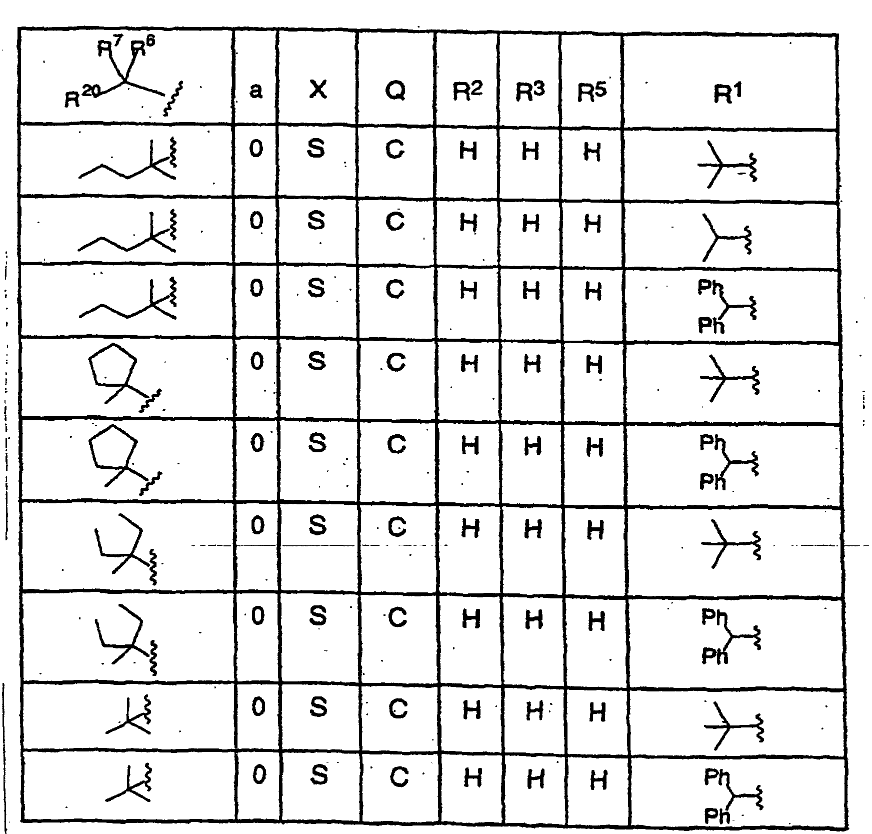

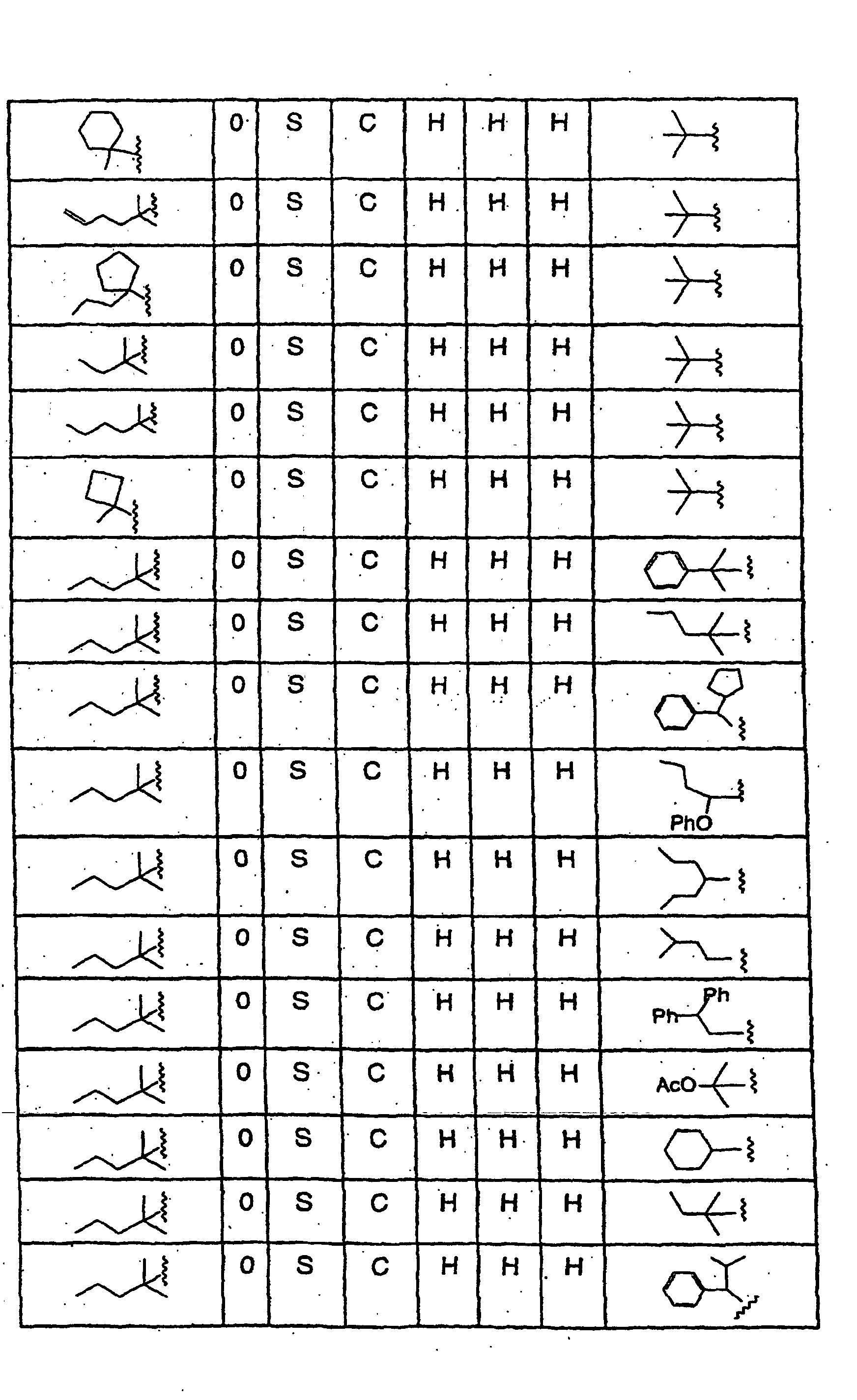

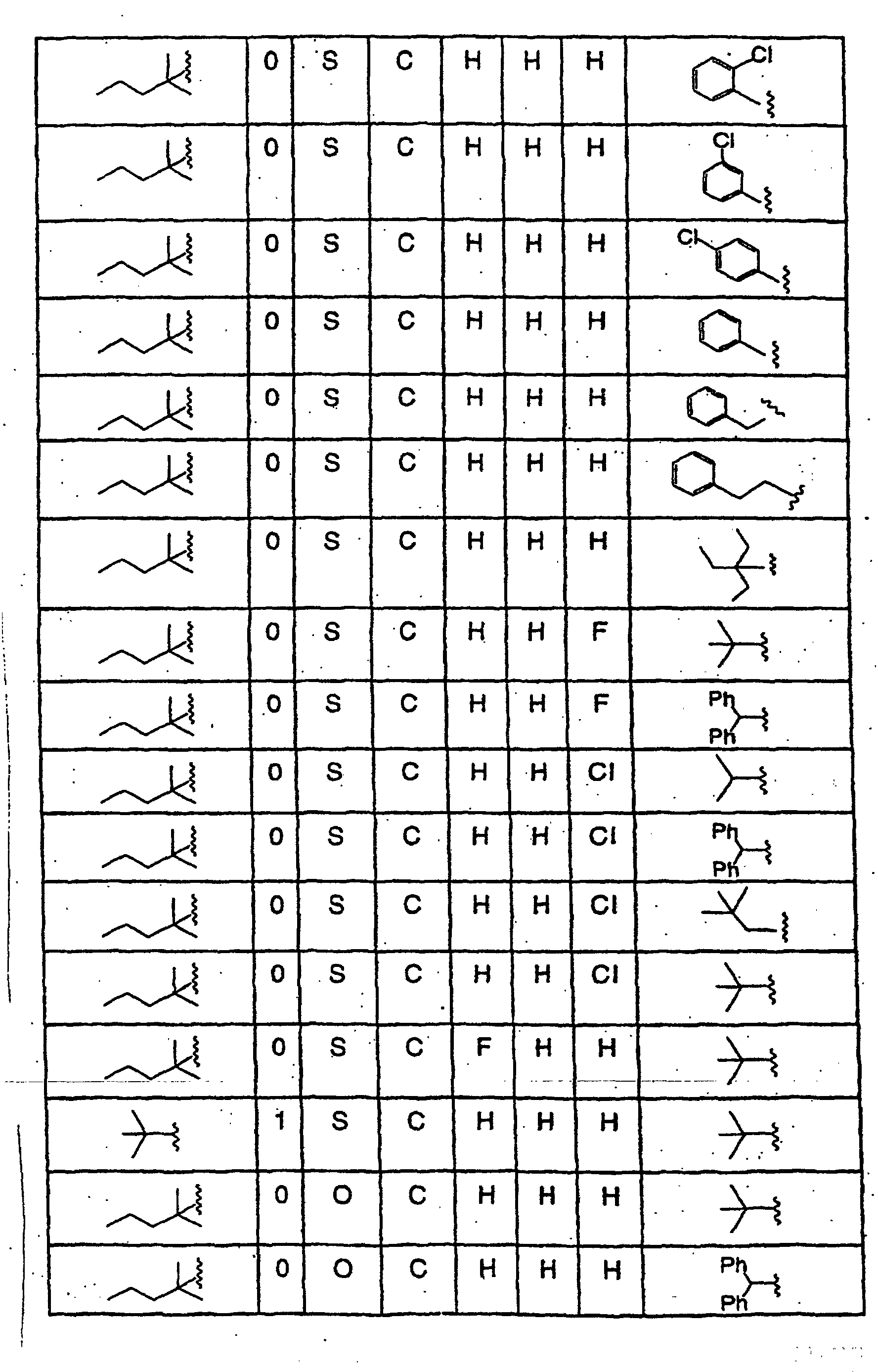

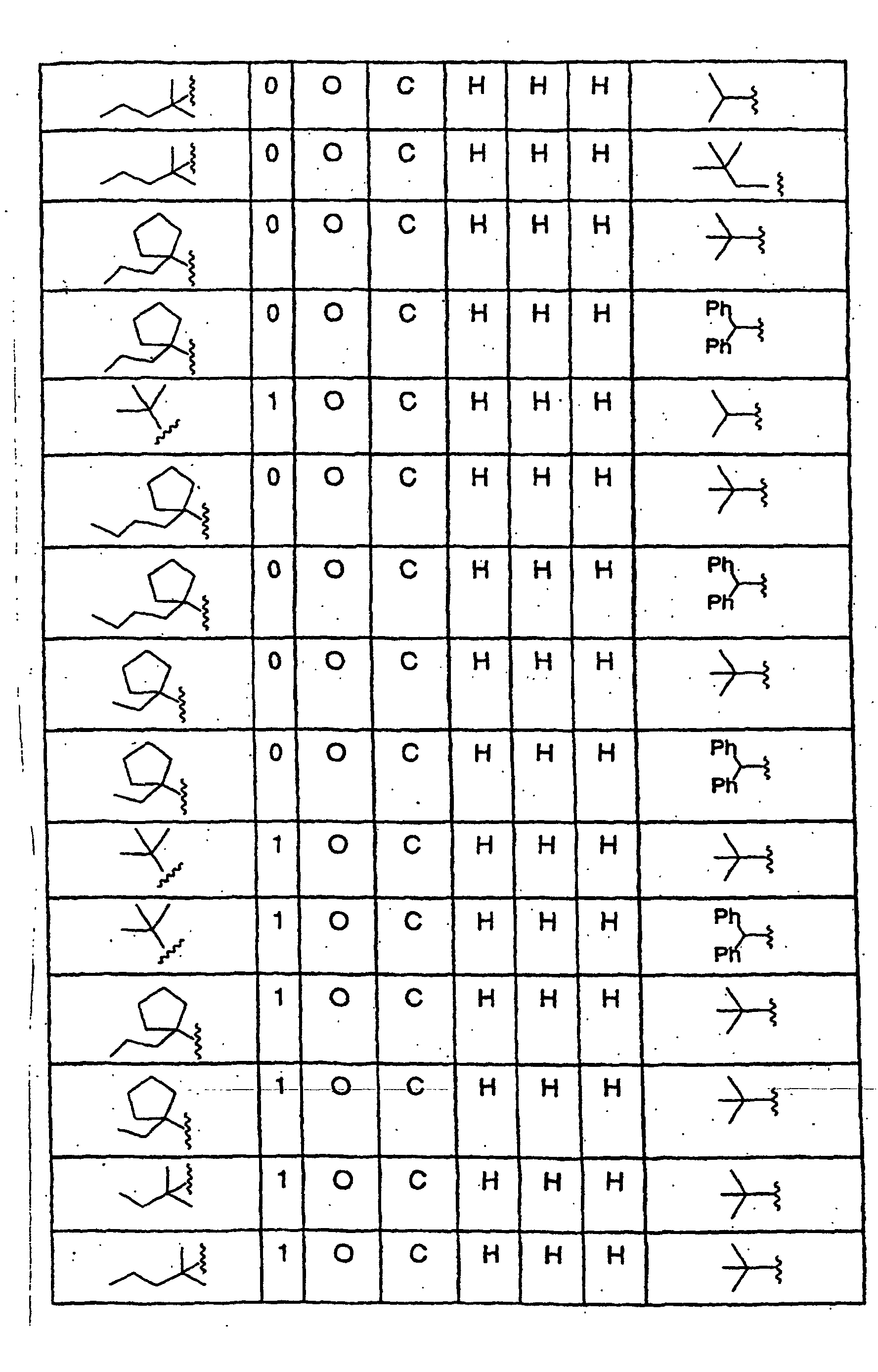

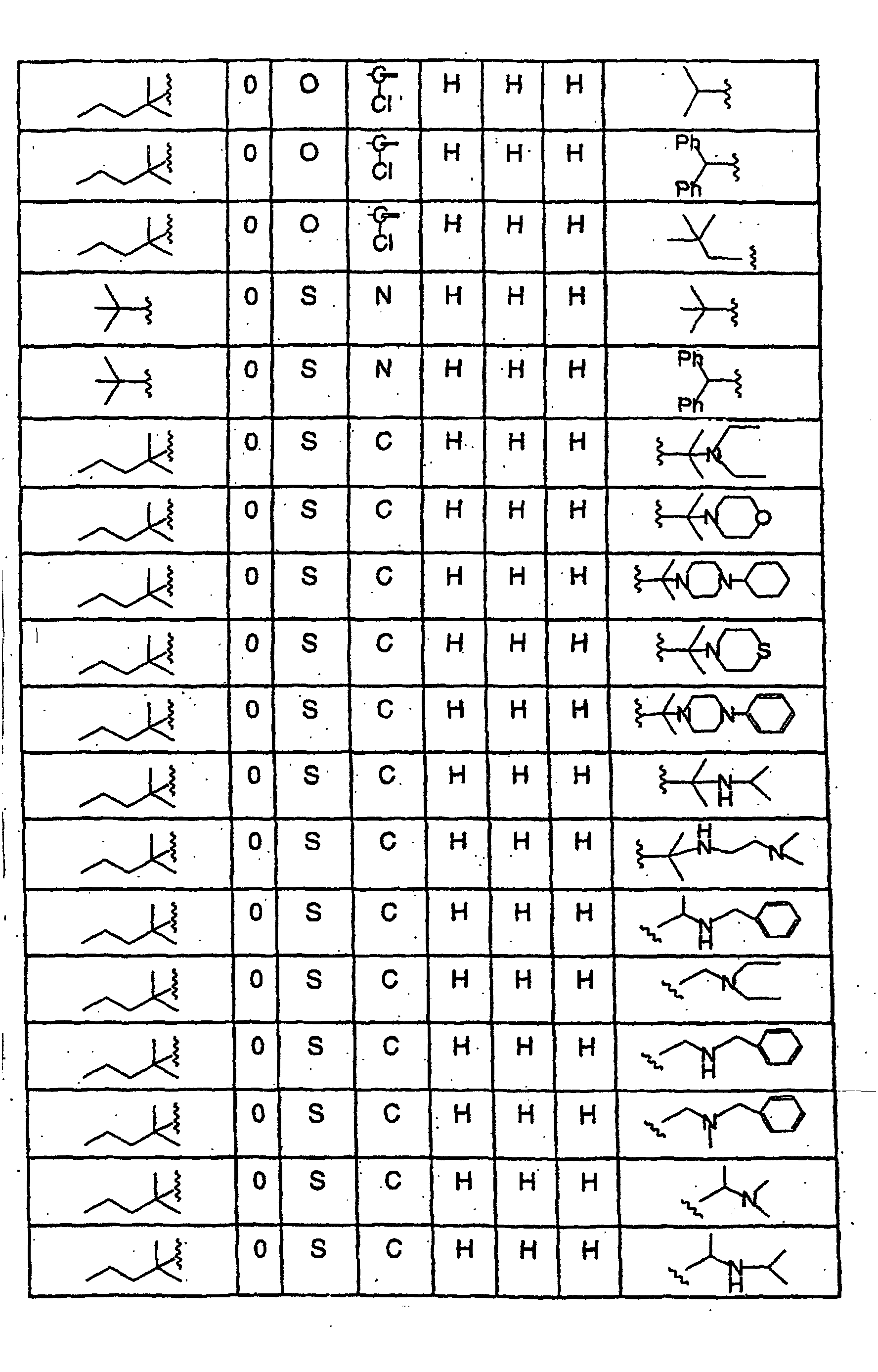

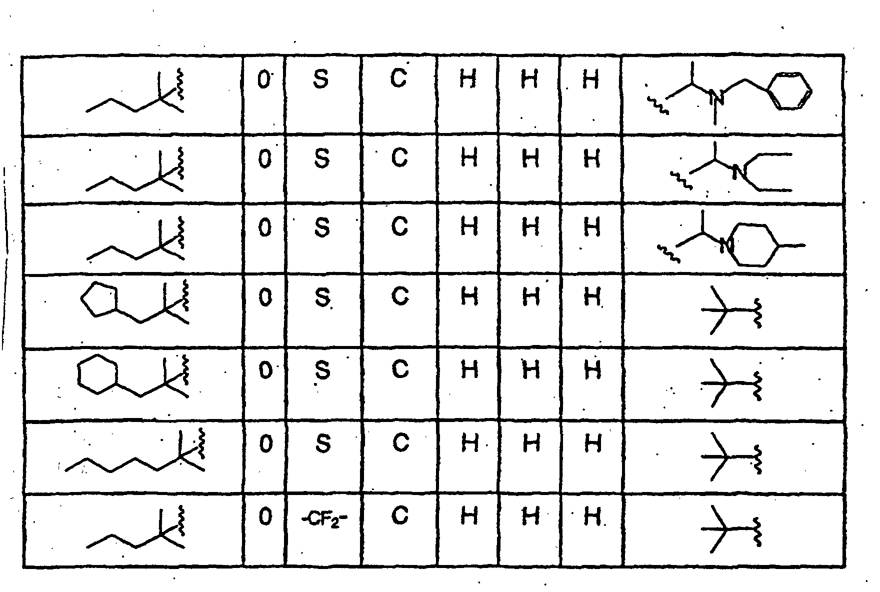

12. Composition pharmaceutique qui comprend un composé tel que défini dans la revendication 1, en combinaison avec un support pharmaceutiquement acceptable.

13. Utilisation d'un composé selon la revendication 1 pour la préparation d'un médicament destiné au traitement des troubles de l'alimentation ou des diabètes.
